# EUROPEAN PATENT SPECIFICATION

(11) **EP 2 630 157 B1**
(45) Date of publication and mention of the grant of the patent: **24.01.2018**
(21) Application number: 11787597.1
(22) Date of filing: 20.10.2011
(51) Int. Cl.: C12N 9/88, C12P 5/00, C12N 9/10, C12N 15/82, A01H 5/08

(54) **1-DEOXY-D-XYLULOSE 5-PHOSPHATE SYNTHASE ALLELES RESPONSIBLE FOR ENHANCED TERPENE BIOSYNTHESIS**
FÜR VERSTÄRKTE TERPENBIOSYNTHESE VERANTWORTLICHE 1-DESOXY-D-XYLULOSE-5-PHOSPHATSYNTHASEALLELE
ALLÈLES DE 1-DÉSOXY-D-XYLULOSE 5-PHOSPHATE SYNTHASE RESPONSABLES DE LA BIOSYNTHÈSE AMÉLIORÉE DES TERPÈNES

(30) Priority: 10.05.2011 EP 11003842; 20.10.2010 EP 10013809
(43) Date of publication of application: 28.08.2013
(73) Proprietor: Genoplante-Valor, 75015 Paris (FR)
(72) Inventor: HUGUENEY, Phlippe, 79232 March-Hugstetten (DE); DUCHENE, Eric, 67000 Strasbourg (FR); MERDINOGLU, Didier, 68000 Colmar (FR)
(74) Representative: Barbot, Willy
(86) International application number: PCT/EP2011/005283
(87) International publication number: WO 2012/052171

(56) References cited:
- WO-A1-00/44912
- WO-A1-99/11757
- GB-A- 2 430 200
- LANGE ET AL: "A family of transketolases that directs isoprenoid biosynthesis via a mevalonate-independent pathway", FASEB JOURNAL, FED. OF AMERICAN SOC. FOR EXPERIMENTAL BIOLOGY, US, vol. 95, 1 March 1998 (1998-03-01), pages 2100-2104, XP002116672, ISSN: 0892-6638
- KUZUYAMA TOMOHISA ET AL: "CLONING AND CHARACTERIZATION OF 1-DEOXY-D-XYLULOSE 5-PHOSPHATE SYNTHASE FROM STREPTOMYCES SP. STRAIN CL190, WHICH USES BOTH THE MEVALONATE AND NONMEVALONATE PATHWAYS FOR ISOPENTENYL DIPHOSPHATE BIOSYNTHESIS", JOURNAL OF BACTERIOLOGY, AMERICAN SOCIETY FOR MICROBIOLOGY, WASHINGTON, DC; US, vol. 182, no. 4, 1 February 2000 (2000-02-01), pages 891-897, XP009081766, ISSN: 0021-9193, DOI: 10.1128/JB.182.4.891-897.2000
- DUCHENE ERIC ET AL: "A grapevine (Vitis vinifera L.) deoxy-d-xylulose synthase gene colocates with a major quantitative trait loci for terpenol content", THEORETICAL AND APPLIED GENETICS, vol. 118, no. 3, February 2009 (2009-02), pages 541-552, XP002620521, ISSN: 0040-5752, DOI: 10.1007/s00122-008-0919-8
- JURI BATTILANA ET AL: "The 1-deoxy-d-xylulose 5-phosphate synthase gene co-localizes with a major QTL affecting monoterpene content in grapevine", THEORETICAL AND APPLIED GENETICS ; INTERNATIONAL JOURNAL OF PLANT BREEDING RESEARCH, vol. 118, no. 4, 27 November 2008 (2008-11-27), pages 653-669, XP019698334, SPRINGER, BERLIN, DE ISSN: 1432-2242
- XIANG SONG ET AL: "Crystal structure of 1-deoxy-d-xylulose 5-phosphate synthase, a crucial enzyme for isoprenoids biosynthesis", JOURNAL OF BIOLOGICAL CHEMISTRY, vol. 282, no. 4, January 2007 (2007-01), pages 2676-2682, XP002620522, ISSN: 0021-9258, DOI: 10.1074/jbc.m610235200
- JAILLON OLIVIER ET AL: "The grapevine genome sequence suggests ancestral hexaploidization in major angiosperm phyla", NATURE (LONDON), vol. 449, no. 7161, September 2007 (2007-09), pages 463-467-METHODS, XP002620523, ISSN: 0028-0836, DOI: 10.1038/nature06148
- PAETZOLD HEIKE ET AL: "The Isogene 1-Deoxy-D-Xylulose 5-Phosphate Synthase 2 Controls Isoprenoid Profiles, Precursor Pathway Allocation, and Density of Tomato Trichomes", MOLECULAR PLANT, vol. 3, no. 5, September 2010 (2010-09), pages 904-916, XP002620524,
- MUNOZ-BERTOMEU JESUS ET AL: "Up-regulation of 1-deoxy-D-xylulose-5-phosphate synthase enhances production of essential oils in transgenic spike lavender", PLANT PHYSIOLOGY (ROCKVILLE), vol. 142, no. 3, November 2006 (2006-11), pages 890-900, XP002620525, ISSN: 0032-0889, DOI: 10.1104/pp.106.086355

### SEQUENCE LISTING

<110> genoplante x, x Philippe, HUGUENEY
<120> 1-DEOXY-D-XYLULOSE 5-PHOSPHATE SYNTHASE DXS2 ALLELES RESPONSIBLE FOR ENHANCED TERPENE BIOSYNTHESIS
<130> GPL-B-0001 PCT
<150> EP 10013809.8
   <151> 2010-10-20
<160> 29
<170> PatentIn version 3.5
<210> 1
   <211> 720
   <212> PRT
   <213> Artificial sequence
<220>
   <223> plant DXS consens sequence
<220>
   <221> MISC_FEATURE
   <222> (3)..(3)
   <223> X=L or S
<220>
   <221> MISC_FEATURE
   <222> (4)..(4)
   <223> X=C or S
<220>
   <221> MISC_FEATURE
   <222> (5)..(5)
   <223> X= T, N or A
<220>
   <221> MISC_FEATURE
   <222> (6)..(6)
   <223> X= L, F or Y
<220>
   <221> MISC_FEATURE
   <222> (7)..(7)
   <223> X= S or A
<220>
   <221> MISC_FEATURE
   <222> (8)..(8)
   <223> X= F or V
<220>
   <221> MISC_FEATURE
   <222> (10)..(10)
   <223> X= A, S or G
<220>
   <221> MISC_FEATURE
   <222> (11)..(11)
   <223> X= H, Y or I
<220>
   <221> MISC_FEATURE
   <222> (12)..(12)
   <223> X=F, I or L
<220>
   <221> MISC_FEATURE
   <222> (13)..(13)
   <223> X=S, I or N
<220>
   <221> MISC_FEATURE
   <222> (14)..(14)
   <223> X= Q, T or R
<220>
   <221> MISC_FEATURE
   <222> (15)..(15)
   <223> X= A, K or T
<220>
   <221> MISC_FEATURE
   <222> (16)..(16)
   <223> X= G, V or no amino acids
<220>
   <221> MISC_FEATURE
   <222> (17)..(17)
   <223> X= G, A or no amino acid
<220>
   <221> MISC_FEATURE
   <222> (18)..(18)
   <223> X= A, L or V
<220>
   <221> MISC_FEATURE
   <222> (19)..(19)
   <223> X= A or S
<220>
   <221> MISC_FEATURE
   <222> (20)..(20)
   <223> X= S or T
<220>
   <221> MISC_FEATURE
   <222> (21)..(21)
   <223> X= N or D
<220>
   <221> MISC_FEATURE
   <222> (22)..(22)
   <223> X= P, S, A or Y
<220>
   <221> MISC_FEATURE
   <222> (23)..(23)
   <223> X= Q, C or S
<220>
   <221> MISC_FEATURE
   <222> (24)..(24)
   <223> X= R or K
<220>
   <221> MISC_FEATURE
   <222> (25)..(25)
   <223> X=L, S, P or Q
<220>
   <221> MISC_FEATURE
   <222> (26)..(26)
   <223> X= T, L or S
<220>
   <221> MISC_FEATURE
   <222> (27)..(27)
   <223> X= P or S
<220>
   <221> MISC_FEATURE
   <222> (28)..(28)
   <223> X= Q or L
<220>
   <221> MISC_FEATURE
   <222> (29)..(29)
   <223> X= C, F, S or P
<220>
   <221> MISC_FEATURE
   <222> (31)..(31)
   <223> X= H, S or E
<220>
   <221> MISC_FEATURE
   <222> (32)..(32)
   <223> X= L, R, S or W
<220>
   <221> MISC_FEATURE
   <222> (33)..(33)
   <223> X= F, S, I or Q
<220>
   <221> MISC_FEATURE
   <222> (34)..(34)
   <223> X= L, H or W
<220>
   <221> MISC_FEATURE
   <222> (35)..(35)
   <223> X= G or V
<220>
   <221> MISC_FEATURE
   <222> (36)..(36)
   <223> X= no amino acid, V or T
<220>
   <221> MISC_FEATURE
   <222> (37)..(37)
   <223> X= D or no amino acid
<220>
   <221> MISC_FEATURE
   <222> (39)..(39)
   <223> X= Q, P or H
<220>
   <221> MISC_FEATURE
   <222> (40)..(40)
   <223> X= C, S, F or Y
<220>
   <221> MISC_FEATURE
   <222> (41)..(41)
   <223> X= Q, P or L
<220>
   <221> MISC_FEATURE
   <222> (42)..(42)
   <223> X= C, F, A or no amino acid
<220>
   <221> MISC_FEATURE
   <222> (43)..(43)
   <223> X= S, L, H or Q
<220>
   <221> MISC_FEATURE
   <222> (44)..(44)
   <223> X= Q, K, A or H
<220>
   <221> MISC_FEATURE
   <222> (45)..(45)
   <223> X= Q, P, K or H
<220>
   <221> MISC_FEATURE
   <222> (46)..(46)
   <223> X= R, N or L
<220>
   <221> MISC_FEATURE
   <222> (47)..(47)
   <223> X= S, N, T or L
<220>
   <221> MISC_FEATURE
   <222> (48)..(48)
   <223> X= K, N, Q, H or R
<220>
   <221> MISC_FEATURE
   <222> (49)..(49)
   <223> X= A, S, V or Q
<220>
   <221> MISC_FEATURE
   <222> (50)..(50)
   <223> X= H, M or no amino acid
<220>
   <221> MISC_FEATURE
   <222> (51)..(51)
   <223> X= R, S or K
<220>
   <221> MISC_FEATURE
   <222> (52)..(52)
   <223> X= K or N
<220>
   <221> MISC_FEATURE
   <222> (54)..(54)
   <223> X=P, R or S
<220>
   <221> MISC_FEATURE
   <222> (55)..(55)
   <223> X= N, A, R or C
<220>
   <221> MISC_FEATURE
   <222> (56)..(56)
   <223> X= G, K or T
<220>
   <221> MISC_FEATURE
   <222> (58)..(58)
   <223> X= C, Q, Y or A
<220>
   <221> MISC_FEATURE
   <222> (61)..(61)
   <223> X= L or G
<220>
   <221> MISC_FEATURE
   <222> (62)..(62)
   <223> X= S or A
<220>
   <221> MISC_FEATURE
   <222> (63)..(63)
   <223> X= D, E or W
<220>
   <221> MISC_FEATURE
   <222> (64)..(64)
   <223> X=R, K or S
<220>
   <221> MISC_FEATURE
   <222> (65)..(65)
   <223> X=E, G or I
<220>
   <221> MISC_FEATURE
   <222> (66)..(66)
   <223> X=E or D
<220>
   <221> MISC_FEATURE
   <222> (67)..(67)
   <223> X= F or Y
<220>
   <221> MISC_FEATURE
   <222> (68)..(68)
   <223> X=H, Y or S
<220>
   <221> MISC_FEATURE
   <222> (69)..(69)
   <223> X=S, T, G or A
<220>
   <221> MISC_FEATURE
   <222> (70)..(70)
   <223> X= Q, N, E or H
<220>
   <221> MISC_FEATURE
   <222> (71)..(71)
   <223> X=R or K
<220>
   <221> MISC_FEATURE
   <222> (73)..(73)
   <223> X=P or A
<220>
   <221> MISC_FEATURE
   <222> (74)..(74)
   <223> X=T or V
<220>
   <221> MISC_FEATURE
   <222> (76)..(76)
   <223> X=L or I
<220>
   <221> MISC_FEATURE
   <222> (77)..(77)
   <223> X=L or V
<220>
   <221> MISC_FEATURE
   <222> (80)..(80)
   <223> X=I or V
<220>
   <221> MISC_FEATURE
   <222> (82)..(82)
   <223> X=Y or D
<220>
   <221> MISC_FEATURE
   <222> (84)..(84)
   <223> X=I, V or A
<220>
   <221> MISC_FEATURE
   <222> (91)..(91)
   <223> X=V, L, A or T
<220>
   <221> MISC_FEATURE
   <222> (92)..(92)
   <223> X=K, H, Q or P
<220>
   <221> MISC_FEATURE
   <222> (93)..(93)
   <223> X=E or D
<220>
   <221> MISC_FEATURE
   <222> (95)..(95)
   <223> X=K or E
<220>
   <221> MISC_FEATURE
   <222> (98)..(98)
   <223> X=A or S
<220>
   <221> MISC_FEATURE
   <222> (99)..(99)
   <223> X=D, A, S or E
<220>
   <221> MISC_FEATURE
   <222> (103)..(103)
   <223> X=S, A or L
<220>
   <221> MISC_FEATURE
   <222> (104)..(104)
   <223> X=D or E
<220>
   <221> MISC_FEATURE
   <222> (105)..(105)
   <223> X=V, T or I
<220>
   <221> MISC_FEATURE
   <222> (106)..(106)
   <223> X= V or I
<220>
   <221> MISC_FEATURE
   <222> (107)..(107)
   <223> X= F, Y or H
<220>
   <221> MISC_FEATURE
   <222> (108)..(108)
   <223> X= N, S or T
<220>
   <221> MISC_FEATURE
   <222> (110)..(110)
   <223> X=S or A
<220>
   <221> MISC_FEATURE
   <222> (111)..(111)
   <223> X=K or R
<220>
   <221> MISC_FEATURE
   <222> (117)..(117)
   <223> X=G or S
<220>
   <221> MISC_FEATURE
   <222> (118)..(118)
   <223> X=S or A
<220>
   <221> MISC_FEATURE
   <222> (124)..(124)
   <223> X=D or E
<220>
   <221> MISC_FEATURE
   <222> (126)..(126)
   <223> X=T or A
<220>
   <221> MISC_FEATURE
   <222> (128)..(128)
   <223> X=V or A
<220>
   <221> MISC_FEATURE
   <222> (131)..(131)
   <223> X=Y or H
<220>
   <221> MISC_FEATURE
   <222> (132)..(132)
   <223> X=V or I
<220>
   <221> MISC_FEATURE
   <222> (134)..(134)
   <223> X=N or D
<220>
   <221> MISC_FEATURE
   <222> (135)..(135)
   <223> X=A or T
<220>
   <221> MISC_FEATURE
   <222> (137)..(137)
   <223> X=Q, E or D
<220>
   <221> MISC_FEATURE
   <222> (139)..(139)
   <223> X= R or K
<220>
   <221> MISC_FEATURE
   <222> (141)..(141)
   <223> X= L or I
<220>
   <221> MISC_FEATURE
   <222> (148)..(148)
   <223> X= S or A
<220>
   <221> MISC_FEATURE
   <222> (150)..(150)
   <223> X= P or A
<220>
   <221> MISC_FEATURE
   <222> (159)..(159)
   <223> X= D, G, E or S
<220>
   <221> MISC_FEATURE
   <222> (160)..(160)
   <223> X= K, Q or R
<220>
   <221> MISC_FEATURE
   <222> (162)..(162)
   <223> X= H, P or S
<220>
   <221> MISC_FEATURE
   <222> (164)..(164)
   <223> X= M, L or I
<220>
   <221> MISC_FEATURE
   <222> (168)..(168)
   <223> X= D, N or S
<220>
   <221> MISC_FEATURE
   <222> (171)..(171)
   <223> X= A or S
<220>
   <221> MISC_FEATURE
   <222> (174)..(174)
   <223> X= T or P
<220>
   <221> MISC_FEATURE
   <222> (177)..(177)
   <223> X= G, S or D
<220>
   <221> MISC_FEATURE
   <222> (180)..(180)
   <223> X= E, I, V or A
<220>
   <221> MISC_FEATURE
   <222> (181)..(181)
   <223> X= Y, H, or F
<220>
   <221> MISC_FEATURE
   <222> (183)..(183)
   <223> X= C or A
<220>
   <221> MISC_FEATURE
   <222> (186)..(186)
   <223> X= T or A
<220>
   <221> MISC_FEATURE
   <222> (192)..(192)
   <223> X= T or S
<220>
   <221> MISC_FEATURE
   <222> (196)..(196)
   <223> X= G or A
<220>
   <221> MISC_FEATURE
   <222> (202)..(202)
   <223> X= G or A
<220>
   <221> MISC_FEATURE
   <222> (205)..(205)
   <223> X= L or I
<220>
   <221> MISC_FEATURE
   <222> (206)..(206)
   <223> X= L or K
<220>
   <221> MISC_FEATURE
   <222> (208)..(208)
   <223> X= R or K
<220>
   <221> MISC_FEATURE
   <222> (209)..(209)
   <223> X= N, S or K
<220>
   <221> MISC_FEATURE
   <222> (211)..(211)
   <223> X= N or H
<220>
   <221> MISC_FEATURE
   <222> (213)..(213)
   <223> X= I or V
<220>
   <221> MISC_FEATURE
   <222> (214)..(214)
   <223> X= A or S
<220>
   <221> MISC_FEATURE
   <222> (220)..(220)
   <223> X= A or V
<220>
   <221> MISC_FEATURE
   <222> (233)..(233)
   <223> X= A, S or V
<220>
   <221> MISC_FEATURE
   <222> (235)..(235)
   <223> X= Y or F
<220>
   <221> MISC_FEATURE
   <222> (238)..(238)
   <223> X=S or A
<220>
   <221> MISC_FEATURE
   <222> (239)..(239)
   <223> X= N or D
<220>
   <221> MISC_FEATURE
   <222> (240)..(240)
   <223> X=M or L
<220>
   <221> MISC_FEATURE
   <222> (243)..(243)
   <223> X=I or V
<220>
   <221> MISC_FEATURE
   <222> (248)..(248)
   <223> X= K or R
<220>
   <221> MISC_FEATURE
   <222> (258)..(258)
   <223> X= D or N
<220>
   <221> MISC_FEATURE
   <222> (261)..(261)
   <223> X= I, S, V or A
<220>
   <221> MISC_FEATURE
   <222> (262)..(262)
   <223> X= P or T
<220>
   <221> MISC_FEATURE
   <222> (269)..(269)
   <223> X= S, G, R or K
<220>
   <221> MISC_FEATURE
   <222> (272)..(272)
   <223> X= S, A or T
<220>
   <221> MISC_FEATURE
   <222> (273)..(273)
   <223> X= R or K
<220>
   <221> MISC_FEATURE
   <222> (276)..(276)
   <223> X= S or A
<220>
   <221> MISC_FEATURE
   <222> (277)..(277)
   <223> X= S or N
<220>
   <221> MISC_FEATURE
   <222> (278)..(278)
   <223> X= R, P, A or T
<220>
   <221> MISC_FEATURE
   <222> (279)..(279)
   <223> X= K, P or A
<220>
   <221> MISC_FEATURE
   <222> (280)..(280)
   <223> X= L or F
<220>
   <221> MISC_FEATURE
   <222> (282)..(282)
   <223> X= E or Q
<220>
   <221> MISC_FEATURE
   <222> (286)..(286)
   <223> X= V or A
<220>
   <221> MISC_FEATURE
   <222> (288)..(288)
   <223> X= K or N
<220>
   <221> MISC_FEATURE
   <222> (289)..(289)
   <223> X= G, S or T
<220>
   <221> MISC_FEATURE
   <222> (290)..(290)
   <223> X= V, M or I
<220>
   <221> MISC_FEATURE
   <222> (293)..(293)
   <223> X= Q or R
<220>
   <221> MISC_FEATURE
   <222> (296)..(296)
   <223> X= G or P
<220>
   <221> MISC_FEATURE
   <222> (297)..(297)
   <223> X= Q or P
<220>
   <221> MISC_FEATURE
   <222> (298)..(298)
   <223> X= M or A
<220>
   <221> MISC_FEATURE
   <222> (300)..(300)
   <223> X= E or Q
<220>
   <221> MISC_FEATURE
   <222> (301)..(301)
   <223> X= L or V
<220>
   <221> MISC_FEATURE
   <222> (307)..(307)
   <223> X= E or V
<220>
   <221> MISC_FEATURE
   <222> (310)..(310)
   <223> X= R or C
<220>
   <221> MISC_FEATURE
   <222> (313)..(313)
   <223> X= I, L or V
<220>
   <221> MISC_FEATURE
   <222> (315)..(315)
   <223> X= G or A
<220>
   <221> MISC_FEATURE
   <222> (316)..(316)
   <223> X= S or T
<220>
   <221> MISC_FEATURE
   <222> (318)..(318)
   <223> X= S or A
<220>
   <221> MISC_FEATURE
   <222> (319)..(319)
   <223> X= S or T
<220>
   <221> MISC_FEATURE
   <222> (336)..(336)
   <223> X= S or N
<220>
   <221> MISC_FEATURE
   <222> (337)..(337)
   <223> X= I or V
<220>
   <221> MISC_FEATURE
   <222> (338)..(338)
   <223> X= D or E
<220>
   <221> MISC_FEATURE
   <222> (341)..(341)
   <223> X= I or V
<220>
   <221> MISC_FEATURE
   <222> (342)..(342)
   <223> X= A, S, or T
<220>
   <221> MISC_FEATURE
   <222> (344)..(344)
   <223> X= L or F
<220>
   <221> MISC_FEATURE
   <222> (345)..(345)
   <223> X= K, Q or N
<220>
   <221> MISC_FEATURE
   <222> (346)..(346)
   <223> X= K or E
<220>
   <221> MISC_FEATURE
   <222> (348)..(348)
   <223> X= K or R
<220>
   <221> MISC_FEATURE
   <222> (349)..(349)
   <223> X= S or A
<220>
   <221> MISC_FEATURE
   <222> (350)..(350)
   <223> X= T or M
<220>
   <221> MISC_FEATURE
   <222> (351)..(351)
   <223> X= K, R or P
<220>
   <221> MISC_FEATURE
   <222> (352)..(352)
   <223> X= T, A or S
<220>
   <221> MISC_FEATURE
   <222> (353)..(353)
   <223> X= T or P
<220>
   <221> MISC_FEATURE
   <222> (358)..(358)
   <223> X= I or V
<220>
   <221> MISC_FEATURE
   <222> (360)..(360)
   <223> X= I or V
<220>
   <221> MISC_FEATURE
   <222> (361)..(361)
   <223> X= I or V
<220>
   <221> MISC_FEATURE
   <222> (366)..(366)
   <223> X= R or K
<220>
   <221> MISC_FEATURE
   <222> (370)..(370)
   <223> X= Y or P
<220>
   <221> MISC_FEATURE
   <222> (373)..(373)
   <223> X= K, R, V or A
<220>
   <221> MISC_FEATURE
   <222> (375)..(375)
   <223> X= D or A
<220>
   <221> MISC_FEATURE
   <222> (377)..(377)
   <223> X= K or R
<220>
   <221> MISC_FEATURE
   <222> (378)..(378)
   <223> X= Y or M
<220>
   <221> MISC_FEATURE
   <222> (382)..(382)
   <223> X= T, A or V
<220>
   <221> MISC_FEATURE
   <222> (387)..(387)
   <223> X= T, A, P or K
<220>
   <221> MISC_FEATURE
   <222> (390)..(390)
   <223> X= R or K
<220>
   <221> MISC_FEATURE
   <222> (394)..(394)
   <223> X= S, T, G or V
<220>
   <221> MISC_FEATURE
   <222> (395)..(395)
   <223> X= S, T or K
<220>
   <221> MISC_FEATURE
   <222> (396)..(396)
   <223> X= S, A, N, F or C
<220>
   <221> MISC_FEATURE
   <222> (397)..(397)
   <223> X= P, E, K, T or S
<220>
   <221> MISC_FEATURE
   <222> (399)..(399)
   <223> X= Q or L
<220>
   <221> MISC_FEATURE
   <222> (403)..(403)
   <223> X= Q or T
<220>
   <221> MISC_FEATURE
   <222> (408)..(408)
   <223> X= A or S
<220>
   <221> MISC_FEATURE
   <222> (410)..(410)
   <223> X= I or V
<220>
   <221> MISC_FEATURE
   <222> (411)..(411)
   <223> X= A, K or R
<220>
   <221> MISC_FEATURE
   <222> (415)..(415)
   <223> X= A, V or I
<220>
   <221> MISC_FEATURE
   <222> (417)..(417)
   <223> X= K, N, E or D
<220>
   <221> MISC_FEATURE
   <222> (418)..(418)
   <223> X= K or D
<220>
   <221> MISC_FEATURE
   <222> (419)..(419)
   <223> X= I or V
<220>
   <221> MISC_FEATURE
   <222> (420)..(420)
   <223> X= I or V
<220>
   <221> MISC_FEATURE
   <222> (421)..(421)
   <223> X= A or G
<220>
   <221> MISC_FEATURE
   <222> (432)..(432)
   <223> X L or M
<220>
   <221> MISC_FEATURE
   <222> (434)..(434)
   <223> X= L, Y, F or I
<220>
   <221> MISC_FEATURE
   <222> (436)..(436)
   <223> X= L, H or Q
<220>
   <221> MISC_FEATURE
   <222> (437)..(437)
   <223> X= R or K
<220>
   <221> MISC_FEATURE
   <222> (438)..(438)
   <223> X= R or K
<220>
   <221> MISC_FEATURE
   <222> (441)..(441)
   <223> X= T, D, N or E
<220>
   <221> MISC_FEATURE
   <222> (462)..(462)
   <223> X= T, C or A
<220>
   <221> MISC_FEATURE
   <222> (465)..(465)
   <223> X= L or I
<220>
   <221> MISC_FEATURE
   <222> (476)..(476)
   <223> X= L or M
<220>
   <221> MISC_FEATURE
   <222> (479)..(479)
   <223> X= A or G
<220>
   <221> MISC_FEATURE
   <222> (491)..(491)
   <223> X= K or R
<220>
   <221> MISC_FEATURE
   <222> (495)..(495)
   <223> X= K or R
<220>
   <221> MISC_FEATURE
   <222> (498)..(498)
   <223> X= M or L
<220>
   <221> MISC_FEATURE
   <222> (514)..(514)
   <223> X= A or S
<220>
   <221> MISC_FEATURE
   <222> (518)..(518)
   <223> X= A or T
<220>
   <221> MISC_FEATURE
   <222> (519)..(519)
   <223> X= F or Y
<220>
   <221> MISC_FEATURE
   <222> (527)..(527)
   <223> X= V or I
<220>
   <221> MISC_FEATURE
   <222> (532)..(532)
   <223> X= A or S
<220>
   <221> MISC_FEATURE
   <222> (535)..(535)
   <223> X= A or T
<220>
   <221> MISC_FEATURE
   <222> (536)..(536)
   <223> X= E or D
<220>
   <221> MISC_FEATURE
   <222> (538)..(538)
   <223> X= F or M
<220>
   <221> MISC_FEATURE
   <222> (539)..(539)
   <223> X= H or N
<220>
   <221> MISC_FEATURE
   <222> (540)..(540)
   <223> X= I or M
<220>
   <221> MISC_FEATURE
   <222> (545)..(545)
   <223> X= A or V
<220>
   <221> MISC_FEATURE
   <222> (546)..(546)
   <223> X= A or K
<220>
   <221> MISC_FEATURE
   <222> (548)..(548)
   <223> X= D or N
<220>
   <221> MISC_FEATURE
   <222> (552)..(552)
   <223> X= S or C
<220>
   <221> MISC_FEATURE
   <222> (556)..(556)
   <223> X= Y or F
<220>
   <221> MISC_FEATURE
   <222> (562)..(562)
   <223> X= V or I
<220>
   <221> MISC_FEATURE
   <222> (564)..(564)
   <223> X= V, A or I
<220>
   <221> MISC_FEATURE
   <222> (565)..(565)
   <223> X= E, A, Q or V
<220>
   <221> MISC_FEATURE
   <222> (568)..(568)
   <223> X= P, A, T, S or V
<220>
   <221> MISC_FEATURE
   <222> (569)..(569)
   <223> X= G, N or D
<220>
   <221> MISC_FEATURE
   <222> (570)..(570)
   <223> X= Y, N or H
<220>
   <221> MISC_FEATURE
   <222> (573)..(573)
   <223> X= I, V or T
<220>
   <221> MISC_FEATURE
   <222> (575)..(575)
   <223> X= I or L
<220>
   <221> MISC_FEATURE
   <222> (577)..(577)
   <223> X= I or V
<220>
   <221> MISC_FEATURE
   <222> (579)..(579)
   <223> X= K or R
<220>
   <221> MISC_FEATURE
   <222> (582)..(582)
   <223> X= I, or V
<220>
   <221> MISC_FEATURE
   <222> (584)..(584)
   <223> X= I, K, M or V
<220>
   <221> MISC_FEATURE
   <222> (585)..(585)
   <223> X= E or G
<220>
   <221> MISC_FEATURE
   <222> (587)..(587)
   <223> X= E, S, D or N
<220>
   <221> MISC_FEATURE
   <222> (588)..(588)
   <223> X= R or K
<220>
   <221> MISC_FEATURE
   <222> (591)..(591)
   <223> X= L or I
<220>
   <221> MISC_FEATURE
   <222> (596)..(596)
   <223> X= T or S
<220>
   <221> MISC_FEATURE
   <222> (597)..(597)
   <223> X= A, I or M
<220>
   <221> MISC_FEATURE
   <222> (600)..(600)
   <223> X= A, S, N or Q
<220>
   <221> MISC_FEATURE
   <222> (602)..(602)
   <223> X= L or M
<220>
   <221> MISC_FEATURE
   <222> (603)..(603)
   <223> X= V, G, A or K
<220>
   <221> MISC_FEATURE
   <222> (605)..(605)
   <223> X= A or S
<220>
   <221> MISC_FEATURE
   <222> (606)..(606)
   <223> X= A, V, E, G or S
<220>
   <221> MISC_FEATURE
   <222> (607)..(607)
   <223> X= A, V, L, M or S
<220>
   <221> MISC_FEATURE
   <222> (608)..(608)
   <223> X= L or V
<220>
   <221> MISC_FEATURE
   <222> (609)..(609)
   <223> X= E, K, R or V
<220>
   <221> MISC_FEATURE
   <222> (610)..(610)
   <223> X= E, Q, S, C or T
<220>
   <221> MISC_FEATURE
   <222> (611)..(611)
   <223> X= H or R
<220>
   <221> MISC_FEATURE
   <222> (612)..(612)
   <223> X= G or N
<220>
   <221> MISC_FEATURE
   <222> (613)..(613)
   <223> X= L, V or I
<220>
   <221> MISC_FEATURE
   <222> (614)..(614)
   <223> X= R, N, Q, S or Y
<220>
   <221> MISC_FEATURE
   <222> (615)..(615)
   <223> X= I, V, P, L or A
<220>
   <221> MISC_FEATURE
   <222> (620)..(620)
   <223> X= A or G
<220>
   <221> MISC_FEATURE
   <222> (621)..(621)
   <223> X= R or K
<220>
   <221> MISC_FEATURE
   <222> (628)..(628)
   <223> X= R, H, G, S or T
<220>
   <221> MISC_FEATURE
   <222> (629)..(629)
   <223> X= A, D, G, S or E
<220>
   <221> MISC_FEATURE
   <222> (632)..(632)
   <223> X= R or K
<220>
   <221> MISC_FEATURE
   <222> (633)..(633)
   <223> X= T, R, S or E
<220>
   <221> MISC_FEATURE
   <222> (635)..(635)
   <223> X= A or V
<220>
   <221> MISC_FEATURE
   <222> (636)..(636)
   <223> X= A, K or N
<220>
   <221> MISC_FEATURE
   <222> (637)..(637)
   <223> X= S or E
<220>
   <221> MISC_FEATURE
   <222> (640)..(640)
   <223> X= V or I
<220>
   <221> MISC_FEATURE
   <222> (642)..(642)
   <223> X= V or I
<220>
   <221> MISC_FEATURE
   <222> (646)..(646)
   <223> X= E or K
<220>
   <221> MISC_FEATURE
   <222> (653)..(653)
   <223> X= G or A
<220>
   <221> MISC_FEATURE
   <222> (657)..(657)
   <223> X= V, T, S or A
<220>
   <221> MISC_FEATURE
   <222> (658)..(658)
   <223> X= Q or H
<220>
   <221> MISC_FEATURE
   <222> (659)..(659)
   <223> X= F or Y
<220>
   <221> MISC_FEATURE
   <222> (660)..(660)
   <223> X= L or M
<220>
   <221> MISC_FEATURE
   <222> (661)..(661)
   <223> X= A or S
<220>
   <221> MISC_FEATURE
   <222> (663)..(663)
   <223> X= N, D, T or S
<220>
   <221> MISC_FEATURE
   <222> (665)..(665)
   <223> X= L or I
<220>
   <221> MISC_FEATURE
   <222> (669)..(669)
   <223> X= T, K, P or H
<220>
   <221> MISC_FEATURE
   <222> (670)..(670)
   <223> X= T, L or I
<220>
   <221> MISC_FEATURE
   <222> (672)..(672)
   <223> X= W, V or L
<220>
   <221> MISC_FEATURE
   <222> (673)..(673)
   <223> X= S or R
<220>
   <221> MISC_FEATURE
   <222> (674)..(674)
   <223> X= S or P
<220>
   <221> MISC_FEATURE
   <222> (675)..(675)
   <223> X= I, L or M
<220>
   <221> MISC_FEATURE
   <222> (676)..(676)
   <223> X= V or F
<220>
   <221> MISC_FEATURE
   <222> (686)..(686)
   <223> X= A or S
<220>
   <221> MISC_FEATURE
   <222> (688)..(688)
   <223> X= A, V or K
<220>
   <221> MISC_FEATURE
   <222> (691)..(691)
   <223> X= L or I
<220>
   <221> MISC_FEATURE
   <222> (692)..(692)
   <223> X= A, E, or S
<220>
   <221> MISC_FEATURE
   <222> (693)..(693)
   <223> X= M, E, or L
<220>
   <221> MISC_FEATURE
   <222> (697)..(697)
   <223> X= M, T, or S
<220>
   <221> MISC_FEATURE
   <222> (698)..(698)
   <223> X= P or S
<220>
   <221> MISC_FEATURE
   <222> (699)..(699)
   <223> X= R, K or S
<220>
   <221> MISC_FEATURE
   <222> (702)..(702)
   <223> X= A or C
<220>
   <221> MISC_FEATURE
   <222> (703)..(703)
   <223> X= A or G
<220>
   <221> MISC_FEATURE
   <222> (705)..(705)
   <223> X= A, V or I
<220>
   <221> MISC_FEATURE
   <222> (706)..(706)
   <223> X= F or L
<220>
   <221> MISC_FEATURE
   <222> (707)..(707)
   <223> X= N or S
<220>
   <221> MISC_FEATURE
   <222> (708)..(708)
   <223> X= I, L or V
<220>
   <221> MISC_FEATURE
   <222> (709)..(709)
   <223> X= I or L
<220>
   <221> MISC_FEATURE
   <222> (710)..(710)
   <223> X=G or R
<220>
   <221> MISC_FEATURE
   <222> (711)..(711)
   <223> X= Q, A, K or R
<220>
   <221> MISC_FEATURE
   <222> (712)..(712)
   <223> X= T or P
<220>
   <221> MISC_FEATURE
   <222> (713)..(713)
   <223> X= L, M, K or R
<220>
   <221> MISC_FEATURE
   <222> (716)..(716)
   <223> X= L or F
<220>
   <221> MISC_FEATURE
   <222> (717)..(717)
   <223> X= Q, E or F
<220>
   <221> MISC_FEATURE
   <222> (718)..(718)
   <223> X= I, V, L or no amino acid
<400> 1
   Met Ala Xaa Xaa Xaa Xaa Xaa Xaa Pro Xaa Xaa Xaa Xaa Xaa Xaa Xaa
   1               5                   10                  15
   Xaa Xaa Xaa Xaa Xaa Xaa Xaa Xaa Xaa Xaa Xaa Xaa Xaa Ser Xaa Xaa
               20                  25                  30
   Xaa Xaa Xaa Xaa Xaa Leu Xaa Xaa Xaa Xaa Xaa Xaa Xaa Xaa Xaa Xaa
           35                  40                  45
   Xaa Xaa Xaa Xaa Arg Xaa Xaa Xaa Val Xaa Ala Ser Xaa Xaa Xaa Xaa
       50                  55                  60
   Xaa Xaa Xaa Xaa Xaa Xaa Xaa Pro Xaa Xaa Pro Xaa Xaa Asp Thr Xaa
   65                  70                  75                  80
   Asn Xaa Pro Xaa His Met Lys Asn Leu Ser Xaa Xaa Xaa Leu Xaa Gln
                   85                  90                  95
   Leu Xaa Xaa Glu Leu Arg Xaa Xaa Xaa Xaa Xaa Xaa Val Xaa Xaa Thr
               100                 105                 110
   Gly Gly His Leu Xaa Xaa Ser Leu Gly Val Val Xaa Leu Xaa Val Xaa
           115                 120                 125
   Leu His Xaa Xaa Phe Xaa Xaa Pro Xaa Asp Xaa Ile Xaa Trp Asp Val
       130                 135                 140
   Gly His Lys Xaa Tyr Xaa His Lys Ile Leu Thr Gly Arg Arg Xaa Xaa
   145                 150                 155                 160
   Met Xaa Thr Xaa Arg Gln Thr Xaa Gly Leu Xaa Gly Phe Xaa Lys Arg
                   165                 170                 175
   Xaa Glu Ser Xaa Xaa Asp Xaa Phe Gly Xaa Gly His Ser Ser Thr Xaa
               180                 185                 190
   Ile Ser Ala Xaa Leu Gly Met Ala Val Xaa Arg Asp Xaa Xaa Gly Xaa
           195                 200                 205
   Xaa Asn Xaa Val Xaa Xaa Val Ile Gly Asp Gly Xaa Met Thr Ala Gly
       210                 215                 220
   Gln Ala Tyr Glu Ala Met Asn Asn Xaa Gly Xaa Leu Asp Xaa Xaa Xaa
   225                 230                 235                 240
   Ile Val Xaa Leu Asn Asp Asn Xaa Gln Val Ser Leu Pro Thr Ala Thr
                   245                 250                 255
   Leu Xaa Gly Pro Xaa Xaa Pro Val Gly Ala Leu Ser Xaa Ala Leu Xaa
               260                 265                 270
   Xaa Leu Gln Xaa Xaa Xaa Xaa Xaa Arg Xaa Leu Arg Glu Xaa Ala Xaa
           275                 280                 285
   Xaa Xaa Thr Lys Xaa Ile Gly Xaa Xaa Xaa His Xaa Xaa Ala Ala Lys
   290 295 300
   Val Asp Xaa Tyr Ala Xaa Gly Met Xaa Ser Xaa Xaa Gly Xaa Xaa Leu
   305 310 315 320
   Phe Glu Glu Leu Gly Leu Tyr Tyr Ile Gly Pro Val Asp Gly His Xaa
   325 330 335
   Xaa Xaa Asp Leu Xaa Xaa Ile Xaa Xaa Xaa Val Xaa Xaa Xaa Xaa Xaa
   340 345 350
   Xaa Gly Pro Val Leu Xaa His Xaa Xaa Thr Glu Lys Gly Xaa Gly Tyr
   355 360 365
   Pro Xaa Ala Glu Xaa Ala Xaa Asp Xaa Xaa His Gly Val Xaa Lys Phe
   370 375 380
   Asp Pro Xaa Thr Gly Xaa Gln Phe Lys Xaa Xaa Xaa Xaa Thr Xaa Ser
   385 390 395 400
   Tyr Thr Xaa Tyr Phe Ala Glu Xaa Leu Xaa Xaa Glu Ala Glu Xaa Asp
   405 410 415
   Xaa Xaa Xaa Xaa Xaa Ile His Ala Ala Met Gly Gly Gly Thr Gly Xaa
   420 425 430
   Asn Xaa Phe Xaa Xaa Xaa Phe Pro Xaa Arg Cys Phe Asp Val Gly Ile
   435 440 445
   Ala Glu Gln His Ala Val Thr Phe Ala Ala Gly Leu Ala Xaa Glu Gly
   450 455 460
   Xaa Lys Pro Phe Cys Ala Ile Tyr Ser Ser Phe Xaa Gln Arg Xaa Tyr
   465 470 475 480
   Asp Gln Val Val His Asp Val Asp Leu Gln Xaa Leu Pro Val Xaa Phe
   485 490 495
   Ala Xaa Asp Arg Ala Gly Leu Val Gly Ala Asp Gly Pro Thr His Cys
   500 505 510
   Gly Xaa Phe Asp Val Xaa Xaa Met Ala Cys Leu Pro Asn Met Xaa Val
   515 520 525
   Met Ala Pro Xaa Asp Glu Xaa Xaa Leu Xaa Xaa Xaa Val Ala Thr Ala
   530 535 540
   Xaa Xaa Ile Xaa Asp Arg Pro Xaa Cys Phe Arg Xaa Pro Arg Gly Asn
   545 550 555 560
   Gly Xaa Gly Xaa Xaa Leu Pro Xaa Xaa Xaa Lys Gly Xaa Pro Xaa Glu
   565 570 575
   Xaa Gly Xaa Gly Arg Xaa Leu Xaa Xaa Gly Xaa Xaa Val Ala Xaa Leu
   580 585 590
   Gly Tyr Gly Xaa Xaa Val Gln Xaa Cys Xaa Xaa Ala Xaa Xaa Xaa Xaa
   595 600 605
   Xaa Xaa Xaa Xaa Xaa Xaa Xaa Thr Val Ala Asp Xaa Xaa Phe Cys Lys
   610 615 620
   Pro Leu Asp Xaa Xaa Leu Ile Xaa Xaa Leu Xaa Xaa Xaa His Glu Xaa
   625 630 635 640
   Leu Xaa Thr Val Glu Xaa Gly Ser Ile Gly Gly Phe Xaa Ser His Val
   645 650 655
   Xaa Xaa Xaa Xaa Xaa Leu Xaa Gly Xaa Leu Asp Gly Xaa Xaa Lys Xaa
   660 665 670
   Xaa Xaa Xaa Xaa Leu Pro Asp Arg Tyr Ile Asp His Gly Xaa Pro Xaa
   675 680 685
   Asp Gln Xaa Xaa Xaa Ala Gly Leu Xaa Xaa Xaa His Ile Xaa Xaa Thr
   690 695 700
   Xaa Xaa Xaa Xaa Xaa Xaa Xaa Xaa Xaa Glu Ala Xaa Xaa Xaa Met Leu
   705 710 715 720
<210> 2
   <211> 716
   <212> PRT
   <213> Vitis Vinifera
<220>
   <221> misc_feature
   <222> (284)..(284)
   <223> Xaa can be any naturally occurring amino acid
<220>
   <221> misc_feature
   <222> (306)..(306)
   <223> Xaa can be any naturally occurring amino acid
<400> 2
   Met Ala Leu Cys Thr Leu Ser Phe Pro Ala His Phe Ser Gln Ala Ala
   1               5                   10                  15
   Ala Ser Asn Pro Gln Arg Leu Thr Pro Gln Cys Ser His Leu Phe Leu
               20                  25                  30
   Gly Val Asp Leu Gln Cys Gln Ser Gln Gln Arg Ser Lys Ala Arg Lys
           35                  40                  45
   Arg Pro Asn Gly Val Cys Ala Ser Leu Ser Asp Arg Glu Glu Tyr His
       50                  55                  60
   Ser Gln Arg Pro Pro Thr Pro Leu Leu Asp Thr Ile Asn Tyr Pro Ile
   65                  70                  75                  80
   His Met Lys Asn Leu Ser Val Lys Glu Leu Lys Gln Leu Ala Asp Glu
                   85                  90                  95
   Leu Arg Ser Asp Val Val Phe Asn Val Ser Lys Thr Gly Gly His Leu
               100                 105                 110
   Gly Ser Ser Leu Gly Val Val Glu Leu Thr Val Ala Leu His Tyr Val
           115                 120                 125
   Phe Asn Ala Pro Gln Asp Arg Ile Leu Trp Asp Val Gly His Gln Ser
       130                 135                 140
   Tyr Pro His Lys Ile Leu Thr Gly Arg Arg Asp Gln Met His Thr Met
   145                 150                 155                 160
   Arg Gln Thr Asp Gly Leu Ala Gly Phe Thr Lys Arg Ser Glu Ser Glu
                   165                 170                 175
   Tyr Asp Cys Phe Gly Thr Gly His Ser Ser Thr Thr Ile Ser Ala Gly
               180                 185                 190
   Leu Gly Met Ala Val Gly Arg Asp Leu Lys Gly Lys Asn Asn Asn Val
           195                 200                 205
   Ile Ala Val Ile Gly Asp Gly Ala Met Thr Ala Gly Gln Ala Tyr Glu
       210                 215                 220
   Ala Met Asn Asn Ala Gly Tyr Leu Asp Ser Asp Met Ile Val Ile Leu
   225                 230                 235                 240
   Asn Asp Asn Lys Gln Val Ser Leu Pro Thr Ala Thr Leu Asp Gly Pro
                   245                 250                 255
   Ile Pro Pro Val Gly Ala Leu Ser Ser Ala Leu Ser Arg Leu Gln Ser
               260                 265                 270
   Asn Arg Pro Leu Arg Glu Leu Arg Glu Val Ala Xaa Gly Val Thr Lys
           275                 280                 285
   Gln Ile Gly Gly Pro Met His Glu Leu Ala Ala Lys Val Asp Glu Tyr
       290                 295                 300
   Ala Xaa Gly Met Ile Ser Gly Ser Gly Ser Thr Leu Phe Glu Glu Leu
   305                 310                 315                 320
   Gly Leu Tyr Tyr Ile Gly Pro Val Asp Gly His Asn Ile Asp Asp Leu
   325 330 335
   Val Ala Ile Leu Lys Glu Val Lys Ser Thr Lys Thr Thr Gly Pro Val
   340 345 350
   Leu Ile His Val Val Thr Glu Lys Gly Arg Gly Tyr Pro Tyr Ala Glu
   355 360 365
   Lys Ala Ala Asp Lys Tyr His Gly Val Thr Lys Phe Asp Pro Ala Thr
   370 375 380
   Gly Lys Gln Phe Lys Ser Ser Ala Pro Thr Gln Ser Tyr Thr Thr Tyr
   385 390 395 400
   Phe Ala Glu Ala Leu Ile Ala Glu Ala Glu Val Asp Lys Asp Ile Val
   405 410 415
   Ala Ile His Ala Ala Met Gly Gly Gly Thr Gly Leu Asn Leu Phe His
   420 425 430
   Arg Arg Phe Pro Thr Arg Cys Phe Asp Val Gly Ile Ala Glu Gln His
   435 440 445
   Ala Val Thr Phe Ala Ala Gly Leu Ala Cys Glu Gly Ile Lys Pro Phe
   450 455 460
   Cys Ala Ile Tyr Ser Ser Phe Met Gln Arg Ala Tyr Asp Gln Val Val
   465 470 475 480
   His Asp Val Asp Leu Gln Lys Leu Pro Val Lys Phe Ala Met Asp Arg
   485 490 495
   Ala Gly Leu Val Gly Ala Asp Gly Pro Thr His Cys Gly Ala Phe Asp
   500 505 510
   Val Ala Phe Met Ala Cys Leu Pro Asn Met Val Val Met Ala Pro Ala
   515 520 525
   Asp Glu Ala Glu Leu Phe His Met Val Ala Thr Ala Ala Ala Ile Asp
   530 535 540
   Asp Arg Pro Ser Cys Phe Arg Tyr Pro Arg Gly Asn Gly Val Gly Val
   545 550 555 560
   Glu Leu Pro Pro Gly Asn Lys Gly Ile Pro Ile Glu Val Gly Arg Gly
   565 570 575
   Arg Ile Leu Ile Glu Gly Glu Arg Val Ala Leu Leu Gly Tyr Gly Thr
   580 585 590
   Ala Val Gln Ser Cys Leu Val Ala Ser Ser Leu Leu Glu Gln His Gly
   595 600 605
   Leu Arg Ile Thr Val Ala Asp Ala Arg Phe Cys Lys Pro Leu Asp His
   610 615 620
   Ala Leu Ile Arg Ser Leu Ala Lys Ser His Glu Val Leu Ile Thr Val
   625 630 635 640
   Glu Glu Gly Ser Ile Gly Gly Phe Gly Ser His Val Ala Gln Phe Leu
   645 650 655
   Ala Leu Asn Gly Leu Leu Asp Gly Thr Thr Lys Trp Ser Pro Met Val
   660 665 670
   Leu Pro Asp Arg Tyr Ile Asp His Gly Ala Pro Ala Asp Gln Leu Ala
   675 680 685
   Met Ala Gly Leu Thr Pro Ser His Ile Ala Ala Thr Val Phe Asn Ile
   690 695 700
   Leu Gly Gln Thr Arg Glu Ala Leu Glu Ile Met Leu
   705 710 715
<210> 3
   <211> 716
   <212> PRT
   <213> Vitis Vinifera
<400> 3
   Met Ala Leu Cys Thr Leu Ser Phe Pro Ala His Phe Ser Gln Ala Ala
   1 5 10 15
   Ala Ser Asn Pro Gln Arg Leu Thr Pro Gln Cys Ser His Leu Phe Leu
   20 25 30
   Gly Val Asp Leu Gln Cys Gln Ser Gln Gln Arg Ser Lys Ala Arg Lys
   35 40 45
   Arg Pro Asn Gly Val Cys Ala Ser Leu Ser Asp Arg Glu Glu Tyr His
   50 55 60
   Ser Gln Arg Pro Pro Thr Pro Leu Leu Asp Thr Ile Asn Tyr Pro Ile
   65 70 75 80
   His Met Lys Asn Leu Ser Val Lys Glu Leu Lys Gln Leu Ala Asp Glu
   85 90 95
   Leu Arg Ser Asp Val Val Phe Asn Val Ser Lys Thr Gly Gly His Leu
   100 105 110
   Gly Ser Ser Leu Gly Val Val Glu Leu Thr Val Ala Leu His Tyr Val
   115 120 125
   Phe Asn Ala Pro Gln Asp Arg Ile Leu Trp Asp Val Gly His Gln Ser
   130 135 140
   Tyr Pro His Lys Ile Leu Thr Gly Arg Arg Asp Gln Met His Thr Met
   145 150 155 160
   Arg Gln Thr Asp Gly Leu Ala Gly Phe Thr Lys Arg Ser Glu Ser Glu
   165 170 175
   Tyr Asp Cys Phe Gly Thr Gly His Ser Ser Thr Thr Ile Ser Ala Gly
   180 185 190
   Leu Gly Met Ala Val Gly Arg Asp Leu Lys Gly Lys Asn Asn Asn Val
   195 200 205
   Ile Ala Val Ile Gly Asp Gly Ala Met Thr Ala Gly Gln Ala Tyr Glu
   210 215 220
   Ala Met Asn Asn Ala Gly Tyr Leu Asp Ser Asp Met Ile Val Ile Leu
   225 230 235 240
   Asn Asp Asn Lys Gln Val Ser Leu Pro Thr Ala Thr Leu Asp Gly Pro
   245 250 255
   Ile Pro Pro Val Gly Ala Leu Ser Ser Ala Leu Ser Arg Leu Gln Ser
   260 265 270
   Asn Arg Pro Leu Arg Glu Leu Arg Glu Val Ala Lys Gly Val Thr Lys
   275 280 285
   Gln Ile Gly Gly Pro Met His Glu Leu Ala Ala Lys Val Asp Glu Tyr
   290 295 300
   Ala Arg Gly Met Ile Ser Gly Ser Gly Ser Thr Leu Phe Glu Glu Leu
   305 310 315 320
   Gly Leu Tyr Tyr Ile Gly Pro Val Asp Gly His Asn Ile Asp Asp Leu
   325 330 335
   Val Ala Ile Leu Lys Glu Val Lys Ser Thr Lys Thr Thr Gly Pro Val
   340 345 350
   Leu Ile His Val Val Thr Glu Lys Gly Arg Gly Tyr Pro Tyr Ala Glu
   355 360 365
   Lys Ala Ala Asp Lys Tyr His Gly Val Thr Lys Phe Asp Pro Ala Thr
   370 375 380
   Gly Lys Gln Phe Lys Ser Ser Ala Pro Thr Gln Ser Tyr Thr Thr Tyr
   385                 390                 395                 400
   Phe Ala Glu Ala Leu Ile Ala Glu Ala Glu Val Asp Lys Asp Ile Val
                   405                 410                 415
   Ala Ile His Ala Ala Met Gly Gly Gly Thr Gly Leu Asn Leu Phe His
               420                 425                 430
   Arg Arg Phe Pro Thr Arg Cys Phe Asp Val Gly Ile Ala Glu Gln His
           435                 440                 445
   Ala Val Thr Phe Ala Ala Gly Leu Ala Cys Glu Gly Ile Lys Pro Phe
       450                 455                 460
   Cys Ala Ile Tyr Ser Ser Phe Met Gln Arg Ala Tyr Asp Gln Val Val
   465                 470                 475                 480
   His Asp Val Asp Leu Gln Lys Leu Pro Val Lys Phe Ala Met Asp Arg
                   485                 490                 495
   Ala Gly Leu Val Gly Ala Asp Gly Pro Thr His Cys Gly Ala Phe Asp
               500                 505                 510
   Val Ala Phe Met Ala Cys Leu Pro Asn Met Val Val Met Ala Pro Ala
           515                 520                 525
   Asp Glu Ala Glu Leu Phe His Met Val Ala Thr Ala Ala Ala Ile Asp
       530                 535                 540
   Asp Arg Pro Ser Cys Phe Arg Tyr Pro Arg Gly Asn Gly Val Gly Val
   545                 550                 555                 560
   Glu Leu Pro Pro Gly Asn Lys Gly Ile Pro Ile Glu Val Gly Arg Gly
                   565                 570                 575
   Arg Ile Leu Ile Glu Gly Glu Arg Val Ala Leu Leu Gly Tyr Gly Thr
               580                 585                 590
   Ala Val Gln Ser Cys Leu Val Ala Ser Ser Leu Leu Glu Gln His Gly
           595                 600                 605
   Leu Arg Ile Thr Val Ala Asp Ala Arg Phe Cys Lys Pro Leu Asp His
       610                 615                 620
   Ala Leu Ile Arg Ser Leu Ala Lys Ser His Glu Val Leu Ile Thr Val
   625                 630                 635                 640
   Glu Glu Gly Ser Ile Gly Gly Phe Gly Ser His Val Ala Gln Phe Leu
                   645                 650                 655
   Ala Leu Asn Gly Leu Leu Asp Gly Thr Thr Lys Trp Ser Pro Met Val
   660 665 670
   Leu Pro Asp Arg Tyr Ile Asp His Gly Ala Pro Ala Asp Gln Leu Ala
   675 680 685
   Met Ala Gly Leu Thr Pro Ser His Ile Ala Ala Thr Val Phe Asn Ile
   690 695 700
   Leu Gly Gln Thr Arg Glu Ala Leu Glu Ile Met Leu
   705 710 715
<210> 4
   <211> 716
   <212> PRT
   <213> Vitis Vinifera
<400> 4
   Met Ala Leu Cys Thr Leu Ser Phe Pro Ala His Phe Ser Gln Ala Ala
   1 5 10 15
   Ala Ser Asn Pro Gln Arg Leu Thr Pro Gln Cys Ser His Leu Phe Leu
   20 25 30
   Gly Val Asp Leu Gln Cys Gln Ser Gln Gln Arg Ser Lys Ala Arg Lys
   35 40 45
   Arg Pro Asn Gly Val Cys Ala Ser Leu Ser Asp Arg Glu Glu Tyr His
   50 55 60
   Ser Gln Arg Pro Pro Thr Pro Leu Leu Asp Thr Ile Asn Tyr Pro Ile
   65 70 75 80
   His Met Lys Asn Leu Ser Val Lys Glu Leu Lys Gln Leu Ala Asp Glu
   85 90 95
   Leu Arg Ser Asp Val Val Phe Asn Val Ser Lys Thr Gly Gly His Leu
   100 105 110
   Gly Ser Ser Leu Gly Val Val Glu Leu Thr Val Ala Leu His Tyr Val
   115 120 125
   Phe Asn Ala Pro Gln Asp Arg Ile Leu Trp Asp Val Gly His Gln Ser
   130 135 140
   Tyr Pro His Lys Ile Leu Thr Gly Arg Arg Asp Gln Met His Thr Met
   145 150 155 160
   Arg Gln Thr Asp Gly Leu Ala Gly Phe Thr Lys Arg Ser Glu Ser Glu
   165 170 175
   Tyr Asp Cys Phe Gly Thr Gly His Ser Ser Thr Thr Ile Ser Ala Gly
   180 185 190
   Leu Gly Met Ala Val Gly Arg Asp Leu Lys Gly Lys Asn Asn Asn Val
   195 200 205
   Ile Ala Val Ile Gly Asp Gly Ala Met Thr Ala Gly Gln Ala Tyr Glu
   210 215 220
   Ala Met Asn Asn Ala Gly Tyr Leu Asp Ser Asp Met Ile Val Ile Leu
   225 230 235 240
   Asn Asp Asn Lys Gln Val Ser Leu Pro Thr Ala Thr Leu Asp Gly Pro
   245 250 255
   Ile Pro Pro Val Gly Ala Leu Ser Ser Ala Leu Ser Arg Leu Gln Ser
   260 265 270
   Asn Arg Pro Leu Arg Glu Leu Arg Glu Val Ala Asn Gly Val Thr Lys
   275 280 285
   Gln Ile Gly Gly Pro Met His Glu Leu Ala Ala Lys Val Asp Glu Tyr
   290 295 300
   Ala Arg Gly Met Ile Ser Gly Ser Gly Ser Thr Leu Phe Glu Glu Leu
   305 310 315 320
   Gly Leu Tyr Tyr Ile Gly Pro Val Asp Gly His Asn Ile Asp Asp Leu
   325 330 335
   Val Ala Ile Leu Lys Glu Val Lys Ser Thr Lys Thr Thr Gly Pro Val
   340 345 350
   Leu Ile His Val Val Thr Glu Lys Gly Arg Gly Tyr Pro Tyr Ala Glu
   355 360 365
   Lys Ala Ala Asp Lys Tyr His Gly Val Thr Lys Phe Asp Pro Ala Thr
   370 375 380
   Gly Lys Gln Phe Lys Ser Ser Ala Pro Thr Gln Ser Tyr Thr Thr Tyr
   385 390 395 400
   Phe Ala Glu Ala Leu Ile Ala Glu Ala Glu Val Asp Lys Asp Ile Val
   405 410 415
   Ala Ile His Ala Ala Met Gly Gly Gly Thr Gly Leu Asn Leu Phe His
   420 425 430
   Arg Arg Phe Pro Thr Arg Cys Phe Asp Val Gly Ile Ala Glu Gln His
   435 440 445
   Ala Val Thr Phe Ala Ala Gly Leu Ala Cys Glu Gly Ile Lys Pro Phe
   450 455 460
   Cys Ala Ile Tyr Ser Ser Phe Met Gln Arg Ala Tyr Asp Gln Val Val
   465 470 475 480
   His Asp Val Asp Leu Gln Lys Leu Pro Val Lys Phe Ala Met Asp Arg
   485 490 495
   Ala Gly Leu Val Gly Ala Asp Gly Pro Thr His Cys Gly Ala Phe Asp
   500 505 510
   Val Ala Phe Met Ala Cys Leu Pro Asn Met Val Val Met Ala Pro Ala
   515 520 525
   Asp Glu Ala Glu Leu Phe His Met Val Ala Thr Ala Ala Ala Ile Asp
   530 535 540
   Asp Arg Pro Ser Cys Phe Arg Tyr Pro Arg Gly Asn Gly Val Gly Ile
   545 550 555 560
   Glu Leu Pro Pro Gly Asn Lys Gly Ile Pro Ile Glu Val Gly Arg Gly
   565 570 575
   Arg Ile Leu Ile Glu Gly Glu Arg Val Ala Leu Leu Gly Tyr Gly Thr
   580 585 590
   Ala Val Gln Ser Cys Leu Val Ala Ser Ser Leu Leu Glu Gln His Gly
   595 600 605
   Leu Arg Ile Thr Val Ala Asp Ala Arg Phe Cys Lys Pro Leu Asp His
   610 615 620
   Ala Leu Ile Arg Ser Leu Ala Lys Ser His Glu Val Leu Ile Thr Val
   625 630 635 640
   Glu Glu Gly Ser Ile Gly Gly Phe Gly Ser His Val Ala Gln Phe Leu
   645 650 655
   Ala Leu Asn Gly Leu Leu Asp Gly Thr Thr Lys Trp Ser Pro Met Val
   660 665 670
   Leu Pro Asp Arg Tyr Ile Asp His Gly Ala Pro Ala Asp Gln Leu Ala
   675 680 685
   Met Ala Gly Leu Thr Pro Ser His Ile Ala Ala Thr Val Phe Asn Ile
   690 695 700
   Leu Gly Gln Thr Arg Glu Ala Leu Glu Ile Met Leu
   705 710 715
<210> 5
   <211> 664
   <212> PRT
   <213> Artificial sequence
<220>
   <223> Truncated DXS polypeptide
<400> 5
   Val Cys Ala Ser Leu Ser Asp Arg Glu Glu Tyr His Ser Gln Arg Pro
   1               5                   10                  15
   Pro Thr Pro Leu Leu Asp Thr Ile Asn Tyr Pro Ile His Met Lys Asn
               20                  25                  30
   Leu Ser Val Lys Glu Leu Lys Gln Leu Ala Asp Glu Leu Arg Ser Asp
           35                  40                  45
   Val Val Phe Asn Val Ser Lys Thr Gly Gly His Leu Gly Ser Ser Leu
       50                  55                  60
   Gly Val Val Glu Leu Thr Val Ala Leu His Tyr Val Phe Asn Ala Pro
   65                  70                  75                  80
   Gln Asp Arg Ile Leu Trp Asp Val Gly His Gln Ser Tyr Pro His Lys
                   85                  90                  95
   Ile Leu Thr Gly Arg Arg Asp Gln Met His Thr Met Arg Gln Thr Asp
               100                 105                 110
   Gly Leu Ala Gly Phe Thr Lys Arg Ser Glu Ser Glu Tyr Asp Cys Phe
           115                 120                 125
   Gly Thr Gly His Ser Ser Thr Thr Ile Ser Ala Gly Leu Gly Met Ala
       130                 135                 140
   Val Gly Arg Asp Leu Lys Gly Lys Asn Asn Asn Val Ile Ala Val Ile
   145                 150                 155                 160
   Gly Asp Gly Ala Met Thr Ala Gly Gln Ala Tyr Glu Ala Met Asn Asn
                   165                 170                 175
   Ala Gly Tyr Leu Asp Ser Asp Met Ile Val Ile Leu Asn Asp Asn Lys
               180                 185                 190
   Gln Val Ser Leu Pro Thr Ala Thr Leu Asp Gly Pro Ile Pro Pro Val
           195                 200                 205
   Gly Ala Leu Ser Ser Ala Leu Ser Arg Leu Gln Ser Asn Arg Pro Leu
       210                 215                 220
   Arg Glu Leu Arg Glu Val Ala Asn Gly Val Thr Lys Gln Ile Gly Gly
   225                 230                 235                 240
   Pro Met His Glu Leu Ala Ala Lys Val Asp Glu Tyr Ala Arg Gly Met
   245 250 255
   Ile Ser Gly Ser Gly Ser Thr Leu Phe Glu Glu Leu Gly Leu Tyr Tyr
   260 265 270
   Ile Gly Pro Val Asp Gly His Asn Ile Asp Asp Leu Val Ala Ile Leu
   275 280 285
   Lys Glu Val Lys Ser Thr Lys Thr Thr Gly Pro Val Leu Ile His Val
   290 295 300
   Val Thr Glu Lys Gly Arg Gly Tyr Pro Tyr Ala Glu Lys Ala Ala Asp
   305 310 315 320
   Lys Tyr His Gly Val Thr Lys Phe Asp Pro Ala Thr Gly Lys Gln Phe
   325 330 335
   Lys Ser Ser Ala Pro Thr Gln Ser Tyr Thr Thr Tyr Phe Ala Glu Ala
   340 345 350
   Leu Ile Ala Glu Ala Glu Val Asp Lys Asp Ile Val Ala Ile His Ala
   355 360 365
   Ala Met Gly Gly Gly Thr Gly Leu Asn Leu Phe His Arg Arg Phe Pro
   370 375 380
   Thr Arg Cys Phe Asp Val Gly Ile Ala Glu Gln His Ala Val Thr Phe
   385 390 395 400
   Ala Ala Gly Leu Ala Cys Glu Gly Ile Lys Pro Phe Cys Ala Ile Tyr
   405 410 415
   Ser Ser Phe Met Gln Arg Ala Tyr Asp Gln Val Val His Asp Val Asp
   420 425 430
   Leu Gln Lys Leu Pro Val Lys Phe Ala Met Asp Arg Ala Gly Leu Val
   435 440 445
   Gly Ala Asp Gly Pro Thr His Cys Gly Ala Phe Asp Val Ala Phe Met
   450 455 460
   Ala Cys Leu Pro Asn Met Val Val Met Ala Pro Ala Asp Glu Ala Glu
   465 470 475 480
   Leu Phe His Met Val Ala Thr Ala Ala Ala Ile Asp Asp Arg Pro Ser
   485 490 495
   Cys Phe Arg Tyr Pro Arg Gly Asn Gly Val Gly Ile Glu Leu Pro Pro
   500 505 510
   Gly Asn Lys Gly Ile Pro Ile Glu Val Gly Arg Gly Arg Ile Leu Ile
           515                 520                 525
   Glu Gly Glu Arg Val Ala Leu Leu Gly Tyr Gly Thr Ala Val Gln Ser
       530                 535                 540
   Cys Leu Val Ala Ser Ser Leu Leu Glu Gln His Gly Leu Arg Ile Thr
   545                 550                 555                 560
   Val Ala Asp Ala Arg Phe Cys Lys Pro Leu Asp His Ala Leu Ile Arg
                   565                 570                 575
   Ser Leu Ala Lys Ser His Glu Val Leu Ile Thr Val Glu Glu Gly Ser
               580                 585                 590
   Ile Gly Gly Phe Gly Ser His Val Ala Gln Phe Leu Ala Leu Asn Gly
           595                 600                 605
   Leu Leu Asp Gly Thr Thr Lys Trp Ser Pro Met Val Leu Pro Asp Arg
       610                 615                 620
   Tyr Ile Asp His Gly Ala Pro Ala Asp Gln Leu Ala Met Ala Gly Leu
   625                 630                 635                 640
   Thr Pro Ser His Ile Ala Ala Thr Val Phe Asn Ile Leu Gly Gln Thr
                   645                 650                 655
   Arg Glu Ala Leu Glu Ile Met Leu
               660
<210> 6
   <211> 716
   <212> PRT
   <213> Vitis Vinifera
<400> 6
   Met Ala Leu Cys Thr Leu Ser Phe Pro Ala His Phe Ser Gln Ala Ala
   1               5                   10                  15
   Ala Ser Asn Pro Gln Arg Leu Thr Pro Gln Cys Ser His Leu Phe Leu
               20                  25                  30
   Gly Val Asp Leu Gln Cys Gln Ser Gln Gln Arg Ser Lys Ala Arg Lys
           35                  40                  45
   Arg Pro Asn Gly Val Cys Ala Ser Leu Ser Asp Arg Glu Glu Tyr His
       50                  55                  60
   Ser Gln Arg Pro Pro Thr Pro Leu Leu Asp Thr Ile Asn Tyr Pro Ile
   65                  70                  75                  80
   His Met Lys Asn Leu Ser Val Lys Glu Leu Lys Gln Leu Ala Asp Glu
   85 90 95
   Leu Arg Ser Asp Val Val Phe Asn Val Ser Lys Thr Gly Gly His Leu
   100 105 110
   Gly Ser Ser Leu Gly Val Val Glu Leu Thr Val Ala Leu His Tyr Val
   115 120 125
   Phe Asn Ala Pro Gln Asp Arg Ile Leu Trp Asp Val Gly His Gln Ser
   130 135 140
   Tyr Pro His Lys Ile Leu Thr Gly Arg Arg Asp Gln Met His Thr Met
   145 150 155 160
   Arg Gln Thr Asp Gly Leu Ala Gly Phe Thr Lys Arg Ser Glu Ser Glu
   165 170 175
   Tyr Asp Cys Phe Gly Thr Gly His Ser Ser Thr Thr Ile Ser Ala Gly
   180 185 190
   Leu Gly Met Ala Val Gly Arg Asp Leu Lys Gly Lys Asn Asn Asn Val
   195 200 205
   Ile Ala Val Ile Gly Asp Gly Ala Met Thr Ala Gly Gln Ala Tyr Glu
   210 215 220
   Ala Met Asn Asn Ala Gly Tyr Leu Asp Ser Asp Met Ile Val Ile Leu
   225 230 235 240
   Asn Asp Asn Lys Gln Val Ser Leu Pro Thr Ala Thr Leu Asp Gly Pro
   245 250 255
   Ile Pro Pro Val Gly Ala Leu Ser Ser Ala Leu Ser Arg Leu Gln Ser
   260 265 270
   Asn Arg Pro Leu Arg Glu Leu Arg Glu Val Ala Lys Gly Val Thr Lys
   275 280 285
   Gln Ile Gly Gly Pro Met His Glu Leu Ala Ala Lys Val Asp Glu Tyr
   290 295 300
   Ala Cys Gly Met Ile Ser Gly Ser Gly Ser Thr Leu Phe Glu Glu Leu
   305 310 315 320
   Gly Leu Tyr Tyr Ile Gly Pro Val Asp Gly His Asn Ile Asp Asp Leu
   325 330 335
   Val Ala Ile Leu Lys Glu Val Lys Ser Thr Lys Thr Thr Gly Pro Val
   340 345 350
   Leu Ile His Val Val Thr Glu Lys Gly Arg Gly Tyr Pro Tyr Ala Glu
   355 360 365
   Lys Ala Ala Asp Lys Tyr His Gly Val Thr Lys Phe Asp Pro Ala Thr
   370 375 380
   Gly Lys Gln Phe Lys Ser Ser Ala Pro Thr Gln Ser Tyr Thr Thr Tyr
   385 390 395 400
   Phe Ala Glu Ala Leu Ile Ala Glu Ala Glu Val Asp Lys Asp Ile Val
   405 410 415
   Ala Ile His Ala Ala Met Gly Gly Gly Thr Gly Leu Asn Leu Phe His
   420 425 430
   Arg Arg Phe Pro Thr Arg Cys Phe Asp Val Gly Ile Ala Glu Gln His
   435 440 445
   Ala Val Thr Phe Ala Ala Gly Leu Ala Cys Glu Gly Ile Lys Pro Phe
   450 455 460
   Cys Ala Ile Tyr Ser Ser Phe Met Gln Arg Ala Tyr Asp Gln Val Val
   465 470 475 480
   His Asp Val Asp Leu Gln Lys Leu Pro Val Lys Phe Ala Met Asp Arg
   485 490 495
   Ala Gly Leu Val Gly Ala Asp Gly Pro Thr His Cys Gly Ala Phe Asp
   500 505 510
   Val Ala Phe Met Ala Cys Leu Pro Asn Met Val Val Met Ala Pro Ala
   515 520 525
   Asp Glu Ala Glu Leu Phe His Met Val Ala Thr Ala Ala Ala Ile Asp
   530 535 540
   Asp Arg Pro Ser Cys Phe Arg Tyr Pro Arg Gly Asn Gly Val Gly Val
   545 550 555 560
   Glu Leu Pro Pro Gly Asn Lys Gly Ile Pro Ile Glu Val Gly Arg Gly
   565 570 575
   Arg Ile Leu Ile Glu Gly Glu Arg Val Ala Leu Leu Gly Tyr Gly Thr
   580 585 590
   Ala Val Gln Ser Cys Leu Val Ala Ser Ser Leu Leu Glu Gln His Gly
   595 600 605
   Leu Arg Ile Thr Val Ala Asp Ala Arg Phe Cys Lys Pro Leu Asp His
   610 615 620
   Ala Leu Ile Arg Ser Leu Ala Lys Ser His Glu Val Leu Ile Thr Val
   625                 630                 635                 640
   Glu Glu Gly Ser Ile Gly Gly Phe Gly Ser His Val Ala Gln Phe Leu
                   645                 650                 655
   Ala Leu Asn Gly Leu Leu Asp Gly Thr Thr Lys Trp Ser Pro Met Val
               660                 665                 670
   Leu Pro Asp Arg Tyr Ile Asp His Gly Ala Pro Ala Asp Gln Leu Ala
           675                 680                 685
   Met Ala Gly Leu Thr Pro Ser His Ile Ala Ala Thr Val Phe Asn Ile
       690                 695                 700
   Leu Gly Gln Thr Arg Glu Ala Leu Glu Ile Met Leu
   705                 710                 715
<210> 7
   <211> 664
   <212> PRT
   <213> Artificial sequence
<220>
   <223> Truncated DXS polypeptide
<400> 7
   Val Cys Ala Ser Leu Ser Asp Arg Glu Glu Tyr His Ser Gln Arg Pro
   1               5                   10                  15
   Pro Thr Pro Leu Leu Asp Thr Ile Asn Tyr Pro Ile His Met Lys Asn
               20                  25                  30
   Leu Ser Val Lys Glu Leu Lys Gln Leu Ala Asp Glu Leu Arg Ser Asp
           35                  40                  45
   Val Val Phe Asn Val Ser Lys Thr Gly Gly His Leu Gly Ser Ser Leu
       50                  55                  60
   Gly Val Val Glu Leu Thr Val Ala Leu His Tyr Val Phe Asn Ala Pro
   65                  70                  75                  80
   Gln Asp Arg Ile Leu Trp Asp Val Gly His Gln Ser Tyr Pro His Lys
                   85                  90                  95
   Ile Leu Thr Gly Arg Arg Asp Gln Met His Thr Met Arg Gln Thr Asp
               100                 105                 110
   Gly Leu Ala Gly Phe Thr Lys Arg Ser Glu Ser Glu Tyr Asp Cys Phe
           115                 120                 125
   Gly Thr Gly His Ser Ser Thr Thr Ile Ser Ala Gly Leu Gly Met Ala
       130                 135                 140
   Val Gly Arg Asp Leu Lys Gly Lys Asn Asn Asn Val Ile Ala Val Ile
   145 150 155 160
   Gly Asp Gly Ala Met Thr Ala Gly Gln Ala Tyr Glu Ala Met Asn Asn
   165 170 175
   Ala Gly Tyr Leu Asp Ser Asp Met Ile Val Ile Leu Asn Asp Asn Lys
   180 185 190
   Gln Val Ser Leu Pro Thr Ala Thr Leu Asp Gly Pro Ile Pro Pro Val
   195 200 205
   Gly Ala Leu Ser Ser Ala Leu Ser Arg Leu Gln Ser Asn Arg Pro Leu
   210 215 220
   Arg Glu Leu Arg Glu Val Ala Lys Gly Val Thr Lys Gln Ile Gly Gly
   225 230 235 240
   Pro Met His Glu Leu Ala Ala Lys Val Asp Glu Tyr Ala Cys Gly Met
   245 250 255
   Ile Ser Gly Ser Gly Ser Thr Leu Phe Glu Glu Leu Gly Leu Tyr Tyr
   260 265 270
   Ile Gly Pro Val Asp Gly His Asn Ile Asp Asp Leu Val Ala Ile Leu
   275 280 285
   Lys Glu Val Lys Ser Thr Lys Thr Thr Gly Pro Val Leu Ile His Val
   290 295 300
   Val Thr Glu Lys Gly Arg Gly Tyr Pro Tyr Ala Glu Lys Ala Ala Asp
   305 310 315 320
   Lys Tyr His Gly Val Thr Lys Phe Asp Pro Ala Thr Gly Lys Gln Phe
   325 330 335
   Lys Ser Ser Ala Pro Thr Gln Ser Tyr Thr Thr Tyr Phe Ala Glu Ala
   340 345 350
   Leu Ile Ala Glu Ala Glu Val Asp Lys Asp Ile Val Ala Ile His Ala
   355 360 365
   Ala Met Gly Gly Gly Thr Gly Leu Asn Leu Phe His Arg Arg Phe Pro
   370 375 380
   Thr Arg Cys Phe Asp Val Gly Ile Ala Glu Gln His Ala Val Thr Phe
   385 390 395 400
   Ala Ala Gly Leu Ala Cys Glu Gly Ile Lys Pro Phe Cys Ala Ile Tyr
   405 410 415
   Ser Ser Phe Met Gln Arg Ala Tyr Asp Gln Val Val His Asp Val Asp
   420 425 430
   Leu Gln Lys Leu Pro Val Lys Phe Ala Met Asp Arg Ala Gly Leu Val
   435 440 445
   Gly Ala Asp Gly Pro Thr His Cys Gly Ala Phe Asp Val Ala Phe Met
   450 455 460
   Ala Cys Leu Pro Asn Met Val Val Met Ala Pro Ala Asp Glu Ala Glu
   465 470 475 480
   Leu Phe His Met Val Ala Thr Ala Ala Ala Ile Asp Asp Arg Pro Ser
   485 490 495
   Cys Phe Arg Tyr Pro Arg Gly Asn Gly Val Gly Val Glu Leu Pro Pro
   500 505 510
   Gly Asn Lys Gly Ile Pro Ile Glu Val Gly Arg Gly Arg Ile Leu Ile
   515 520 525
   Glu Gly Glu Arg Val Ala Leu Leu Gly Tyr Gly Thr Ala Val Gln Ser
   530 535 540
   Cys Leu Val Ala Ser Ser Leu Leu Glu Gln His Gly Leu Arg Ile Thr
   545 550 555 560
   Val Ala Asp Ala Arg Phe Cys Lys Pro Leu Asp His Ala Leu Ile Arg
   565 570 575
   Ser Leu Ala Lys Ser His Glu Val Leu Ile Thr Val Glu Glu Gly Ser
   580 585 590
   Ile Gly Gly Phe Gly Ser His Val Ala Gln Phe Leu Ala Leu Asn Gly
   595 600 605
   Leu Leu Asp Gly Thr Thr Lys Trp Ser Pro Met Val Leu Pro Asp Arg
   610 615 620
   Tyr Ile Asp His Gly Ala Pro Ala Asp Gln Leu Ala Met Ala Gly Leu
   625 630 635 640
   Thr Pro Ser His Ile Ala Ala Thr Val Phe Asn Ile Leu Gly Gln Thr
   645 650 655
   Arg Glu Ala Leu Glu Ile Met Leu
   660
<210> 8
   <211> 1846
   <212> DNA
   <213> Vitis Vinifera
<400> 8
   cttcaatgtt tccaaaactg ggggtcactt gggctccagc ctcggggttg tggagctcac      60
   tgtggctctt cattatgtct tcaatgcccc tcaagacagg atactatggg atgttggtca     120
   tcagtcttac ccacacaaaa ttctaactgg gagaagagat caaatgcata ccatgaggca     180
   aacagatggg ttagcgggat tcaccaagcg ctcggagagt gaatatgact gctttggaac     240
   cggccacagt tctactacca tctcagcagg cttgggaatg gcggtcggcc gggatctaaa     300
   aggaaaaaac aacaacgtca ttgctgtcat aggtgatgga gccatgactg cagggcaagc     360
   ttatgaagca atgaacaatg ctggttacct ggattctgac atgattgtta tccttaatga     420
   caacaagcag gtttctttac ccactgctac tctagatggg cccataccac ctgtaggagc     480
   tttgagcagt gctcttagta ggttacaatc aaacagacct cttagagaat tacgagaggt     540
   tgccaagggc gttaccaaac agattggcgg accgatgcat gaattggctg caaaagttga     600
   tgaatatgct cgtgggatga tcagtggttc tggatcaaca ctttttgaag agcttggact     660
   ctattatata ggtcctgttg atggccacaa catagatgac cttgttgcca ttctcaagga     720
   ggttaagagt accaagacaa caggtccagt tctgatccat gttgtcacag agaaaggccg     780
   cggatatcca tatgctgaga aagctgcaga taagtaccat ggagtgacca agttcgatcc     840
   tgctactgga aaacaattca aatccagtgc tcctactcag tcctacacaa catattttgc     900
   agaggctttg attgcagaag cagaggtgga caaggatatt gttgcaattc atgcagcaat     960
   ggggggtgga acgggcttga atctcttcca tcgccggttc cccacacgat gctttgatgt    1020
   tgggatagca gaacagcatg ctgttacctt tgctgctggt ctagcctgtg aaggcattaa    1080
   acctttttgt gcaatctact catctttcat gcagagagct tatgaccagg tggtgcatga    1140
   tgtagatttg cagaagctgc cagtgaaatt tgcaatggac agagctgggc tggttggagc    1200
   agatggccca acacattgtg gagcttttga tgtcgctttc atggcttgcc ttccaaacat    1260
   ggtggtgatg gctcctgctg atgaggctga gctttttcac atggtggcca cagctgctgc    1320
   catagatgac aggcccagtt gtttccggta cccaagagga aatggggtgg gtgttgaact    1380
   gccaccaggg aacaaaggca ttcctattga ggttggaagg ggccgaatat tgattgaggg    1440
   ggagagagtt gcactcttgg gctatggaac agcagtacag agctgtttgg ttgcgtcttc    1500
   tttgctggaa caacatggct tacgaataac agtcgcagat gcccgcttct gcaaaccatt    1560
   ggaccatgct cttattcgta gcctagcaaa atcacatgaa gttttgatta cagtagaaga    1620
   agggtcaatt ggtggttttg ggtctcatgt tgctcagttt ttggccctta atggtcttct    1680
   tgatggcaca acaaagtgga gtcccatggt tcttcctgat cggtacatag accatggagc    1740
   gccagcggac cagttggcca tggcgggtct gacaccatct catattgcag caacagtatt    1800
   caatatactt ggacaaacaa gggaggccct ggagatcatg ttatag                    1846
<210> 9
   <211> 2151
   <212> DNA
   <213> Vitis Vinifera
<400> 9
   atggctctct gtacgctctc atttcctgcc cattttagcc aggctgctgc ttcaaatcct     60
   cagagactta ctcctcagtg ttcccatttg ttcttggggg tggatttgca gtgccaatcc    120
   cagcaaagga gtaaggccag gaaaaggcca aatggggttt gtgcatcact ttcggatagg    180
   gaggagtatc attcccagag accaccaact cctctcctgg acactatcaa ttatccaatt    240
   cacatgaaaa atctgtctgt caaggagctg aaacaactcg cagatgaact aaggtctgat    300
   gttgtcttca atgtttccaa aactgggggt cacttgggct ccagcctcgg ggttgtggag    360
   ctcactgtgg ctcttcatta tgtcttcaat gcccctcaag acaggatact atgggatgtt    420
   ggtcatcagt cttacccaca caaaattcta actgggagaa gagatcaaat gcataccatg    480
   aggcaaacag atgggttagc gggattcacc aagcgctcgg agagtgaata tgactgcttt    540
   ggaaccggcc acagttctac taccatctca gcaggcttgg gaatggcggt cggccgggat    600
   ctaaaaggaa aaaacaacaa cgtcattgct gtcataggtg atggagccat gactgcaggg    660
   caagcttatg aagcaatgaa caatgctggt tacctggatt ctgacatgat tgttatcctt    720
   aatgacaaca agcaggtttc tttacccact gctactctag atgggcccat accacctgta    780
   ggagctttga gcagtgctct tagtaggtta caatcaaaca gacctcttag agaattacga    840
   gaggttgcca atggcgttac caaacagatt ggcggaccga tgcatgaatt ggctgcaaaa    900
   gttgatgaat atgctcgtgg gatgatcagt ggttctggat caacactttt tgaagagctt    960
   ggactctatt atataggtcc tgttgatggc cacaacatag atgaccttgt tgccattctc   1020
   aaggaggtta agagtaccaa gacaacaggt ccagttctga tccatgttgt cacagagaaa   1080
   ggccgcggat atccatatgc tgagaaagct gcagataagt accatggagt gaccaagttc   1140
   gatcctgcta ctggaaaaca attcaaatcc agtgctccta ctcagtccta cacaacatat   1200
   tttgcagagg ctttgattgc agaagcagag gtggacaagg atattgttgc aattcatgca   1260
   gcaatggggg gtggaacggg cttgaatctc ttccatcgcc ggttccccac acgatgcttt   1320
   gatgttggga tagcagaaca gcatgctgtt acctttgctg ctggtctagc ctgtgaaggc   1380
   attaaacctt tttgtgcaat ctactcatct ttcatgcaga gagcttatga ccaggtggtg   1440
   catgatgtag atttgcagaa gctgccagtg aaatttgcaa tggacagagc tgggctggtt   1500
   ggagcagatg gcccaacaca ttgtggagct tttgatgtcg ctttcatggc ttgccttcca   1560
   aacatggtgg tgatggctcc tgctgatgag gctgagcttt ttcacatggt ggccacagct   1620
   gctgccatag atgacaggcc cagttgtttc cggtacccaa gaggaaatgg ggtgggtatt   1680
   gaactgccac cagggaacaa aggcattcct attgaggttg gaaggggccg aatattgatt   1740
   gagggggaga gagttgcact cttgggctat ggaacagcag tacagagctg tttggttgcg   1800
   tcttctttgc tggaacaaca tggcttacga ataacagtcg cagatgcccg cttctgcaaa   1860
   ccattggacc atgctcttat tcgtagccta gcaaaatcac atgaagtttt gattacagta   1920
   gaagaagggt caattggtgg ttttgggtct catgttgctc agtttttggc ccttaatggt 1980
   cttcttgatg gcacaacaaa gtggagtccc atggttcttc ctgatcggta catagaccat 2040
   ggagcgccag cggaccagtt ggccatggcg ggtctgacac catctcatat tgcagcaaca 2100
   gtattcaata tacttggaca aacaagggag gccctggaga tcatgttata g 2151
<210> 10
   <211> 2151
   <212> DNA
   <213> Vitis Vinifera
<400> 10
   atggctctct gtacgctctc atttcctgcc cattttagcc aggctgctgc ttcaaatcct 60
   cagagactta ctcctcagtg ttcccatttg ttcttggggg tggatttgca gtgccaatcc 120
   cagcaaagga gtaaggccag gaaaaggcca aatggggttt gtgcatcact ttcggatagg 180
   gaggagtatc attcccagag accaccaact cctctcctgg acactatcaa ttatccaatt 240
   cacatgaaaa atctgtctgt caaggagctg aaacaactcg cagatgaact aaggtctgat 300
   gttgtcttca atgtttccaa aactgggggt cacttgggct ccagcctcgg ggttgtggag 360
   ctcactgtgg ctcttcatta tgtcttcaat gcccctcaag acaggatact atgggatgtt 420
   ggtcatcagt cttacccaca caaaattcta actgggagaa gagatcaaat gcataccatg 480
   aggcaaacag atgggttagc gggattcacc aagcgctcgg agagtgaata tgactgcttt 540
   ggaaccggcc acagttctac taccatctca gcaggcttgg gaatggcggt cggccgggat 600
   ctaaaaggaa aaaacaacaa cgtcattgct gtcataggtg atggagccat gactgcaggg 660
   caagcttatg aagcaatgaa caatgctggt tacctggatt ctgacatgat tgttatcctt 720
   aatgacaaca agcaggtttc tttacccact gctactctag atgggcccat accacctgta 780
   ggagctttga gcagtgctct tagtaggtta caatcaaaca gacctcttag agaattacga 840
   gaggttgcca agggcgttac caaacagatt ggcggaccga tgcatgaatt ggctgcaaaa 900
   gttgatgaat atgcttgtgg gatgatcagt ggttctggat caacactttt tgaagagctt 960
   ggactctatt atataggtcc tgttgatggc cacaacatag atgaccttgt tgccattctc 1020
   aaggaggtta agagtaccaa gacaacaggt ccagttctga tccatgttgt cacagagaaa 1080
   ggccgcggat atccatatgc tgagaaagct gcagataagt accatggagt gaccaagttc 1140
   gatcctgcta ctggaaaaca attcaaatcc agtgctccta ctcagtccta cacaacatat 1200
   tttgcagagg ctttgattgc agaagcagag gtggacaagg atattgttgc aattcatgca 1260
   gcaatggggg gtggaacggg cttgaatctc ttccatcgcc ggttccccac acgatgcttt 1320
   gatgttggga tagcagaaca gcatgctgtt acctttgctg ctggtctagc ctgtgaaggc 1380
   attaaacctt tttgtgcaat ctactcatct ttcatgcaga gagcttatga ccaggtggtg 1440
   catgatgtag atttgcagaa gctgccagtg aaatttgcaa tggacagagc tgggctggtt 1500
   ggagcagatg gcccaacaca ttgtggagct tttgatgtcg ctttcatggc ttgccttcca 1560
   aacatggtgg tgatggctcc tgctgatgag gctgagcttt ttcacatggt ggccacagct 1620
   gctgccatag atgacaggcc cagttgtttc cggtacccaa gaggaaatgg ggtgggtgtt 1680
   gaactgccac cagggaacaa aggcattcct attgaggttg gaaggggccg aatattgatt 1740
   gagggggaga gagttgcact cttgggctat ggaacagcag tacagagctg tttggttgcg 1800
   tcttctttgc tggaacaaca tggcttacga ataacagtcg cagatgcccg cttctgcaaa 1860
   ccattggacc atgctcttat tcgtagccta gcaaaatcac atgaagtttt gattacagta 1920
   gaagaagggt caattggtgg ttttgggtct catgttgctc agtttttggc ccttaatggt 1980
   cttcttgatg gcacaacaaa gtggagtccc atggttcttc ctgatcggta catagaccat 2040
   ggagcgccag cggaccagtt ggccatggcg ggtctgacac catctcatat tgcagcaaca 2100
   gtattcaata tacttggaca aacaagggag gccctggaga tcatgttata g 2151
<210> 11
   <211> 72
   <212> DNA
   <213> Artificial sequence
<220>
   <223> PCR primer
<400> 11
   ggggacaagt ttgtacaaaa aagcaggctt ggttccgcgt ggatcaatgg ctctctgtac 60
   gctctcattt cc 72
<210> 12
   <211> 57
   <212> DNA
   <213> Artificial sequence
<220>
   <223> PCR primer
<400> 12
   ggggaccact ttgtacaaga aagctgggtt cactataaca tgatctccag ggcctcc 57
<210> 13
   <211> 1863
   <212> DNA
   <213> Escherichia coli
<400> 13
   atgagttttg atattgccaa atacccgacc ctggcactgg tcgactccac ccaggagtta 60
   cgactgttgc cgaaagagag tttaccgaaa ctctgcgacg aactgcgccg ctatttactc 120
   gacagcgtga gccgttccag cgggcacttc gcctccgggc tgggcacggt cgaactgacc 180
   gtggcgctgc actatgtcta caacaccccg tttgaccaat tgatttggga tgtggggcat 240
   caggcttatc cgcataaaat tttgaccgga cgccgcgaca aaatcggcac catccgtcag 300
   aaaggcggtc tgcacccgtt cccgtggcgc ggcgaaagcg aatatgacgt attaagcgtc 360
   gggcattcat caacctccat cagtgccgga attggtattg cggttgctgc cgaaaaagaa 420
   ggcaaaaatc gccgcaccgt ctgtgtcatt ggcgatggcg cgattaccgc aggcatggcg 480
   tttgaagcga tgaatcacgc gggcgatatc cgtcctgata tgctggtgat tctcaacgac 540
   aatgaaatgt cgatttccga aaatgtcggc gcgctcaaca accatctggc acagctgctt 600
   tccggtaagc tttactcttc actgcgcgaa ggcgggaaaa aagttttctc tggcgtgccg 660
   ccaattaaag agctgctcaa acgcaccgaa gaacatatta aaggcatggt agtgcctggc 720
   acgttgtttg aagagctggg ctttaactac atcggcccgg tggacggtca cgatgtgctg 780
   gggcttatca ccacgctaaa gaacatgcgc gacctgaaag gcccgcagtt cctgcatatc 840
   atgaccaaaa aaggtcgtgg ttatgaaccg gcagaaaaag acccgatcac tttccacgcc 900
   gtgcctaaat ttgatccctc cagcggttgt ttgccgaaaa gtagcggcgg tttgccgagc 960
   tattcaaaaa tctttggcga ctggttgtgc gaaacggcag cgaaagacaa caagctgatg 1020
   gcgattactc cggcgatgcg tgaaggttcc ggcatggtcg agttttcacg taaattcccg 1080
   gatcgctact tcgacgtggc aattgccgag caacacgcgg tgacctttgc tgcgggtctg 1140
   gcgattggtg ggtacaaacc cattgtcgcg atttactcca ctttcctgca acgcgcctat 1200
   gatcaggtgc tgcatgacgt ggcgattcaa aagcttccgg tcctgttcgc catcgaccgc 1260
   gcgggcattg ttggtgctga cggtcaaacc catcagggtg cttttgatct ctcttacctg 1320
   cgctgcatac cggaaatggt cattatgacc ccgagcgatg aaaacgaatg tcgccagatg 1380
   ctctataccg gctatcacta taacgatggc ccgtcagcgg tgcgctaccc gcgtggcaac 1440
   gcggtcggcg tggaactgac gccgctggaa aaactaccaa ttggcaaagg cattgtgaag 1500
   cgtcgtggcg agaaactggc gatccttaac tttggtacgc tgatgccaga agcggcgaaa 1560
   gtcgccgaat cgctgaacgc cacgctggtc gatatgcgtt ttgtgaaacc gcttgatgaa 1620
   gcgttaattc tggaaatggc cgccagccat gaagcgctgg tcaccgtaga agaaaacgcc 1680
   attatgggcg gcgcaggcag cggcgtgaac gaagtgctga tggcccatcg taaaccagta 1740
   cccgtgctga acattggcct gccggacttc tttattccgc aaggaactca ggaagaaatg 1800
   cgcgccgaac tcggcctcga tgccgctggt atggaagcca aaatcaaggc ctggctggca 1860
   taa 1863
<210> 14
   <211> 2505
   <212> DNA
   <213> Artificial sequence
<220>
   <223> Truncated DXS nucleotide
<220>
   <221> misc_feature
   <222> (653)..(653)
   <223> n=no nucleotide
<400> 14
   atgagttttg atattgccaa atacccgacc ctggcactgg tcgactccac ccaggagtta 60
   cgactgttgc cgaaagagag tttaccgaaa ctctgcgacg aactgcgccg ctatttactc 120
   gacagcgtga gccgttccag cgggcacttc gcctccgggc tgggcacggt cgaactgacc 180
   gtggcgctgc actatgtcta caacaccccg tttgaccaat tgatttggga tgtggggcat 240
   caggcttatc cgcataaaat tttgaccgga cgccgcgaca aaatcggcac catccgtcag 300
   aaaggcggtc tgcacccgtt cccgtggcgc ggcgaaagcg aatatgacgt attaagcgtc 360
   gggcattcat caacctccat cagtgccgga attggtattg cggttgctgc cgaaaaagaa 420
   ggcaaaaatc gccgcaccgt ctgtgtcatt ggcgatggcg cgattaccgc aggcatggcg 480
   tttgaagcga tgaatcacgc gggcgatatc cgtcctgata tgctggtgat tctcaacgac 540
   aatgaaatgt cgatttccga aaatgtcggc gcgctcaaca accatctggc acagctgctt 600
   tccggtaagc tttactcttc actgcgcgaa ggcgggaaca aagttttctc tgnattttaa 660
   ttcaataccc gaccctggca ctggtcgact cccccaggag ttacgactgt tgccgaaaga 720
   gagtttaccg aaactctgcg acgaactgcg ccgctattta ctcgacagcg tgagccgttc 780
   cagcgggcac ttcgcctccg ggctgggcac ggtcgaactg accgtggcgc tgcactatgt 840
   ctacaacacc ccgtttgacc aattgatttg ggatgtgggg catcaggctt atccgcataa 900
   aattttgacc ggacgccgcg acaaaatcgg caccatccgt cagaaaggcg gtctgcaccc 960
   gttcccgtgg cgcggcgaaa gcgaatatga cgtattaagc gtcgggcatt catcaacctc 1020
   catcagtgcc ggaattggta ttgcggttgc tgccgaaaaa gaaggcaaaa atcgccgcac 1080
   cgtctgtgtc attggcgatg gcgcgattac cgcaggcatg gcgtttgaag cgatgaatca 1140
   cgcgggcgat atccgtcctg atatgctggt gattctcaac gacaatgaaa tgtcgatttc 1200
   cgaaaatgtc ggcgcgctca acaaccatct ggcacagctg ctttccggta agctttactc 1260
   ttcactgcgc gaaggcggga acaaagtttt ctctgcgtgc cgccaattaa agagctgctc 1320
   aaacgcaccg aagaacatat taaaggcatg gtagtgcctg gcacgttgtt tgaagagctg 1380
   ggctttaact acatcggccc ggtggacggt cacgatgtgc tggggcttat caccacgcta 1440
   aagaacatgc gcgacctgaa aggcccgcag ttcctgcata tcatgaccaa aaaaggtcgt 1500
   ggttatgaac cggcagaaaa agacccgatc actttccacg ccgtgcctaa atttgatccc 1560
   tccagcggtt gtttgccgaa aagtagcggc ggtttgccga gctattcaaa aatctttggc 1620
   gactggttgt gcgaaacggc agcgaaagac aacaagctga tggcgattac tccggcgatg 1680
   cgtgaaggtt ccggcatggt cgagttttca cgtaaattcc cggatcgcta cttcgacgtg 1740
   gcaattgccg agcaacacgc ggtgaccttt gctgcgggtc tggcgattgg tgggtacaaa 1800
   cccattgtcg cgatttactc cactttcctg caacgcgcct atgatcaggt gctgcatgac 1860
   gtggcgattc aaaagcttcc ggtcctgttc gccatcgacc gcgcgggcat tgttggtgct 1920
   gacggtcaaa cccatcaggg tgcttttgat ctctcttacc tgcgctgcat accggaaatg 1980
   gtcattatga ccccgagcga tgaaaacgaa tgtcgccaga tgctctatac cggctatcac 2040
   tataacgatg gcccgtcagc ggtgcgctac ccgcgtggca acgcggtcgg cgtggaactg 2100
   acgccgctgg aaaaactacc aattggcaaa ggcattgtga agcgtcgtgg cgagaaactg 2160
   gcgatcctta actttggtac gctgatgcca gaagcggcga aagtcgccga atcgctgaac 2220
   gccacgctgg tcgatatgcg ttttgtgaaa ccgcttgatg aagcgttaat tctggaaatg 2280
   gccgccagcc atgaagcgct ggtcaccgta gaagaaaacg ccattatggg cggcgcaggc 2340
   agcggcgtga acgaagtgct gatggcccat cgtaaaccag tacccgtgct gaacattggc 2400
   ctgccggact tctttattcc gcaaggaact caggaagaaa tgcgcgccga actcggcctc 2460
   gatgccgctg gtatggaagc caaaatcaag gcctggctgg cataa 2505
<210> 15
   <211> 1863
   <212> DNA
   <213> Escherichia coli
<400> 15
   atgagttttg atattgccaa atacccgacc ctggcactgg tcgactccac ccaggagtta 60
   cgactgttgc cgaaagagag tttaccgaaa ctctgcgacg aactgcgccg ctatttactc 120
   gacagcgtga gccgttccag cgggcacttc gcctccgggc tgggcacggt cgaactgacc 180
   gtggcgctgc actatgtcta caacaccccg tttgaccaat tgatttggga tgtggggcat 240
   caggcttatc cgcataaaat tttgaccgga cgccgcgaca aaatcggcac catccgtcag 300
   aaaggcggtc tgcacccgtt cccgtggcgc ggcgaaagcg aatatgacgt attaagcgtc 360
   gggcattcat caacctccat cagtgccgga attggtattg cggttgctgc cgaaaaagaa 420
   ggcaaaaatc gccgcaccgt ctgtgtcatt ggcgatggcg cgattaccgc aggcatggcg 480
   tttgaagcga tgaatcacgc gggcgatatc cgtcctgata tgctggtgat tctcaacgac 540
   aatgaaatgt cgatttccga aaatgtcggc gcgctcaaca accatctggc acagctgctt 600
   tccggtaagc tttactcttc actgcgcgaa ggcgggaaca aagttttctc tggcgtgccg 660
   ccaattaaag agctgctcaa acgcaccgaa gaacatatta aaggcatggt agtgcctggc 720
   acgttgtttg aagagctggg ctttaactac atcggcccgg tggacggtca cgatgtgctg 780
   gggcttatca ccacgctaaa gaacatgcgc gacctgaaag gcccgcagtt cctgcatatc 840
   atgaccaaaa aaggtcgtgg ttatgaaccg gcagaaaaag acccgatcac tttccacgcc 900
   gtgcctaaat ttgatccctc cagcggttgt ttgccgaaaa gtagcggcgg tttgccgagc 960
   tattcaaaaa tctttggcga ctggttgtgc gaaacggcag cgaaagacaa caagctgatg 1020
   gcgattactc cggcgatgcg tgaaggttcc ggcatggtcg agttttcacg taaattcccg 1080
   gatcgctact tcgacgtggc aattgccgag caacacgcgg tgacctttgc tgcgggtctg 1140
   gcgattggtg ggtacaaacc cattgtcgcg atttactcca ctttcctgca acgcgcctat 1200
   gatcaggtgc tgcatgacgt ggcgattcaa aagcttccgg tcctgttcgc catcgaccgc 1260
   gcgggcattg ttggtgctga cggtcaaacc catcagggtg cttttgatct ctcttacctg 1320
   cgctgcatac cggaaatggt cattatgacc ccgagcgatg aaaacgaatg tcgccagatg 1380
   ctctataccg gctatcacta taacgatggc ccgtcagcgg tgcgctaccc gcgtggcaac 1440
   gcggtcggcg tggaactgac gccgctggaa aaactaccaa ttggcaaagg cattgtgaag 1500
   cgtcgtggcg agaaactggc gatccttaac tttggtacgc tgatgccaga agcggcgaaa 1560
   gtcgccgaat cgctgaacgc cacgctggtc gatatgcgtt ttgtgaaacc gcttgatgaa 1620
   gcgttaattc tggaaatggc cgccagccat gaagcgctgg tcaccgtaga agaaaacgcc 1680
   attatgggcg gcgcaggcag cggcgtgaac gaagtgctga tggcccatcg taaaccagta 1740
   cccgtgctga acattggcct gccggacttc tttattccgc aaggaactca ggaagaaatg 1800
   cgcgccgaac tcggcctcga tgccgctggt atggaagcca aaatcaaggc ctggctggca 1860
   taa 1863
<210> 16
   <211> 1863
   <212> DNA
   <213> Escherichia coli
<400> 16
   atgagttttg atattgccaa atacccgacc ctggcactgg tcgactccac ccaggagtta 60
   cgactgttgc cgaaagagag tttaccgaaa ctctgcgacg aactgcgccg ctatttactc 120
   gacagcgtga gccgttccag cgggcacttc gcctccgggc tgggcacggt cgaactgacc 180
   gtggcgctgc actatgtcta caacaccccg tttgaccaat tgatttggga tgtggggcat 240
   caggcttatc cgcataaaat tttgaccgga cgccgcgaca aaatcggcac catccgtcag 300
   aaaggcggtc tgcacccgtt cccgtggcgc ggcgaaagcg aatatgacgt attaagcgtc 360
   gggcattcat caacctccat cagtgccgga attggtattg cggttgctgc cgaaaaagaa 420
   ggcaaaaatc gccgcaccgt ctgtgtcatt ggcgatggcg cgattaccgc aggcatggcg 480
   tttgaagcga tgaatcacgc gggcgatatc cgtcctgata tgctggtgat tctcaacgac 540
   aatgaaatgt cgatttccga aaatgtcggc gcgctcaaca accatctggc acagctgctt 600
   tccggtaagc tttactcttc actgcgcgaa ggcgggaaaa aagttttctc tggcgtgccg 660
   ccaattaaag agctgctcaa acgcaccgaa gaacatattt gcggcatggt agtgcctggc 720
   acgttgtttg aagagctggg ctttaactac atcggcccgg tggacggtca cgatgtgctg 780
   gggcttatca ccacgctaaa gaacatgcgc gacctgaaag gcccgcagtt cctgcatatc 840
   atgaccaaaa aaggtcgtgg ttatgaaccg gcagaaaaag acccgatcac tttccacgcc 900
   gtgcctaaat ttgatccctc cagcggttgt ttgccgaaaa gtagcggcgg tttgccgagc 960
   tattcaaaaa tctttggcga ctggttgtgc gaaacggcag cgaaagacaa caagctgatg 1020
   gcgattactc cggcgatgcg tgaaggttcc ggcatggtcg agttttcacg taaattcccg 1080
   gatcgctact tcgacgtggc aattgccgag caacacgcgg tgacctttgc tgcgggtctg 1140
   gcgattggtg ggtacaaacc cattgtcgcg atttactcca ctttcctgca acgcgcctat 1200
   gatcaggtgc tgcatgacgt ggcgattcaa aagcttccgg tcctgttcgc catcgaccgc 1260
   gcgggcattg ttggtgctga cggtcaaacc catcagggtg cttttgatct ctcttacctg 1320
   cgctgcatac cggaaatggt cattatgacc ccgagcgatg aaaacgaatg tcgccagatg 1380
   ctctataccg gctatcacta taacgatggc ccgtcagcgg tgcgctaccc gcgtggcaac 1440
   gcggtcggcg tggaactgac gccgctggaa aaactaccaa ttggcaaagg cattgtgaag 1500
   cgtcgtggcg agaaactggc gatccttaac tttggtacgc tgatgccaga agcggcgaaa 1560
   gtcgccgaat cgctgaacgc cacgctggtc gatatgcgtt ttgtgaaacc gcttgatgaa 1620
   gcgttaattc tggaaatggc cgccagccat gaagcgctgg tcaccgtaga agaaaacgcc 1680
   attatgggcg gcgcaggcag cggcgtgaac gaagtgctga tggcccatcg taaaccagta 1740
   cccgtgctga acattggcct gccggacttc tttattccgc aaggaactca ggaagaaatg 1800
   cgcgccgaac tcggcctcga tgccgctggt atggaagcca aaatcaaggc ctggctggca 1860
   taa 1863
<210> 17
   <211> 620
   <212> PRT
   <213> Escherichia coli
<400> 17
   Met Ser Phe Asp Ile Ala Lys Tyr Pro Thr Leu Ala Leu Val Asp Ser
   1               5                   10                  15
   Thr Gln Glu Leu Arg Leu Leu Pro Lys Glu Ser Leu Pro Lys Leu Cys
               20                  25                  30
   Asp Glu Leu Arg Arg Tyr Leu Leu Asp Ser Val Ser Arg Ser Ser Gly
           35                  40                  45
   His Phe Ala Ser Gly Leu Gly Thr Val Glu Leu Thr Val Ala Leu His
       50                  55                  60
   Tyr Val Tyr Asn Thr Pro Phe Asp Gln Leu Ile Trp Asp Val Gly His
   65                  70                  75                  80
   Gln Ala Tyr Pro His Lys Ile Leu Thr Gly Arg Arg Asp Lys Ile Gly
                   85                  90                  95
   Thr Ile Arg Gln Lys Gly Gly Leu His Pro Phe Pro Trp Arg Gly Glu
               100                 105                 110
   Ser Glu Tyr Asp Val Leu Ser Val Gly His Ser Ser Thr Ser Ile Ser
           115                 120                 125
   Ala Gly Ile Gly Ile Ala Val Ala Ala Glu Lys Glu Gly Lys Asn Arg
       130                 135                 140
   Arg Thr Val Cys Val Ile Gly Asp Gly Ala Ile Thr Ala Gly Met Ala
   145                 150                 155                 160
   Phe Glu Ala Met Asn His Ala Gly Asp Ile Arg Pro Asp Met Leu Val
   165 170 175
   Ile Leu Asn Asp Asn Glu Met Ser Ile Ser Glu Asn Val Gly Ala Leu
   180 185 190
   Asn Asn His Leu Ala Gln Leu Leu Ser Gly Lys Leu Tyr Ser Ser Leu
   195 200 205
   Arg Glu Gly Gly Lys Lys Val Phe Ser Gly Val Pro Pro Ile Lys Glu
   210 215 220
   Leu Leu Lys Arg Thr Glu Glu His Ile Lys Gly Met Val Val Pro Gly
   225 230 235 240
   Thr Leu Phe Glu Glu Leu Gly Phe Asn Tyr Ile Gly Pro Val Asp Gly
   245 250 255
   His Asp Val Leu Gly Leu Ile Thr Thr Leu Lys Asn Met Arg Asp Leu
   260 265 270
   Lys Gly Pro Gln Phe Leu His Ile Met Thr Lys Lys Gly Arg Gly Tyr
   275 280 285
   Glu Pro Ala Glu Lys Asp Pro Ile Thr Phe His Ala Val Pro Lys Phe
   290 295 300
   Asp Pro Ser Ser Gly Cys Leu Pro Lys Ser Ser Gly Gly Leu Pro Ser
   305 310 315 320
   Tyr Ser Lys Ile Phe Gly Asp Trp Leu Cys Glu Thr Ala Ala Lys Asp
   325 330 335
   Asn Lys Leu Met Ala Ile Thr Pro Ala Met Arg Glu Gly Ser Gly Met
   340 345 350
   Val Glu Phe Ser Arg Lys Phe Pro Asp Arg Tyr Phe Asp Val Ala Ile
   355 360 365
   Ala Glu Gln His Ala Val Thr Phe Ala Ala Gly Leu Ala Ile Gly Gly
   370 375 380
   Tyr Lys Pro Ile Val Ala Ile Tyr Ser Thr Phe Leu Gln Arg Ala Tyr
   385 390 395 400
   Asp Gln Val Leu His Asp Val Ala Ile Gln Lys Leu Pro Val Leu Phe
   405 410 415
   Ala Ile Asp Arg Ala Gly Ile Val Gly Ala Asp Gly Gln Thr His Gln
   420 425 430
   Gly Ala Phe Asp Leu Ser Tyr Leu Arg Cys Ile Pro Glu Met Val Ile
   435 440 445
   Met Thr Pro Ser Asp Glu Asn Glu Cys Arg Gln Met Leu Tyr Thr Gly
   450 455 460
   Tyr His Tyr Asn Asp Gly Pro Ser Ala Val Arg Tyr Pro Arg Gly Asn
   465 470 475 480
   Ala Val Gly Val Glu Leu Thr Pro Leu Glu Lys Leu Pro Ile Gly Lys
   485 490 495
   Gly Ile Val Lys Arg Arg Gly Glu Lys Leu Ala Ile Leu Asn Phe Gly
   500 505 510
   Thr Leu Met Pro Glu Ala Ala Lys Val Ala Glu Ser Leu Asn Ala Thr
   515 520 525
   Leu Val Asp Met Arg Phe Val Lys Pro Leu Asp Glu Ala Leu Ile Leu
   530 535 540
   Glu Met Ala Ala Ser His Glu Ala Leu Val Thr Val Glu Glu Asn Ala
   545 550 555 560
   Ile Met Gly Gly Ala Gly Ser Gly Val Asn Glu Val Leu Met Ala His
   565 570 575
   Arg Lys Pro Val Pro Val Leu Asn Ile Gly Leu Pro Asp Phe Phe Ile
   580 585 590
   Pro Gln Gly Thr Gln Glu Glu Met Arg Ala Glu Leu Gly Leu Asp Ala
   595 600 605
   Ala Gly Met Glu Ala Lys Ile Lys Ala Trp Leu Ala
   610 615 620
<210> 18
   <211> 219
   <212> PRT
   <213> Artificial sequence
<220>
   <223> Truncated DXS polypeptide
<400> 18
   Met Ser Phe Asp Ile Ala Lys Tyr Pro Thr Leu Ala Leu Val Asp Ser
   1 5 10 15
   Thr Gln Glu Leu Arg Leu Leu Pro Lys Glu Ser Leu Pro Lys Leu Cys
   20 25 30
   Asp Glu Leu Arg Arg Tyr Leu Leu Asp Ser Val Ser Arg Ser Ser Gly
   35 40 45
   His Phe Ala Ser Gly Leu Gly Thr Val Glu Leu Thr Val Ala Leu His
   50 55 60
   Tyr Val Tyr Asn Thr Pro Phe Asp Gln Leu Ile Trp Asp Val Gly His
   65 70 75 80
   Gln Ala Tyr Pro His Lys Ile Leu Thr Gly Arg Arg Asp Lys Ile Gly
   85 90 95
   Thr Ile Arg Gln Lys Gly Gly Leu His Pro Phe Pro Trp Arg Gly Glu
   100 105 110
   Ser Glu Tyr Asp Val Leu Ser Val Gly His Ser Ser Thr Ser Ile Ser
   115 120 125
   Ala Gly Ile Gly Ile Ala Val Ala Ala Glu Lys Glu Gly Lys Asn Arg
   130 135 140
   Arg Thr Val Cys Val Ile Gly Asp Gly Ala Ile Thr Ala Gly Met Ala
   145 150 155 160
   Phe Glu Ala Met Asn His Ala Gly Asp Ile Arg Pro Asp Met Leu Val
   165 170 175
   Ile Leu Asn Asp Asn Glu Met Ser Ile Ser Glu Asn Val Gly Ala Leu
   180 185 190
   Asn Asn His Leu Ala Gln Leu Leu Ser Gly Lys Leu Tyr Ser Ser Leu
   195 200 205
   Arg Glu Gly Gly Asn Lys Val Phe Ser Gly Phe
   210 215
<210> 19
   <211> 620
   <212> PRT
   <213> Escherichia coli
<400> 19
   Met Ser Phe Asp Ile Ala Lys Tyr Pro Thr Leu Ala Leu Val Asp Ser
   1 5 10 15
   Thr Gln Glu Leu Arg Leu Leu Pro Lys Glu Ser Leu Pro Lys Leu Cys
   20 25 30
   Asp Glu Leu Arg Arg Tyr Leu Leu Asp Ser Val Ser Arg Ser Ser Gly
   35 40 45
   His Phe Ala Ser Gly Leu Gly Thr Val Glu Leu Thr Val Ala Leu His
   50 55 60
   Tyr Val Tyr Asn Thr Pro Phe Asp Gln Leu Ile Trp Asp Val Gly His
   65 70 75 80
   Gln Ala Tyr Pro His Lys Ile Leu Thr Gly Arg Arg Asp Lys Ile Gly
   85 90 95
   Thr Ile Arg Gln Lys Gly Gly Leu His Pro Phe Pro Trp Arg Gly Glu
   100 105 110
   Ser Glu Tyr Asp Val Leu Ser Val Gly His Ser Ser Thr Ser Ile Ser
   115 120 125
   Ala Gly Ile Gly Ile Ala Val Ala Ala Glu Lys Glu Gly Lys Asn Arg
   130 135 140
   Arg Thr Val Cys Val Ile Gly Asp Gly Ala Ile Thr Ala Gly Met Ala
   145 150 155 160
   Phe Glu Ala Met Asn His Ala Gly Asp Ile Arg Pro Asp Met Leu Val
   165 170 175
   Ile Leu Asn Asp Asn Glu Met Ser Ile Ser Glu Asn Val Gly Ala Leu
   180 185 190
   Asn Asn His Leu Ala Gln Leu Leu Ser Gly Lys Leu Tyr Ser Ser Leu
   195 200 205
   Arg Glu Gly Gly Asn Lys Val Phe Ser Gly Val Pro Pro Ile Lys Glu
   210 215 220
   Leu Leu Lys Arg Thr Glu Glu His Ile Lys Gly Met Val Val Pro Gly
   225 230 235 240
   Thr Leu Phe Glu Glu Leu Gly Phe Asn Tyr Ile Gly Pro Val Asp Gly
   245 250 255
   His Asp Val Leu Gly Leu Ile Thr Thr Leu Lys Asn Met Arg Asp Leu
   260 265 270
   Lys Gly Pro Gln Phe Leu His Ile Met Thr Lys Lys Gly Arg Gly Tyr
   275 280 285
   Glu Pro Ala Glu Lys Asp Pro Ile Thr Phe His Ala Val Pro Lys Phe
   290 295 300
   Asp Pro Ser Ser Gly Cys Leu Pro Lys Ser Ser Gly Gly Leu Pro Ser
   305 310 315 320
   Tyr Ser Lys Ile Phe Gly Asp Trp Leu Cys Glu Thr Ala Ala Lys Asp
   325 330 335
   Asn Lys Leu Met Ala Ile Thr Pro Ala Met Arg Glu Gly Ser Gly Met
               340                 345                 350
   Val Glu Phe Ser Arg Lys Phe Pro Asp Arg Tyr Phe Asp Val Ala Ile
           355                 360                 365
   Ala Glu Gln His Ala Val Thr Phe Ala Ala Gly Leu Ala Ile Gly Gly
       370                 375                 380
   Tyr Lys Pro Ile Val Ala Ile Tyr Ser Thr Phe Leu Gln Arg Ala Tyr
   385                 390                 395                 400
   Asp Gln Val Leu His Asp Val Ala Ile Gln Lys Leu Pro Val Leu Phe
                   405                 410                 415
   Ala Ile Asp Arg Ala Gly Ile Val Gly Ala Asp Gly Gln Thr His Gln
               420                 425                 430
   Gly Ala Phe Asp Leu Ser Tyr Leu Arg Cys Ile Pro Glu Met Val Ile
           435                 440                 445
   Met Thr Pro Ser Asp Glu Asn Glu Cys Arg Gln Met Leu Tyr Thr Gly
       450                 455                 460
   Tyr His Tyr Asn Asp Gly Pro Ser Ala Val Arg Tyr Pro Arg Gly Asn
   465                 470                 475                 480
   Ala Val Gly Val Glu Leu Thr Pro Leu Glu Lys Leu Pro Ile Gly Lys
                   485                 490                 495
   Gly Ile Val Lys Arg Arg Gly Glu Lys Leu Ala Ile Leu Asn Phe Gly
               500                 505                 510
   Thr Leu Met Pro Glu Ala Ala Lys Val Ala Glu Ser Leu Asn Ala Thr
           515                 520                 525
   Leu Val Asp Met Arg Phe Val Lys Pro Leu Asp Glu Ala Leu Ile Leu
       530                 535                 540
   Glu Met Ala Ala Ser His Glu Ala Leu Val Thr Val Glu Glu Asn Ala
   545                 550                 555                 560
   Ile Met Gly Gly Ala Gly Ser Gly Val Asn Glu Val Leu Met Ala His
                   565                 570                 575
   Arg Lys Pro Val Pro Val Leu Asn Ile Gly Leu Pro Asp Phe Phe Ile
               580                 585                 590
   Pro Gln Gly Thr Gln Glu Glu Met Arg Ala Glu Leu Gly Leu Asp Ala
           595                 600                 605
   Ala Gly Met Glu Ala Lys Ile Lys Ala Trp Leu Ala
   610 615 620
<210> 20
   <211> 620
   <212> PRT
   <213> Escherichia coli
<400> 20
   Met Ser Phe Asp Ile Ala Lys Tyr Pro Thr Leu Ala Leu Val Asp Ser
   1               5                   10                  15
   Thr Gln Glu Leu Arg Leu Leu Pro Lys Glu Ser Leu Pro Lys Leu Cys
               20                  25                  30
   Asp Glu Leu Arg Arg Tyr Leu Leu Asp Ser Val Ser Arg Ser Ser Gly
           35                  40                  45
   His Phe Ala Ser Gly Leu Gly Thr Val Glu Leu Thr Val Ala Leu His
       50                  55                  60
   Tyr Val Tyr Asn Thr Pro Phe Asp Gln Leu Ile Trp Asp Val Gly His
   65                  70                  75                  80
   Gln Ala Tyr Pro His Lys Ile Leu Thr Gly Arg Arg Asp Lys Ile Gly
                   85                  90                  95
   Thr Ile Arg Gln Lys Gly Gly Leu His Pro Phe Pro Trp Arg Gly Glu
               100                 105                 110
   Ser Glu Tyr Asp Val Leu Ser Val Gly His Ser Ser Thr Ser Ile Ser
           115                 120                 125
   Ala Gly Ile Gly Ile Ala Val Ala Ala Glu Lys Glu Gly Lys Asn Arg
       130                 135                 140
   Arg Thr Val Cys Val Ile Gly Asp Gly Ala Ile Thr Ala Gly Met Ala
   145                 150                 155                 160
   Phe Glu Ala Met Asn His Ala Gly Asp Ile Arg Pro Asp Met Leu Val
                   165                 170                 175
   Ile Leu Asn Asp Asn Glu Met Ser Ile Ser Glu Asn Val Gly Ala Leu
               180                 185                 190
   Asn Asn His Leu Ala Gln Leu Leu Ser Gly Lys Leu Tyr Ser Ser Leu
           195                 200                 205
   Arg Glu Gly Gly Lys Lys Val Phe Ser Gly Val Pro Pro Ile Lys Glu
       210                 215                 220
   Leu Leu Lys Arg Thr Glu Glu His Ile Cys Gly Met Val Val Pro Gly
   225 230 235 240
   Thr Leu Phe Glu Glu Leu Gly Phe Asn Tyr Ile Gly Pro Val Asp Gly
   245 250 255
   His Asp Val Leu Gly Leu Ile Thr Thr Leu Lys Asn Met Arg Asp Leu
   260 265 270
   Lys Gly Pro Gln Phe Leu His Ile Met Thr Lys Lys Gly Arg Gly Tyr
   275 280 285
   Glu Pro Ala Glu Lys Asp Pro Ile Thr Phe His Ala Val Pro Lys Phe
   290 295 300
   Asp Pro Ser Ser Gly Cys Leu Pro Lys Ser Ser Gly Gly Leu Pro Ser
   305 310 315 320
   Tyr Ser Lys Ile Phe Gly Asp Trp Leu Cys Glu Thr Ala Ala Lys Asp
   325 330 335
   Asn Lys Leu Met Ala Ile Thr Pro Ala Met Arg Glu Gly Ser Gly Met
   340 345 350
   Val Glu Phe Ser Arg Lys Phe Pro Asp Arg Tyr Phe Asp Val Ala Ile
   355 360 365
   Ala Glu Gln His Ala Val Thr Phe Ala Ala Gly Leu Ala Ile Gly Gly
   370 375 380
   Tyr Lys Pro Ile Val Ala Ile Tyr Ser Thr Phe Leu Gln Arg Ala Tyr
   385 390 395 400
   Asp Gln Val Leu His Asp Val Ala Ile Gln Lys Leu Pro Val Leu Phe
   405 410 415
   Ala Ile Asp Arg Ala Gly Ile Val Gly Ala Asp Gly Gln Thr His Gln
   420 425 430
   Gly Ala Phe Asp Leu Ser Tyr Leu Arg Cys Ile Pro Glu Met Val Ile
   435 440 445
   Met Thr Pro Ser Asp Glu Asn Glu Cys Arg Gln Met Leu Tyr Thr Gly
   450 455 460
   Tyr His Tyr Asn Asp Gly Pro Ser Ala Val Arg Tyr Pro Arg Gly Asn
   465 470 475 480
   Ala Val Gly Val Glu Leu Thr Pro Leu Glu Lys Leu Pro Ile Gly Lys
   485 490 495
   Gly Ile Val Lys Arg Arg Gly Glu Lys Leu Ala Ile Leu Asn Phe Gly
   500 505 510
   Thr Leu Met Pro Glu Ala Ala Lys Val Ala Glu Ser Leu Asn Ala Thr
   515 520 525
   Leu Val Asp Met Arg Phe Val Lys Pro Leu Asp Glu Ala Leu Ile Leu
   530 535 540
   Glu Met Ala Ala Ser His Glu Ala Leu Val Thr Val Glu Glu Asn Ala
   545 550 555 560
   Ile Met Gly Gly Ala Gly Ser Gly Val Asn Glu Val Leu Met Ala His
   565 570 575
   Arg Lys Pro Val Pro Val Leu Asn Ile Gly Leu Pro Asp Phe Phe Ile
   580 585 590
   Pro Gln Gly Thr Gln Glu Glu Met Arg Ala Glu Leu Gly Leu Asp Ala
   595 600 605
   Ala Gly Met Glu Ala Lys Ile Lys Ala Trp Leu Ala
   610 615 620
<210> 21
   <211> 626
   <212> PRT
   <213> Artificial sequence
<220>
   <223> Bacterial consensus DXS sequence including Deinococcus radiodurans
<220>
   <221> MISC_FEATURE
   <222> (2)..(2)
   <223> X=S, T or L
<220>
   <221> MISC_FEATURE
   <222> (3)..(3)
   <223> X=L, F, I or P
<220>
   <221> MISC_FEATURE
   <222> (4)..(4)
   <223> X=D, E or G
<220>
   <221> MISC_FEATURE
   <222> (5)..(5)
   <223> X= I, T, S or L
<220>
   <221> MISC_FEATURE
   <222> (6)..(6)
   <223> X= A, T or S
<220>
   <221> MISC_FEATURE
   <222> (7)..(7)
   <223> X= K or D
<220>
   <221> MISC_FEATURE
   <222> (8)..(8)
   <223> X= Y or T
<220>
   <221> MISC_FEATURE
   <222> (10)..(10)
   <223> X= T or L
<220>
   <221> MISC_FEATURE
   <222> (12)..(12)
   <223> X= A or D
<220>
   <221> MISC_FEATURE
   <222> (13)..(13)
   <223> X= L or Q
<220>
   <221> MISC_FEATURE
   <222> (14)..(14)
   <223> X= V, A or I
<220>
   <221> MISC_FEATURE
   <222> (15)..(15)
   <223> X= D, E, S or H
<220>
   <221> MISC_FEATURE
   <222> (16)..(16)
   <223> X=S, N, T or G
<220>
   <221> MISC_FEATURE
   <222> (17)..(17)
   <223> X=T, P or V
<220>
   <221> MISC_FEATURE
   <222> (18)..(18)
   <223> X=Q, A, E, D or K
<220>
   <221> MISC_FEATURE
   <222> (19)..(19)
   <223> X=A, E or D
<220>
   <221> MISC_FEATURE
   <222> (21)..(21)
   <223> X=R or K
<220>
   <221> MISC_FEATURE
   <222> (22)..(22)
   <223> X=L, M, A, S or R
<220>
   <221> MISC_FEATURE
   <222> (24)..(24)
   <223> X=P or S
<220>
   <221> MISC_FEATURE
   <222> (25)..(25)
   <223> X=K or R
<220>
   <221> MISC_FEATURE
   <222> (26)..(26)
   <223> X=E or D
<220>
   <221> MISC_FEATURE
   <222> (27)..(27)
   <223> X=S, K, T or Q
<220>
   <221> MISC_FEATURE
   <222> (30)..(30)
   <223> X=K, T or A
<220>
   <221> MISC_FEATURE
   <222> (32)..(32)
   <223> X=C, S or P
<220>
   <221> MISC_FEATURE
   <222> (33)..(33)
   <223> X=D, N or E
<220>
   <221> MISC_FEATURE
   <222> (37)..(37)
   <223> X=R, Q or G
<220>
   <221> MISC_FEATURE
   <222> (38)..(38)
   <223> X=Y, F or E
<220>
   <221> MISC_FEATURE
   <222> (39)..(39)
   <223> X=L or I
<220>
   <221> MISC_FEATURE
   <222> (40)..(40)
   <223> X=L or V
<220>
   <221> MISC_FEATURE
   <222> (41)..(41)
   <223> X=D, T, N, A or R
<220>
   <221> MISC_FEATURE
   <222> (42)..(42)
   <223> X=S, C or V
<220>
   <221> MISC_FEATURE
   <222> (43)..(43)
   <223> X=V or C
<220>
   <221> MISC_FEATURE
   <222> (45)..(45)
   <223> X=R or Q
<220>
   <221> MISC_FEATURE
   <222> (46)..(46)
   <223> X=S or G
<220>
   <221> MISC_FEATURE
   <222> (47)..(47)
   <223> X=S or G
<220>
   <221> MISC_FEATURE
   <222> (48)..(48)
   <223> X=G or L
<220>
   <221> MISC_FEATURE
   <222> (50)..(50)
   <223> X=F or L
<220>
   <221> MISC_FEATURE
   <222> (53)..(53)
   <223> X=G or S
<220>
   <221> MISC_FEATURE
   <222> (56)..(56)
   <223> X=T, V or A
<220>
   <221> MISC_FEATURE
   <222> (58)..(58)
   <223> X=E or D
<220>
   <221> MISC_FEATURE
   <222> (59)..(59)
   <223> X=L or I
<220>
   <221> MISC_FEATURE
   <222> (60)..(60)
   <223> X=T or I
<220>
   <221> MISC_FEATURE
   <222> (61)..(61)
   <223> X=V or T
<220>
   <221> MISC_FEATURE
   <222> (63)..(63)
   <223> X=L or M
<220>
   <221> MISC_FEATURE
   <222> (67)..(67)
   <223> X=Y or L
<220>
   <221> MISC_FEATURE
   <222> (68)..(68)
   <223> X=N, K or D
<220>
   <221> MISC_FEATURE
   <222> (69)..(69)
   <223> X=T or S
<220>
   <221> MISC_FEATURE
   <222> (71)..(71)
   <223> X=F or R
<220>
   <221> MISC_FEATURE
   <222> (73)..(73)
   <223> X=Q, R, H or N
<220>
   <221> MISC_FEATURE
   <222> (74)..(74)
   <223> X=L, V or I
<220>
   <221> MISC_FEATURE
   <222> (75)..(75)
   <223> X=I, V or L
<220>
   <221> MISC_FEATURE
   <222> (76)..(76)
   <223> X=W or F
<220>
   <221> MISC_FEATURE
   <222> (84)..(84)
   <223> X=P or A
<220>
   <221> MISC_FEATURE
   <222> (94)..(94)
   <223> X=K, R or Q
<220>
   <221> MISC_FEATURE
   <222> (95)..(95)
   <223> X=I or M
<220>
   <221> MISC_FEATURE
   <222> (96)..(96)
   <223> X=G, S, A or D
<220>
   <221> MISC_FEATURE
   <222> (97)..(97)
   <223> X=T, S or D
<220>
   <221> MISC_FEATURE
   <222> (99)..(99)
   <223> X=R or K
<220>
   <221> MISC_FEATURE
   <222> (100)..(100)
   <223> X=Q or K
<220>
   <221> MISC_FEATURE
   <222> (101)..(101)
   <223> X=K or E
<220>
   <221> MISC_FEATURE
   <222> (102)..(102)
   <223> X=G, D or N
<220>
   <221> MISC_FEATURE
   <222> (104)..(104)
   <223> X=L, V or I
<220>
   <221> MISC_FEATURE
   <222> (105)..(105)
   <223> X=H or S
<220>
   <221> MISC_FEATURE
   <222> (106)..(106)
   <223> X=P or G
<220>
   <221> MISC_FEATURE
   <222> (108)..(108)
   <223> X=P or T
<220>
   <221> MISC_FEATURE
   <222> (109)..(109)
   <223> X= W or K
<220>
   <221> MISC_FEATURE
   <222> (110)..(110)
   <223> X= R or V
<220>
   <221> MISC_FEATURE
   <222> (111)..(111)
   <223> X= G, E, D, A or S
<220>
   <221> MISC_FEATURE
   <222> (114)..(114)
   <223> X=E or D
<220>
   <221> MISC_FEATURE
   <222> (115)..(115)
   <223> X=Y, F or H
<220>
   <221> MISC_FEATURE
   <222> (117)..(117)
   <223> X=V, T or A
<220>
   <221> MISC_FEATURE
   <222> (118)..(118)
   <223> X=L or I
<220>
   <221> MISC_FEATURE
   <222> (119)..(119)
   <223> X=S, N, C or T
<220>
   <221> MISC_FEATURE
   <222> (123)..(123)
   <223> X=S or A
<220>
   <221> MISC_FEATURE
   <222> (127)..(127)
   <223> X=I or L
<220>
   <221> MISC_FEATURE
   <222> (128)..(128)
   <223> X=S or A
<220>
   <221> MISC_FEATURE
   <222> (129)..(129)
   <223> X=A or N
<220>
   <221> MISC_FEATURE
   <222> (130)..(130)
   <223> X=G or A
<220>
   <221> MISC_FEATURE
   <222> (131)..(131)
   <223> X=I or L
<220>
   <221> MISC_FEATURE
   <222> (133)..(133)
   <223> X=I, V or M
<220>
   <221> MISC_FEATURE
   <222> (135)..(135)
   <223> X=V, I, A or L
<220>
   <221> MISC_FEATURE
   <222> (137)..(137)
   <223> X=A or R
<220>
   <221> MISC_FEATURE
   <222> (138)..(138)
   <223> X=E, A or D
<220>
   <221> MISC_FEATURE
   <222> (139)..(139)
   <223> X=K, R or A
<220>
   <221> MISC_FEATURE
   <222> (140)..(140)
   <223> X=E or Q
<220>
   <221> MISC_FEATURE
   <222> (141)..(141)
   <223> X=G, N, D or A
<220>
   <221> MISC_FEATURE
   <222> (142)..(142)
   <223> X=K, Q or L
<220>
   <221> MISC_FEATURE
   <222> (143)..(143)
   <223> X=N, D, Q or G
<220>
   <221> MISC_FEATURE
   <222> (144)..(144)
   <223> X=R or F
<220>
   <221> MISC_FEATURE
   <222> (145)..(145)
   <223> X=R, K or H
<220>
   <221> MISC_FEATURE
   <222> (146)..(146)
   <223> X=T, A or V
<220>
   <221> MISC_FEATURE
   <222> (147)..(147)
   <223> X=V or A
<220>
   <221> MISC_FEATURE
   <222> (148)..(148)
   <223> X=C or A
<220>
   <221> MISC_FEATURE
   <222> (154)..(154)
   <223> X=A or S
<220>
   <221> MISC_FEATURE
   <222> (155)..(155)
   <223> X=I or L
<220>
   <221> MISC_FEATURE
   <222> (157)..(157)
   <223> X=A or G
<220>
   <221> MISC_FEATURE
   <222> (161)..(161)
   <223> X=F or L
<220>
   <221> MISC_FEATURE
   <222> (162)..(162)
   <223> X=E or A
<220>
   <221> MISC_FEATURE
   <222> (164)..(164)
   <223> X=M or L
<220>
   <221> MISC_FEATURE
   <222> (165)..(165)
   <223> X=N or S
<220>
   <221> MISC_FEATURE
   <222> (166)..(166)
   <223> X=H or T
<220>
   <221> MISC_FEATURE
   <222> (167)..(167)
   <223> X=A or I
<220>
   <221> MISC_FEATURE
   <222> (170)..(170)
   <223> X=I or M
<220>
   <221> MISC_FEATURE
   <222> (171)..(171)
   <223> X=R, K, H, D or N
<220>
   <221> MISC_FEATURE
   <222> (172)..(172)
   <223> X=P, S, A or R
<220>
   <221> MISC_FEATURE
   <222> (173)..(173)
   <223> X=D or K
<220>
   <221> MISC_FEATURE
   <222> (174)..(174)
   <223> X=M or L
<220>
   <221> MISC_FEATURE
   <222> (176)..(176)
   <223> X=V or I
<220>
   <221> MISC_FEATURE
   <222> (177)..(177)
   <223> X=I or V
<220>
   <221> MISC_FEATURE
   <222> (182)..(182)
   <223> X=E or G
<220>
   <221> MISC_FEATURE
   <222> (191)..(191)
   <223> X=A or G
<220>
   <221> MISC_FEATURE
   <222> (192)..(192)
   <223> X=L or M
<220>
   <221> MISC_FEATURE
   <222> (194)..(194)
   <223> X=N or K
<220>
   <221> MISC_FEATURE
   <222> (195)..(195)
   <223> X=H or F
<220>
   <221> MISC_FEATURE
   <222> (196)..(196)
   <223> X=L or M
<220>
   <221> MISC_FEATURE
   <222> (197)..(197)
   <223> X=A or R
<220>
   <221> MISC_FEATURE
   <222> (198)..(198)
   <223> X=Q or G
<220>
   <221> MISC_FEATURE
   <222> (199)..(199)
   <223> X=L or I
<220>
   <221> MISC_FEATURE
   <222> (200)..(200)
   <223> X=L or Q
<220>
   <221> MISC_FEATURE
   <222> (201)..(201)
   <223> X=S or V
<220>
   <221> MISC_FEATURE
   <222> (202)..(202)
   <223> X=G or Q
<220>
   <221> MISC_FEATURE
   <222> (204)..(204)
   <223> X=L, T or W
<220>
   <221> MISC_FEATURE
   <222> (205)..(205)
   <223> X=Y or F
<220>
   <221> MISC_FEATURE
   <222> (206)..(206)
   <223> X=S, A, T or Q
<220>
   <221> MISC_FEATURE
   <222> (207)..(207)
   <223> X=S, T, R or E
<220>
   <221> MISC_FEATURE
   <222> (208)..(208)
   <223> X=L or G
<220>
   <221> MISC_FEATURE
   <222> (209)..(209)
   <223> X=R or E
<220>
   <221> MISC_FEATURE
   <222> (210)..(210)
   <223> X=E or G
<220>
   <221> MISC_FEATURE
   <222> (211)..(211)
   <223> X=G, S or A
<220>
   <221> MISC_FEATURE
   <222> (214)..(214)
   <223> X=K or R
<220>
   <221> MISC_FEATURE
   <222> (215)..(215)
   <223> X=V or A
<220>
   <221> MISC_FEATURE
   <222> (216)..(216)
   <223> X=F, L or V
<220>
   <221> MISC_FEATURE
   <222> (217)..(217)
   <223> X=S, D, T or E
<220>
   <221> MISC_FEATURE
   <222> (218)..(218)
   <223> X=G, N or A
<220>
   <221> MISC_FEATURE
   <222> (219)..(219)
   <223> X=V or L
<220>
   <221> MISC_FEATURE
   <222> (220)..(220)
   <223> X=P or S
<220>
   <221> MISC_FEATURE
   <222> (221)..(221)
   <223> X=K or no amino acid
<220>
   <221> MISC_FEATURE
   <222> (223)..(223)
   <223> X=I or L
<220>
   <221> MISC_FEATURE
   <222> (224)..(224)
   <223> X=K or A
<220>
   <221> MISC_FEATURE
   <222> (225)..(225)
   <223> X=E or D
<220>
   <221> MISC_FEATURE
   <222> (226)..(226)
   <223> X=L or F
<220>
   <221> MISC_FEATURE
   <222> (227)..(227)
   <223> X=L, V or M
<220>
   <221> MISC_FEATURE
   <222> (228)..(228)
   <223> X=K, R or S
<220>
   <221> MISC_FEATURE
   <222> (229)..(229)
   <223> X=R or K
<220>
   <221> MISC_FEATURE
   <222> (230)..(230)
   <223> X=T or A
<220>
   <221> MISC_FEATURE
   <222> (231)..(231)
   <223> X=E or K
<220>
   <221> MISC_FEATURE
   <222> (232)..(232)
   <223> X=E or N
<220>
   <221> MISC_FEATURE
   <222> (233)..(233)
   <223> X=H or S
<220>
   <221> MISC_FEATURE
   <222> (234)..(234)
   <223> X=I, L or T
<220>
   <221> MISC_FEATURE
   <222> (235)..(235)
   <223> X=K or R
<220>
   <221> MISC_FEATURE
   <222> (236)..(236)
   <223> X=G or H
<220>
   <221> MISC_FEATURE
   <222> (237)..(237)
   <223> X=M or F
<220>
   <221> MISC_FEATURE
   <222> (238)..(238)
   <223> X=V, M or F
<220>
   <221> MISC_FEATURE
   <222> (239)..(239)
   <223> X=V or D
<220>
   <221> MISC_FEATURE
   <222> (241)..(241)
   <223> X=G, S or A
<220>
   <221> MISC_FEATURE
   <222> (242)..(242)
   <223> X=T or S
<220>
   <221> MISC_FEATURE
   <222> (243)..(243)
   <223> X=L or V
<220>
   <221> MISC_FEATURE
   <222> (244)..(244)
   <223> X=N or no amino acid
<220>
   <221> MISC_FEATURE
   <222> (245)..(245)
   <223> X=P or no amino acid
<220>
   <221> MISC_FEATURE
   <222> (247)..(247)
   <223> X=E or A
<220>
   <221> MISC_FEATURE
   <222> (248)..(248)
   <223> X=E or A
<220>
   <221> MISC_FEATURE
   <222> (249)..(249)
   <223> X=L or M
<220>
   <221> MISC_FEATURE
   <222> (251)..(251)
   <223> X=F or V
<220>
   <221> MISC_FEATURE
   <222> (252)..(252)
   <223> X=N or R
<220>
   <221> MISC_FEATURE
   <222> (254)..(254)
   <223> X=I or V
<220>
   <221> MISC_FEATURE
   <222> (261)..(261)
   <223> X=D or N
<220>
   <221> MISC_FEATURE
   <222> (263)..(263)
   <223> X=L, M, H, Q or V
<220>
   <221> MISC_FEATURE
   <222> (264)..(264)
   <223> X=G, T, A, S or E
<220>
   <221> MISC_FEATURE
   <222> (266)..(266)
   <223> X=I, V, T, S or A
<220>
   <221> MISC_FEATURE
   <222> (267)..(267)
   <223> X=T, S, N, Q, H, A or W
<220>
   <221> MISC_FEATURE
   <222> (268)..(268)
   <223> X=T or L
<220>
   <221> MISC_FEATURE
   <222> (270)..(270)
   <223> X=K, R, S or E
<220>
   <221> MISC_FEATURE
   <222> (271)..(271)
   <223> X=N or R
<220>
   <221> MISC_FEATURE
   <222> (272)..(272)
   <223> X=M or L
<220>
   <221> MISC_FEATURE
   <222> (273)..(273)
   <223> X=R or V
<220>
   <221> MISC_FEATURE
   <222> (274)..(274)
   <223> X=D, A, S or E
<220>
   <221> MISC_FEATURE
   <222> (276)..(276)
   <223> X=K or D
<220>
   <221> MISC_FEATURE
   <222> (277)..(277)
   <223> X=G or S
<220>
   <221> MISC_FEATURE
   <222> (279)..(279)
   <223> X=Q or T
<220>
   <221> MISC_FEATURE
   <222> (280)..(280)
   <223> X=F, L or I
<220>
   <221> MISC_FEATURE
   <222> (283)..(283)
   <223> X=I or V
<220>
   <221> MISC_FEATURE
   <222> (284)..(284)
   <223> X=M or V
<220>
   <221> MISC_FEATURE
   <222> (286)..(286)
   <223> X=T or K
<220>
   <221> MISC_FEATURE
   <222> (289)..(289)
   <223> X=R or K
<220>
   <221> MISC_FEATURE
   <222> (291)..(291)
   <223> X=Y or L
<220>
   <221> MISC_FEATURE
   <222> (292)..(292)
   <223> X=E, A or S
<220>
   <221> MISC_FEATURE
   <222> (293)..(293)
   <223> X=P or Y
<220>
   <221> MISC_FEATURE
   <222> (296)..(296)
   <223> X=K, Q or A
<220>
   <221> MISC_FEATURE
   <222> (300)..(300)
   <223> X=T, G, S or Y
<220>
   <221> MISC_FEATURE
   <222> (301)..(301)
   <223> X=F or W
<220>
   <221> MISC_FEATURE
   <222> (303)..(303)
   <223> X=A or G
<220>
   <221> MISC_FEATURE
   <222> (304)..(304)
   <223> X=V or P
<220>
   <221> MISC_FEATURE
   <222> (305)..(305)
   <223> X=P or A
<220>
   <221> MISC_FEATURE
   <222> (309)..(309)
   <223> X=P, H or V
<220>
   <221> MISC_FEATURE
   <222> (310)..(310)
   <223> X=S, T, E or A
<220>
   <221> misc_feature
   <222> (311)..(311)
   <223> xaa can be any naturally occurring amino acid
<220>
   <221> MISC_FEATURE
   <222> (313)..(313)
   <223> X=C, T, L or E
<220>
   <221> MISC_FEATURE
   <222> (314)..(314)
   <223> X=L or Y
<220>
   <221> MISC_FEATURE
   <222> (315)..(315)
   <223> X=P or V
<220>
   <221> MISC_FEATURE
   <222> (316)..(316)
   <223> X=K or P
<220>
   <221> MISC_FEATURE
   <222> (317)..(317)
   <223> X=S or N
<220>
   <221> MISC_FEATURE
   <222> (318)..(318)
   <223> X=S, Q, A or K
<220>
   <221> MISC_FEATURE
   <222> (319)..(319)
   <223> X=G, S, E, D or A
<220>
   <221> MISC_FEATURE
   <222> (320)..(320)
   <223> X=G, S, A, T or Y
<220>
   <221> MISC_FEATURE
   <222> (321)..(321)
   <223> X=A or no amino acid
<220>
   <221> MISC_FEATURE
   <222> (322)..(322)
   <223> X=L, M, Q, R or no amino acid
<220>
   <221> MISC_FEATURE
   <222> (323)..(323)
   <223> X=P or no amino acid
<220>
   <221> MISC_FEATURE
   <222> (324)..(324)
   <223> X=S, G or T
<220>
   <221> MISC_FEATURE
   <222> (325)..(325)
   <223> X=Y, F or W
<220>
   <221> MISC_FEATURE
   <222> (327)..(327)
   <223> X=K or A
<220>
   <221> MISC_FEATURE
   <222> (328)..(328)
   <223> X=I, V or A
<220>
   <221> MISC_FEATURE
   <222> (331)..(331)
   <223> X=D, E, Q or N
<220>
   <221> MISC_FEATURE
   <222> (332)..(332)
   <223> X=W or A
<220>
   <221> MISC_FEATURE
   <222> (333)..(333)
   <223> X=L or V
<220>
   <221> MISC_FEATURE
   <222> (334)..(334)
   <223> X=C, S, K, Q or T
<220>
   <221> MISC_FEATURE
   <222> (336)..(336)
   <223> X=T, M, E or W
<220>
   <221> MISC_FEATURE
   <222> (338)..(338)
   <223> X=A or K
<220>
   <221> MISC_FEATURE
   <222> (339)..(339)
   <223> X=K, V, H, A, Q or T
<220>
   <221> MISC_FEATURE
   <222> (341)..(341)
   <223> X=N, D, S, T, K or P
<220>
   <221> MISC_FEATURE
   <222> (342)..(342)
   <223> X=K, S or R
<220>
   <221> MISC_FEATURE
   <222> (343)..(343)
   <223> X=L or T
<220>
   <221> MISC_FEATURE
   <222> (344)..(344)
   <223> X=M or F
<220>
   <221> MISC_FEATURE
   <222> (345)..(345)
   <223> X=A or V
<220>
   <221> MISC_FEATURE
   <222> (346)..(346)
   <223> X=I or V
<220>
   <221> MISC_FEATURE
   <222> (356)..(356)
   <223> X=M or L
<220>
   <221> MISC_FEATURE
   <222> (357)..(357)
   <223> X=V or L
<220>
   <221> MISC_FEATURE
   <222> (358)..(358)
   <223> X=E, R, S, G, Q
<220>
   <221> MISC_FEATURE
   <222> (361)..(361)
   <223> X=R or K
<220>
   <221> MISC_FEATURE
   <222> (362)..(362)
   <223> X=K, E, D, T or V
<220>
   <221> MISC_FEATURE
   <222> (363)..(363)
   <223> X=F, Y or H
<220>
   <221> MISC_FEATURE
   <222> (365)..(365)
   <223> X=D, A, Q, N, E or H
<220>
   <221> MISC_FEATURE
   <222> (366)..(366)
   <223> X=R or Q
<220>
   <221> MISC_FEATURE
   <222> (368)..(368)
   <223> X=F or L
<220>
   <221> MISC_FEATURE
   <222> (371)..(371)
   <223> X=A or G
<220>
   <221> MISC_FEATURE
   <222> (375)..(375)
   <223> X=Q or E
<220>
   <221> MISC_FEATURE
   <222> (376)..(376)
   <223> X=H or V
<220>
   <221> MISC_FEATURE
   <222> (377)..(377)
   <223> X=A or S
<220>
   <221> MISC_FEATURE
   <222> (378)..(378)
   <223> X=V or A
<220>
   <221> MISC_FEATURE
   <222> (380)..(380)
   <223> X=F or T
<220>
   <221> MISC_FEATURE
   <222> (384)..(384)
   <223> X=L or M
<220>
   <221> MISC_FEATURE
   <222> (386)..(386)
   <223> X=I, V or L
<220>
   <221> MISC_FEATURE
   <222> (387)..(387)
   <223> X=V or Q
<220>
   <221> MISC_FEATURE
   <222> (388)..(388)
   <223> X=G or D
<220>
   <221> MISC_FEATURE
   <222> (389)..(389)
   <223> X=Y or M
<220>
   <221> MISC_FEATURE
   <222> (390)..(390)
   <223> X=K, H or R
<220>
   <221> MISC_FEATURE
   <222> (392)..(392)
   <223> X=I or V
<220>
   <221> MISC_FEATURE
   <222> (407)..(407)
   <223> X=V or L
<220>
   <221> MISC_FEATURE
   <222> (408)..(408)
   <223> X=L or I
<220>
   <221> MISC_FEATURE
   <222> (411)..(411)
   <223> X=V or I
<220>
   <221> MISC_FEATURE
   <222> (414)..(414)
   <223> X=Q or E
<220>
   <221> MISC_FEATURE
   <222> (415)..(415)
   <223> X=K, N, S or H
<220>
   <221> MISC_FEATURE
   <222> (417)..(417)
   <223> X=P or N
<220>
   <221> MISC_FEATURE
   <222> (419)..(419)
   <223> X=L, M or T
<220>
   <221> MISC_FEATURE
   <222> (421)..(421)
   <223> X=A, T or C
<220>
   <221> MISC_FEATURE
   <222> (422)..(422)
   <223> X=I or V
<220>
   <221> MISC_FEATURE
   <222> (425)..(425)
   <223> X=A or G
<220>
   <221> MISC_FEATURE
   <222> (427)..(427)
   <223> X=I or L
<220>
   <221> MISC_FEATURE
   <222> (433)..(433)
   <223> X=Q or A
<220>
   <221> MISC_FEATURE
   <222> (436)..(436)
   <223> X=Q or N
<220>
   <221> MISC_FEATURE
   <222> (438)..(438)
   <223> X=A, S or V
<220>
   <221> MISC_FEATURE
   <222> (442)..(442)
   <223> X=S or A
<220>
   <221> MISC_FEATURE
   <222> (443)..(443)
   <223> X=Y or F
<220>
   <221> MISC_FEATURE
   <222> (444)..(444)
   <223> X=L or M
<220>
   <221> MISC_FEATURE
   <222> (446)..(446)
   <223> X=C or S
<220>
   <221> MISC_FEATURE
   <222> (447)..(447)
   <223> X=I or V
<220>
   <221> MISC_FEATURE
   <222> (449)..(449)
   <223> X=E, D, N, Q, T or G
<220>
   <221> MISC_FEATURE
   <222> (450)..(450)
   <223> X=M or V
<220>
   <221> MISC_FEATURE
   <222> (451)..(451)
   <223> X=V, I, T, R
<220>
   <221> MISC_FEATURE
   <222> (452)..(452)
   <223> X=I or V
<220>
   <221> MISC_FEATURE
   <222> (453)..(453)
   <223> X=M or G
<220>
   <221> MISC_FEATURE
   <222> (454)..(454)
   <223> X=T, A or L
<220>
   <221> MISC_FEATURE
   <222> (456)..(456)
   <223> X=S or K
<220>
   <221> MISC_FEATURE
   <222> (458)..(458)
   <223> X=E or A
<220>
   <221> MISC_FEATURE
   <222> (459)..(459)
   <223> X=N or A
<220>
   <221> MISC_FEATURE
   <222> (461)..(461)
   <223> X=C or L
<220>
   <221> MISC_FEATURE
   <222> (463)..(463)
   <223> X=Q, L or G
<220>
   <221> MISC_FEATURE
   <222> (466)..(466)
   <223> X=Y, F, H or K
<220>
   <221> MISC_FEATURE
   <222> (467)..(467)
   <223> X=T or Y
<220>
   <221> MISC_FEATURE
   <222> (468)..(468)
   <223> X=G or A
<220>
   <221> MISC_FEATURE
   <222> (469)..(469)
   <223> X=Y, H or Q
<220>
   <221> MISC_FEATURE
   <222> (470)..(470)
   <223> X=H or T
<220>
   <221> MISC_FEATURE
   <222> (471)..(471)
   <223> X=Y or H
<220>
   <221> MISC_FEATURE
   <222> (472)..(472)
   <223> X=N, Q, S, E or no amino acid
<220>
   <221> MISC_FEATURE
   <222> (473)..(473)
   <223> X=D, G, Q, K, S, E or no amino acid
<220>
   <221> MISC_FEATURE
   <222> (476)..(476)
   <223> X=S, T, C, A, V or F
<220>
   <221> MISC_FEATURE
   <222> (477)..(477)
   <223> X=A, V or T
<220>
   <221> MISC_FEATURE
   <222> (478)..(478)
   <223> X=V or I
<220>
   <221> MISC_FEATURE
   <222> (484)..(484)
   <223> X=N, S, T or A
<220>
   <221> MISC_FEATURE
   <222> (485)..(485)
   <223> X=A, G, V or T
<220>
   <221> MISC_FEATURE
   <222> (486)..(486)
   <223> X=V, Q, L, T or A
<220>
   <221> MISC_FEATURE
   <222> (487)..(487)
   <223> X=G, S or Q
<220>
   <221> MISC_FEATURE
   <222> (488)..(488)
   <223> X=V, A or T
<220>
   <221> MISC_FEATURE
   <222> (489)..(489)
   <223> X=E, A, T, V, P or Q
<220>
   <221> MISC_FEATURE
   <222> (490)..(490)
   <223> X=L, F, S or A
<220>
   <221> MISC_FEATURE
   <222> (491)..(491)
   <223> X=T, E, Q or G
<220>
   <221> MISC_FEATURE
   <222> (492)..(492)
   <223> X=P or T
<220>
   <221> MISC_FEATURE
   <222> (493)..(493)
   <223> X=L or W
<220>
   <221> MISC_FEATURE
   <222> (494)..(494)
   <223> X=E, T, Q, A, S, G or P
<220>
   <221> MISC_FEATURE
   <222> (495)..(495)
   <223> X=K, Q, S, M, A, T, E and D
<220>
   <221> MISC_FEATURE
   <222> (496)..(496)
   <223> X=L or M
<220>
   <221> MISC_FEATURE
   <222> (497)..(497)
   <223> X=P or K
<220>
   <221> MISC_FEATURE
   <222> (498)..(498)
   <223> X=I, L or W
<220>
   <221> MISC_FEATURE
   <222> (500)..(500)
   <223> X=K or E
<220>
   <221> MISC_FEATURE
   <222> (501)..(501)
   <223> X=G or W
<220>
   <221> MISC_FEATURE
   <222> (502)..(502)
   <223> X=I, L, V or E
<220>
   <221> MISC_FEATURE
   <222> (503)..(503)
   <223> X=V, L, I or R
<220>
   <221> MISC_FEATURE
   <222> (504)..(504)
   <223> X=K, R or L
<220>
   <221> MISC_FEATURE
   <222> (505)..(505)
   <223> X=R or K
<220>
   <221> MISC_FEATURE
   <222> (506)..(506)
   <223> X=R, H, Q or G
<220>
   <221> MISC_FEATURE
   <222> (508)..(508)
   <223> X=E, K or D
<220>
   <221> MISC_FEATURE
   <222> (509)..(509)
   <223> X=K, T or D
<220>
   <221> MISC_FEATURE
   <222> (510)..(510)
   <223> X=L, V or I
<220>
   <221> MISC_FEATURE
   <222> (511)..(511)
   <223> X=A or V
<220>
   <221> MISC_FEATURE
   <222> (512)..(512)
   <223> X=I or L
<220>
   <221> MISC_FEATURE
   <222> (514)..(514)
   <223> X=N, C or A
<220>
   <221> MISC_FEATURE
   <222> (515)..(515)
   <223> X=F or G
<220>
   <221> MISC_FEATURE
   <222> (517)..(517)
   <223> X=T or K
<220>
   <221> MISC_FEATURE
   <222> (518)..(518)
   <223> X=L or A
<220>
   <221> MISC_FEATURE
   <222> (519)..(519)
   <223> X=M or L
<220>
   <221> MISC_FEATURE
   <222> (520)..(520)
   <223> X=P, A or D
<220>
   <221> MISC_FEATURE
   <222> (521)..(521)
   <223> X=E, Q or Y
<220>
   <221> MISC_FEATURE
   <222> (523)..(523)
   <223> X=A, T, Q, S or L
<220>
   <221> MISC_FEATURE
   <222> (524)..(524)
   <223> X=K, Q, A, L or V
<220>
   <221> MISC_FEATURE
   <222> (525)..(525)
   <223> X=V, A or T
<220>
   <221> MISC_FEATURE
   <222> (527)..(527)
   <223> X=E or D
<220>
   <221> MISC_FEATURE
   <222> (528)..(528)
   <223> X=S, A, K, N, T or D
<220>
   <221> MISC_FEATURE
   <222> (529)..(529)
   <223> X=L, M or R
<220>
   <221> MISC_FEATURE
   <222> (530)..(530)
   <223> X=P or no amino acid
<220>
   <221> MISC_FEATURE
   <222> (531)..(531)
   <223> X=N or G
<220>
   <221> MISC_FEATURE
   <222> (532)..(532)
   <223> X=A or V
<220>
   <221> MISC_FEATURE
   <222> (533)..(533)
   <223> X=T or G
<220>
   <221> MISC_FEATURE
   <222> (534)..(534)
   <223> X=L or V
<220>
   <221> MISC_FEATURE
   <222> (536)..(536)
   <223> X=D or N
<220>
   <221> MISC_FEATURE
   <222> (537)..(537)
   <223> X=M or A
<220>
   <221> MISC_FEATURE
   <222> (545)..(545)
   <223> X=E, D, T or K
<220>
   <221> MISC_FEATURE
   <222> (546)..(546)
   <223> X=A, T, E, S or Q
<220>
   <221> MISC_FEATURE
   <222> (547)..(547)
   <223> X=L or M
<220>
   <221> MISC_FEATURE
   <222> (548)..(548)
   <223> X=I, V, L or T
<220>
   <221> MISC_FEATURE
   <222> (549)..(549)
   <223> X=L, M, A, E, Q or R
<220>
   <221> MISC_FEATURE
   <222> (550)..(550)
   <223> X=E, Q or S
<220>
   <221> MISC_FEATURE
   <222> (551)..(551)
   <223> X=M, L or V
<220>
   <221> MISC_FEATURE
   <222> (552)..(552)
   <223> X=A or G
<220>
   <221> MISC_FEATURE
   <222> (553)..(553)
   <223> X=A, S, G or Q
<220>
   <221> MISC_FEATURE
   <222> (554)..(554)
   <223> X=S, Q, R, E or K
<220>
   <221> MISC_FEATURE
   <222> (555)..(555)
   <223> X=H or A
<220>
   <221> MISC_FEATURE
   <222> (556)..(556)
   <223> X=E, D, Q, S or R
<220>
   <221> MISC_FEATURE
   <222> (557)..(557)
   <223> X=A, V, S, T or L
<220>
   <221> MISC_FEATURE
   <222> (558)..(558)
   <223> X=L or F
<220>
   <221> MISC_FEATURE
   <222> (559)..(559)
   <223> X=V or I
<220>
   <221> MISC_FEATURE
   <222> (561)..(561)
   <223> X=V, I or L
<220>
   <221> MISC_FEATURE
   <222> (563)..(563)
   <223> X=E or D
<220>
   <221> MISC_FEATURE
   <222> (564)..(564)
   <223> X=N or G
<220>
   <221> MISC_FEATURE
   <222> (565)..(565)
   <223> X=A or T
<220>
   <221> MISC_FEATURE
   <222> (566)..(566)
   <223> X=V or I
<220>
   <221> MISC_FEATURE
   <222> (567)..(567)
   <223> X=M, K or V
<220>
   <221> MISC_FEATURE
   <222> (570)..(570)
   <223> X=A or F
<220>
   <221> MISC_FEATURE
   <222> (572)..(572)
   <223> X=S or G
<220>
   <221> MISC_FEATURE
   <222> (573)..(573)
   <223> X=G or A
<220>
   <221> MISC_FEATURE
   <222> (575)..(575)
   <223> X=N or L
<220>
   <221> MISC_FEATURE
   <222> (577)..(577)
   <223> X=V, L, F, Y or A
<220>
   <221> MISC_FEATURE
   <222> (578)..(578)
   <223> X=L or V
<220>
   <221> MISC_FEATURE
   <222> (579)..(579)
   <223> X=M or N
<220>
   <221> MISC_FEATURE
   <222> (580)..(580)
   <223> X=A, Q or S
<220>
   <221> MISC_FEATURE
   <222> (581)..(581)
   <223> X=H, N, K, E, R or M
<220>
   <221> MISC_FEATURE
   <222> (582)..(582)
   <223> X=R, K or N
<220>
   <221> MISC_FEATURE
   <222> (583)..(583)
   <223> X=K, R, L or I
<220>
   <221> MISC_FEATURE
   <222> (584)..(584)
   <223> X=P, A, W, G, L or H
<220>
   <221> MISC_FEATURE
   <222> (585)..(585)
   <223> X=V, I or P
<220>
   <221> MISC_FEATURE
   <222> (586)..(586)
   <223> X=P, S or T
<220>
   <221> MISC_FEATURE
   <222> (588)..(588)
   <223> X=L or R
<220>
   <221> MISC_FEATURE
   <222> (589)..(589)
   <223> X=N or V
<220>
   <221> MISC_FEATURE
   <222> (590)..(590)
   <223> X=I or L
<220>
   <221> MISC_FEATURE
   <222> (592)..(592)
   <223> X=L or I
<220>
   <221> MISC_FEATURE
   <222> (593)..(593)
   <223> X=P or A
<220>
   <221> MISC_FEATURE
   <222> (595)..(595)
   <223> X=F, R, H, Y, L, E, I, V or S
<220>
   <221> MISC_FEATURE
   <222> (597)..(597)
   <223> X=I, V or Q
<220>
   <221> MISC_FEATURE
   <222> (598)..(598)
   <223> X=P, S or E
<220>
   <221> MISC_FEATURE
   <222> (599)..(599)
   <223> X=Q, P or H
<220>
   <221> MISC_FEATURE
   <222> (600)..(600)
   <223> X=G or A
<220>
   <221> MISC_FEATURE
   <222> (601)..(601)
   <223> X=T, E or S
<220>
   <221> MISC_FEATURE
   <222> (602)..(602)
   <223> X=Q or A
<220>
   <221> MISC_FEATURE
   <222> (603)..(603)
   <223> X=E, D or A
<220>
   <221> MISC_FEATURE
   <222> (604)..(604)
   <223> X=E or S
<220>
   <221> MISC_FEATURE
   <222> (605)..(605)
   <223> X=M, A, I, L or V
<220>
   <221> MISC_FEATURE
   <222> (606)..(606)
   <223> X=R or H
<220>
   <221> MISC_FEATURE
   <222> (607)..(607)
   <223> X=A, S, H, T or V
<220>
   <221> MISC_FEATURE
   <222> (608)..(608)
   <223> X=E, D or R
<220>
   <221> MISC_FEATURE
   <222> (609)..(609)
   <223> X=L, I, Y or A
<220>
   <221> MISC_FEATURE
   <222> (610)..(610)
   <223> X=G, Q or S
<220>
   <221> MISC_FEATURE
   <222> (611)..(611)
   <223> X=L or I
<220>
   <221> MISC_FEATURE
   <222> (612)..(612)
   <223> X=D, N or T
<220>
   <221> MISC_FEATURE
   <222> (613)..(613)
   <223> X=A or I
<220>
   <221> MISC_FEATURE
   <222> (614)..(614)
   <223> X=A, S, N, D, E or P
<220>
   <221> MISC_FEATURE
   <222> (615)..(615)
   <223> X=G or A
<220>
   <221> MISC_FEATURE
   <222> (616)..(616)
   <223> X=M or I
<220>
   <221> MISC_FEATURE
   <222> (617)..(617)
   <223> X=E, Q, R, K or D
<220>
   <221> MISC_FEATURE
   <222> (618)..(618)
   <223> X=A, T, Q, R or N
<220>
   <221> MISC_FEATURE
   <222> (619)..(619)
   <223> X=K, R, Q or V
<220>
   <221> MISC_FEATURE
   <222> (620)..(620)
   <223> X=I, V or L
<220>
   <221> MISC_FEATURE
   <222> (621)..(621)
   <223> X=K, R, E, T, S or A
<220>
   <221> MISC_FEATURE
   <222> (622)..(622)
   <223> X=A, T, D, R, N, K, Q or E
<220>
   <221> MISC_FEATURE
   <222> (623)..(623)
   <223> X=W, Y or L
<220>
   <221> MISC_FEATURE
   <222> (624)..(624)
   <223> X=L, Q, M or G
<220>
   <221> MISC_FEATURE
   <222> (625)..(625)
   <223> X=A, V or no amino acid
<220>
   <221> MISC_FEATURE
   <222> (626)..(626)
   <223> X=D or no amino acid
<400> 21
   Met Xaa Xaa Xaa Xaa Xaa Xaa Xaa Pro Xaa Leu Xaa Xaa Xaa Xaa Xaa
   1               5                   10                  15
   Xaa Xaa Xaa Leu Xaa Xaa Leu Xaa Xaa Xaa Xaa Leu Pro Xaa Leu Xaa
               20                  25                  30
   Xaa Glu Leu Arg Xaa Xaa Xaa Xaa Xaa Xaa Xaa Ser Xaa Xaa Xaa Xaa
           35                  40                  45
   His Xaa Ala Ser Xaa Leu Gly Xaa Val Xaa Xaa Xaa Xaa Ala Xaa His
       50                  55                  60
   Tyr Val Xaa Xaa Xaa Pro Xaa Asp Xaa Xaa Xaa Xaa Asp Val Gly His
   65                  70                  75                  80
   Gln Ala Tyr Xaa His Lys Ile Leu Thr Gly Arg Arg Asp Xaa Xaa Xaa
                   85                  90                  95
   Xaa Ile Xaa Xaa Xaa Xaa Gly Xaa Xaa Xaa Phe Xaa Xaa Xaa Xaa Glu
               100                 105                 110
   Ser Xaa Xaa Asp Xaa Xaa Xaa Val Gly His Xaa Ser Thr Ser Xaa Xaa
           115                 120                 125
   Xaa Xaa Xaa Gly Xaa Ala Xaa Ala Xaa Xaa Xaa Glx Xaa Xaa Xaa Xaa
       130                 135                 140
   Xaa Xaa Xaa Xaa Val Ile Gly Asp Gly Xaa Xaa Thr Xaa Gly Met Ala
   145                 150                 155                 160
   Xaa Xaa Ala Xaa Xaa Xaa Xaa Gly Asp Xaa Xaa Xaa Xaa Xaa Leu Xaa
                   165                 170                 175
   Xaa Leu Asn Asp Asn Xaa Met Ser Ile Ser Glu Asn Val Gly Xaa Xaa
               180                 185                 190
   Asn Xaa Xaa Xaa Xaa Xaa Xaa Xaa Xaa Xaa Lys Xaa Xaa Xaa Xaa Xaa
           195                 200                 205
   Xaa Xaa Xaa Gly Lys Xaa Xaa Xaa Xaa Xaa Xaa Xaa Xaa Pro Xaa Xaa
       210                 215                 220
   Xaa Xaa Xaa Xaa Xaa Xaa Xaa Xaa Xaa Xaa Xaa Xaa Xaa Xaa Xaa Pro
   225                 230                 235                 240
   Xaa Xaa Xaa Xaa Xaa Phe Xaa Xaa Xaa Gly Xaa Xaa Tyr Xaa Gly Pro
                   245                 250                 255
   Val Asp Gly His Xaa Val Xaa Xaa Leu Xaa Xaa Xaa Leu Xaa Xaa Xaa
               260                 265                 270
   Xaa Xaa Leu Xaa Xaa Pro Xaa Xaa Leu His Xaa Xaa Thr Xaa Lys Gly
           275                 280                 285
   Xaa Gly Xaa Xaa Xaa Ala Glu Xaa Asp Pro Ile Xaa Xaa His Xaa Xaa
       290                 295                 300
   Xaa Lys Phe Asp Xaa Xaa Xaa Gly Xaa Xaa Xaa Xaa Xaa Xaa Xaa Xaa
   305                 310                 315                 320
   Xaa Xaa Xaa Xaa Xaa Ser Xaa Xaa Phe Gly Xaa Xaa Xaa Xaa Glu Xaa
                   325                 330                 335
   Ala Xaa Xaa Asp Xaa Xaa Xaa Xaa Xaa Xaa Thr Pro Ala Met Arg Glu
               340                 345                 350
   Gly Ser Gly Xaa Xaa Xaa Phe Ser Xaa Xaa Xaa Pro Xaa Xaa Tyr Xaa
           355                 360                 365
   Asp Val Xaa Ile Ala Glu Xaa Xaa Xaa Xaa Thr Xaa Ala Ala Gly Xaa
       370                 375                 380
   Ala Xaa Xaa Xaa Xaa Xaa Pro Xaa Val Ala Ile Tyr Ser Thr Phe Leu
   385                 390                 395                 400
   Gln Arg Ala Tyr Asp Gln Xaa Xaa His Asp Xaa Ala Ile Xaa Xaa Leu
                   405                 410                 415
   Xaa Val Xaa Phe Xaa Xaa Asp Arg Xaa Gly Xaa Val Gly Ala Asp Gly
               420                 425                 430
   Xaa Thr His Xaa Gly Xaa Phe Asp Leu Xaa Xaa Xaa Arg Xaa Xaa Pro
   435 440 445
   Xaa Xaa Xaa Xaa Xaa Xaa Pro Xaa Asp Xaa Xaa Glu Xaa Arg Xaa Met
   450 455 460
   Leu Xaa Xaa Xaa Xaa Xaa Xaa Xaa Xaa Gly Pro Xaa Xaa Xaa Arg Tyr
   465 470 475 480
   Pro Arg Gly Xaa Xaa Xaa Xaa Xaa Xaa Xaa Xaa Xaa Xaa Xaa Xaa Xaa
   485 490 495
   Xaa Xaa Gly Xaa Xaa Xaa Xaa Xaa Xaa Xaa Gly Xaa Xaa Xaa Xaa Xaa
   500 505 510
   Leu Xaa Xaa Gly Xaa Xaa Xaa Xaa Xaa Ala Xaa Xaa Xaa Ala Xaa Xaa
   515 520 525
   Xaa Xaa Xaa Xaa Xaa Xaa Val Xaa Xaa Arg Phe Val Lys Pro Leu Asp
   530 535 540
   Xaa Xaa Xaa Xaa Xaa Xaa Xaa Xaa Xaa Xaa Xaa Xaa Xaa Xaa Xaa Thr
   545 550 555 560
   Xaa Glu Xaa Xaa Xaa Xaa Xaa Gly Gly Xaa Gly Xaa Xaa Val Xaa Glu
   565 570 575
   Xaa Xaa Xaa Xaa Xaa Xaa Xaa Xaa Xaa Xaa Val Xaa Xaa Xaa Gly Xaa
   580 585 590
   Xaa Asp Xaa Phe Xaa Xaa Xaa Xaa Xaa Xaa Xaa Xaa Xaa Xaa Xaa Xaa
   595 600 605
   Xaa Xaa Xaa Xaa Xaa Xaa Xaa Xaa Xaa Xaa Xaa Xaa Xaa Xaa Xaa Xaa
   610 615 620
   Xaa Xaa
   625
<210> 22
   <211> 621
   <212> PRT
   <213> Artificial sequence
<220>
   <223> Bacterial DXS consensus sequence
<220>
   <221> MISC_FEATURE
   <222> (2)..(2)
   <223> X= S or T
<220>
   <221> MISC_FEATURE
   <222> (3)..(3)
   <223> X= F, L or I
<220>
   <221> MISC_FEATURE
   <222> (4)..(4)
   <223> X= D or E
<220>
   <221> MISC_FEATURE
   <222> (5)..(5)
   <223> X= I, T, S or L
<220>
   <221> MISC_FEATURE
   <222> (6)..(6)
   <223> X= A or T
<220>
   <221> MISC_FEATURE
   <222> (13)..(13)
   <223> X= L or Q
<220>
   <221> MISC_FEATURE
   <222> (14)..(14)
   <223> X= V or A
<220>
   <221> MISC_FEATURE
   <222> (15)..(15)
   <223> X= D, E or S
<220>
   <221> MISC_FEATURE
   <222> (16)..(16)
   <223> X= S, N or T
<220>
   <221> MISC_FEATURE
   <222> (17)..(17)
   <223> X= T, P or V
<220>
   <221> MISC_FEATURE
   <222> (18)..(18)
   <223> X= Q, A, E or D
<220>
   <221> MISC_FEATURE
   <222> (19)..(19)
   <223> X= A, E or D
<220>
   <221> MISC_FEATURE
   <222> (22)..(22)
   <223> X= L, M, A or S
<220>
   <221> MISC_FEATURE
   <222> (25)..(25)
   <223> X= K or R
<220>
   <221> MISC_FEATURE
   <222> (26)..(26)
   <223> X= E or D
<220>
   <221> MISC_FEATURE
   <222> (27)..(27)
   <223> X= S, K or T
<220>
   <221> MISC_FEATURE
   <222> (30)..(30)
   <223> X= K, T or A
<220>
   <221> MISC_FEATURE
   <222> (32)..(32)
   <223> X= C or S
<220>
   <221> MISC_FEATURE
   <222> (33)..(33)
   <223> X= D or N
<220>
   <221> MISC_FEATURE
   <222> (37)..(37)
   <223> X= R or Q
<220>
   <221> MISC_FEATURE
   <222> (38)..(38)
   <223> X= Y or F
<220>
   <221> MISC_FEATURE
   <222> (41)..(41)
   <223> X= D, T, N or A
<220>
   <221> MISC_FEATURE
   <222> (42)..(42)
   <223> X= S or C
<220>
   <221> MISC_FEATURE
   <222> (45)..(45)
   <223> X= R or Q
<220>
   <221> MISC_FEATURE
   <222> (56)..(56)
   <223> X= T, V or A
<220>
   <221> MISC_FEATURE
   <222> (63)..(63)
   <223> X=L or M
<220>
   <221> MISC_FEATURE
   <222> (68)..(68)
   <223> X=N or K
<220>
   <221> MISC_FEATURE
   <222> (73)..(73)
   <223> X= Q, R, H or N
<220>
   <221> MISC_FEATURE
   <222> (74)..(74)
   <223> X= L or V
<220>
   <221> MISC_FEATURE
   <222> (75)..(75)
   <223> X= I or V
<220>
   <221> MISC_FEATURE
   <222> (94)..(94)
   <223> X= K or R
<220>
   <221> MISC_FEATURE
   <222> (96)..(96)
   <223> X= G, S, A or D
<220>
   <221> MISC_FEATURE
   <222> (97)..(97)
   <223> X= T or S
<220>
   <221> MISC_FEATURE
   <222> (102)..(102)
   <223> X= G, D or N
<220>
   <221> MISC_FEATURE
   <222> (104)..(104)
   <223> X= L or V
<220>
   <221> MISC_FEATURE
   <222> (111)..(111)
   <223> X= G, D, E or A
<220>
   <221> MISC_FEATURE
   <222> (114)..(114)
   <223> X= E or D
<220>
   <221> MISC_FEATURE
   <222> (115)..(115)
   <223> X= Y or F
<220>
   <221> MISC_FEATURE
   <222> (117)..(117)
   <223> X= V or T
<220>
   <221> MISC_FEATURE
   <222> (119)..(119)
   <223> X= S, N or C
<220>
   <221> MISC_FEATURE
   <222> (131)..(131)
   <223> X= I or L
<220>
   <221> MISC_FEATURE
   <222> (133)..(133)
   <223> X= I, V or M
<220>
   <221> MISC_FEATURE
   <222> (135)..(135)
   <223> X= V, I or A
<220>
   <221> MISC_FEATURE
   <222> (138)..(138)
   <223> X= E, A or D
<220>
   <221> MISC_FEATURE
   <222> (139)..(139)
   <223> X= K or R
<220>
   <221> MISC_FEATURE
   <222> (141)..(141)
   <223> X= G, N, D or A
<220>
   <221> MISC_FEATURE
   <222> (142)..(142)
   <223> X= K, Q or L
<220>
   <221> MISC_FEATURE
   <222> (143)..(143)
   <223> X= N, E, D, Q or G
<220>
   <221> MISC_FEATURE
   <222> (145)..(145)
   <223> X= R or K
<220>
   <221> MISC_FEATURE
   <222> (146)..(146)
   <223> X= T or A
<220>
   <221> MISC_FEATURE
   <222> (147)..(147)
   <223> X= V or A
<220>
   <221> MISC_FEATURE
   <222> (165)..(165)
   <223> X= N or S
<220>
   <221> MISC_FEATURE
   <222> (171)..(171)
   <223> X= R, K, H, D or N
<220>
   <221> MISC_FEATURE
   <222> (172)..(172)
   <223> X= P, S or A
<220>
   <221> MISC_FEATURE
   <222> (174)..(174)
   <223> X= M or L
<220>
   <221> MISC_FEATURE
   <222> (177)..(177)
   <223> X= I or V
<220>
   <221> MISC_FEATURE
   <222> (182)..(182)
   <223> X= E or G
<220>
   <221> MISC_FEATURE
   <222> (191)..(191)
   <223> X= A or G
<220>
   <221> MISC_FEATURE
   <222> (195)..(195)
   <223> X= H or R
<220>
   <221> MISC_FEATURE
   <222> (199)..(199)
   <223> X= L or I
<220>
   <221> MISC_FEATURE
   <222> (204)..(204)
   <223> X= L or T
<220>
   <221> MISC_FEATURE
   <222> (206)..(206)
   <223> X= S, A or T
<220>
   <221> MISC_FEATURE
   <222> (207)..(207)
   <223> X= S, T or R
<220>
   <221> MISC_FEATURE
   <222> (211)..(211)
   <223> X= G or S
<220>
   <221> MISC_FEATURE
   <222> (214)..(214)
   <223> X= K or R
<220>
   <221> MISC_FEATURE
   <222> (215)..(215)
   <223> X= V or A
<220>
   <221> MISC_FEATURE
   <222> (216)..(216)
   <223> X= F or L
<220>
   <221> MISC_FEATURE
   <222> (217)..(217)
   <223> X= S, D or T
<220>
   <221> MISC_FEATURE
   <222> (218)..(218)
   <223> X= G, N or A
<220>
   <221> MISC_FEATURE
   <222> (219)..(219)
   <223> X= V or L
<220>
   <221> MISC_FEATURE
   <222> (224)..(224)
   <223> X= E or D
<220>
   <221> MISC_FEATURE
   <222> (226)..(226)
   <223> X= L or V
<220>
   <221> MISC_FEATURE
   <222> (227)..(227)
   <223> X= K or R
<220>
   <221> MISC_FEATURE
   <222> (228)..(228)
   <223> X= R or K
<220>
   <221> MISC_FEATURE
   <222> (233)..(233)
   <223> X= I or L
<220>
   <221> MISC_FEATURE
   <222> (237)..(237)
   <223> X= V or M
<220>
   <221> MISC_FEATURE
   <222> (240)..(240)
   <223> X= G or S
<220>
   <221> MISC_FEATURE
   <222> (260)..(260)
   <223> X= L, M, H, Q or V
<220>
   <221> MISC_FEATURE
   <222> (261)..(261)
   <223> X= G, T, A, S
<220>
   <221> MISC_FEATURE
   <222> (263)..(263)
   <223> X= I, V, T, S or A
<220>
   <221> MISC_FEATURE
   <222> (264)..(264)
   <223> X= T, S, N, Q, H or A
<220>
   <221> MISC_FEATURE
   <222> (267)..(267)
   <223> X= K, R or S
<220>
   <221> MISC_FEATURE
   <222> (271)..(271)
   <223> X= D, A, S or E
<220>
   <221> MISC_FEATURE
   <222> (274)..(274)
   <223> X= G or S
<220>
   <221> MISC_FEATURE
   <222> (277)..(277)
   <223> X= F or L
<220>
   <221> MISC_FEATURE
   <222> (280)..(280)
   <223> X= I or V
<220>
   <221> MISC_FEATURE
   <222> (286)..(286)
   <223> X= R or K
<220>
   <221> MISC_FEATURE
   <222> (289)..(289)
   <223> X= E or A
<220>
   <221> MISC_FEATURE
   <222> (293)..(293)
   <223> X= K or Q
<220>
   <221> MISC_FEATURE
   <222> (297)..(297)
   <223> X= T, G or S
<220>
   <221> MISC_FEATURE
   <222> (298)..(298)
   <223> X= F or W
<220>
   <221> MISC_FEATURE
   <222> (306)..(306)
   <223> X= P, H or V
<220>
   <221> MISC_FEATURE
   <222> (307)..(307)
   <223> X= S, T, E or A
<220>
   <221> MISC_FEATURE
   <222> (308)..(308)
   <223> X= S or T
<220>
   <221> MISC_FEATURE
   <222> (310)..(310)
   <223> X= C, T, V, L, E or S
<220>
   <221> MISC_FEATURE
   <222> (314)..(314)
   <223> X= S or N
<220>
   <221> MISC_FEATURE
   <222> (315)..(315)
   <223> X= S, Q, A or K
<220>
   <221> MISC_FEATURE
   <222> (316)..(316)
   <223> X= G, S, E or D
<220>
   <221> MISC_FEATURE
   <222> (317)..(317)
   <223> X= G, S, A or T
<220>
   <221> MISC_FEATURE
   <222> (318)..(318)
   <223> X= A or no amino acid
<220>
   <221> MISC_FEATURE
   <222> (319)..(319)
   <223> X= L, M, Q or R
<220>
   <221> MISC_FEATURE
   <222> (321)..(321)
   <223> X= S, G or T
<220>
   <221> MISC_FEATURE
   <222> (322)..(322)
   <223> X= Y or F
<220>
   <221> MISC_FEATURE
   <222> (324)..(324)
   <223> X= K or A
<220>
   <221> MISC_FEATURE
   <222> (325)..(325)
   <223> X= I or V
<220>
   <221> MISC_FEATURE
   <222> (328)..(328)
   <223> X= D, E, Q or N
<220>
   <221> MISC_FEATURE
   <222> (331)..(331)
   <223> X= C, K or Q
<220>
   <221> MISC_FEATURE
   <222> (333)..(333)
   <223> X= T, M or E
<220>
   <221> MISC_FEATURE
   <222> (336)..(336)
   <223> X= K, V, H, A or Q
<220>
   <221> MISC_FEATURE
   <222> (338)..(338)
   <223> X= N, S, D, T or K
<220>
   <221> MISC_FEATURE
   <222> (339)..(339)
   <223> X= K, R or S
<220>
   <221> MISC_FEATURE
   <222> (343)..(343)
   <223> X= I or V
<220>
   <221> MISC_FEATURE
   <222> (354)..(354)
   <223> X= V or L
<220>
   <221> MISC_FEATURE
   <222> (355)..(355)
   <223> X= E, R, S, G or Q
<220>
   <221> MISC_FEATURE
   <222> (358)..(358)
   <223> X= R or K
<220>
   <221> MISC_FEATURE
   <222> (359)..(359)
   <223> X= K, E, D or T
<220>
   <221> MISC_FEATURE
   <222> (360)..(360)
   <223> X= F or Y
<220>
   <221> MISC_FEATURE
   <222> (362)..(362)
   <223> X= D, A, Q, N or E
<220>
   <221> MISC_FEATURE
   <222> (363)..(363)
   <223> X= R or Q
<220>
   <221> MISC_FEATURE
   <222> (374)..(374)
   <223> X= A or S
<220>
   <221> MISC_FEATURE
   <222> (375)..(375)
   <223> X= V or A
<220>
   <221> MISC_FEATURE
   <222> (383)..(383)
   <223> X= I or V
<220>
   <221> MISC_FEATURE
   <222> (385)..(385)
   <223> X= G or D
<220>
   <221> MISC_FEATURE
   <222> (387)..(387)
   <223> X= K, R or H
<220>
   <221> MISC_FEATURE
   <222> (389)..(389)
   <223> X= I or V
<220>
   <221> MISC_FEATURE
   <222> (404)..(404)
   <223> X= V or L
<220>
   <221> MISC_FEATURE
   <222> (405)..(405)
   <223> X= I or L
<220>
   <221> MISC_FEATURE
   <222> (408)..(408)
   <223> X= I or V
<220>
   <221> MISC_FEATURE
   <222> (412)..(412)
   <223> X= K, N or S
<220>
   <221> MISC_FEATURE
   <222> (416)..(416)
   <223> X= L or M
<220>
   <221> MISC_FEATURE
   <222> (418)..(418)
   <223> X= A or T
<220>
   <221> MISC_FEATURE
   <222> (419)..(419)
   <223> X=I or V
<220>
   <221> MISC_FEATURE
   <222> (422)..(422)
   <223> X=A or G
<220>
   <221> MISC_FEATURE
   <222> (424)..(424)
   <223> X= I or L
<220>
   <221> MISC_FEATURE
   <222> (435)..(435)
   <223> X= A or S
<220>
   <221> MISC_FEATURE
   <222> (439)..(439)
   <223> X= A or S
<220>
   <221> MISC_FEATURE
   <222> (440)..(440)
   <223> X= Y or F
<220>
   <221> MISC_FEATURE
   <222> (441)..(441)
   <223> X= L or M
<220>
   <221> MISC_FEATURE
   <222> (444)..(444)
   <223> X= I or V
<220>
   <221> MISC_FEATURE
   <222> (446)..(446)
   <223> X= E, D, N, Q or T
<220>
   <221> MISC_FEATURE
   <222> (448)..(448)
   <223> X= V, I or T
<220>
   <221> MISC_FEATURE
   <222> (449)..(449)
   <223> X= I or V
<220>
   <221> MISC_FEATURE
   <222> (451)..(451)
   <223> X= T or A
<220>
   <221> MISC_FEATURE
   <222> (460)..(460)
   <223> X= Q or L
<220>
   <221> MISC_FEATURE
   <222> (463)..(463)
   <223> X= Y, F or H
<220>
   <221> MISC_FEATURE
   <222> (466)..(466)
   <223> X= Y or H
<220>
   <221> MISC_FEATURE
   <222> (468)..(468)
   <223> X= Y or H
<220>
   <221> MISC_FEATURE
   <222> (469)..(469)
   <223> X= N, Q, S or E
<220>
   <221> MISC_FEATURE
   <222> (470)..(470)
   <223> X= D, G, Q, K, S, E or no amino acid
<220>
   <221> MISC_FEATURE
   <222> (473)..(473)
   <223> X= S, T, C, A or V
<220>
   <221> MISC_FEATURE
   <222> (474)..(474)
   <223> X= A, V or T
<220>
   <221> MISC_FEATURE
   <222> (481)..(481)
   <223> X= N, S, T or A
<220>
   <221> MISC_FEATURE
   <222> (482)..(482)
   <223> X=A, G or V
<220>
   <221> MISC_FEATURE
   <222> (483)..(483)
   <223> X= V, Q, L or T
<220>
   <221> MISC_FEATURE
   <222> (484)..(484)
   <223> X= G or S
<220>
   <221> MISC_FEATURE
   <222> (485)..(485)
   <223> X= V, A or T
<220>
   <221> MISC_FEATURE
   <222> (486)..(486)
   <223> X= E, A, T, V, P or Q
<220>
   <221> MISC_FEATURE
   <222> (487)..(487)
   <223> X= L, F or S
<220>
   <221> MISC_FEATURE
   <222> (488)..(488)
   <223> X= T, E or Q
<220>
   <221> MISC_FEATURE
   <222> (491)..(491)
   <223> X= E, T, Q, A, S or G
<220>
   <221> MISC_FEATURE
   <222> (492)..(492)
   <223> X= K, Q, S, M, A, T or E
<220>
   <221> MISC_FEATURE
   <222> (493)..(493)
   <223> X= L or M
<220>
   <221> MISC_FEATURE
   <222> (495)..(495)
   <223> X= I or L
<220>
   <221> MISC_FEATURE
   <222> (499)..(499)
   <223> X= I, L or V
<220>
   <221> MISC_FEATURE
   <222> (500)..(500)
   <223> X= V, L or I
<220>
   <221> MISC_FEATURE
   <222> (501)..(501)
   <223> X= K or R
<220>
   <221> MISC_FEATURE
   <222> (503)..(503)
   <223> X= R, H, Q or E
<220>
   <221> MISC_FEATURE
   <222> (505)..(505)
   <223> X= E or K
<220>
   <221> MISC_FEATURE
   <222> (506)..(506)
   <223> X= K or T
<220>
   <221> MISC_FEATURE
   <222> (507)..(507)
   <223> X= L, V or I
<220>
   <221> MISC_FEATURE
   <222> (509)..(509)
   <223> X= I or L
<220>
   <221> MISC_FEATURE
   <222> (511)..(511)
   <223> X= N or C
<220>
   <221> MISC_FEATURE
   <222> (516)..(516)
   <223> X= M or L
<220>
   <221> MISC_FEATURE
   <222> (517)..(517)
   <223> X= P or A
<220>
   <221> MISC_FEATURE
   <222> (518)..(518)
   <223> X= E, Q or Y
<220>
   <221> MISC_FEATURE
   <222> (520)..(520)
   <223> X= A, T, Q, S or L
<220>
   <221> MISC_FEATURE
   <222> (521)..(521)
   <223> X= K, Q, A, L or V
<220>
   <221> MISC_FEATURE
   <222> (522)..(522)
   <223> X= V, A or T
<220>
   <221> MISC_FEATURE
   <222> (524)..(524)
   <223> X= E or D
<220>
   <221> MISC_FEATURE
   <222> (525)..(525)
   <223> X= S, A, K, N or T
<220>
   <221> MISC_FEATURE
   <222> (526)..(526)
   <223> X= L, M or R
<220>
   <221> MISC_FEATURE
   <222> (530)..(530)
   <223> X= L or V
<220>
   <221> MISC_FEATURE
   <222> (541)..(541)
   <223> X= E, D, T or K
<220>
   <221> MISC_FEATURE
   <222> (542)..(542)
   <223> X= A, T, E, S or Q
<220>
   <221> MISC_FEATURE
   <222> (544)..(544)
   <223> X= I, V, L or T
<220>
   <221> MISC_FEATURE
   <222> (545)..(545)
   <223> X= L, M, A or Q
<220>
   <221> MISC_FEATURE
   <222> (546)..(546)
   <223> X= E, Q or S
<220>
   <221> MISC_FEATURE
   <222> (547)..(547)
   <223> X= M or L
<220>
   <221> MISC_FEATURE
   <222> (549)..(549)
   <223> X= A, S, G or Q
<220>
   <221> MISC_FEATURE
   <222> (550)..(550)
   <223> X=S, Q, R, E or K
<220>
   <221> MISC_FEATURE
   <222> (552)..(552)
   <223> X= E, D, Q or S
<220>
   <221> MISC_FEATURE
   <222> (553)..(553)
   <223> X= A, V, S, T or L
<220>
   <221> MISC_FEATURE
   <222> (554)..(554)
   <223> X= L or F
<220>
   <221> MISC FEATURE
   <222> (555)..(555)
   <223> X= I or V
<220>
   <221> MISC_FEATURE
   <222> (557)..(557)
   <223> X= V, I or L
<220>
   <221> MISC_FEATURE
   <222> (560)..(560)
   <223> X= N or G
<220>
   <221> MISC FEATURE
   <222> (562)..(562)
   <223> X= I or V
<220>
   <221> MISC_FEATURE
   <222> (563)..(563)
   <223> X= M or K
<220>
   <221> MISC_FEATURE
   <222> (573)..(573)
   <223> X= V, L, F or Y
<220>
   <221> MISC_FEATURE
   <222> (574)..(574)
   <223> X= L or V
<220>
   <221> MISC_FEATURE
   <222> (576)..(576)
   <223> X= A, Q or S
<220>
   <221> MISC_FEATURE
   <222> (577)..(577)
   <223> X= H, N, K, R or E
<220>
   <221> MISC_FEATURE
   <222> (578)..(578)
   <223> X= R or K
<220>
   <221> MISC_FEATURE
   <222> (579)..(579)
   <223> X= K, R, L or I
<220>
   <221> MISC_FEATURE
   <222> (580)..(580)
   <223> X= P, A, W, G or L
<220>
   <221> MISC_FEATURE
   <222> (581)..(581)
   <223> X= V or I
<220>
   <221> MISC_FEATURE
   <222> (582)..(582)
   <223> X= P or S
<220>
   <221> MISC_FEATURE
   <222> (586)..(586)
   <223> X= I or L
<220>
   <221> MISC_FEATURE
   <222> (589)..(589)
   <223> X= P or A
<220>
   <221> MISC_FEATURE
   <222> (591)..(591)
   <223> X= F, R, H, Y, L, E, I, V or S
<220>
   <221> MISC_FEATURE
   <222> (593)..(593)
   <223> X= I or V
<220>
   <221> MISC_FEATURE
   <222> (594)..(594)
   <223> X= P or S
<220>
   <221> MISC_FEATURE
   <222> (595)..(595)
   <223> X= Q or P
<220>
   <221> MISC_FEATURE
   <222> (597)..(597)
   <223> X= T, E or S
<220>
   <221> MISC_FEATURE
   <222> (599)..(599)
   <223> X= E, D, A or Q
<220>
   <221> MISC_FEATURE
   <222> (601)..(601)
   <223> X= M, A, I, L or V
<220>
   <221> MISC_FEATURE
   <222> (602)..(602)
   <223> X= R or H
<220>
   <221> MISC_FEATURE
   <222> (603)..(603)
   <223> X= A, S, H, T or V
<220>
   <221> MISC_FEATURE
   <222> (604)..(604)
   <223> X= E or D
<220>
   <221> MISC_FEATURE
   <222> (605)..(605)
   <223> X= L, I or Y
<220>
   <221> MISC_FEATURE
   <222> (606)..(606)
   <223> X= G, Q or S
<220>
   <221> MISC_FEATURE
   <222> (608)..(608)
   <223> X= D, N or T
<220>
   <221> MISC_FEATURE
   <222> (609)..(609)
   <223> X= A or I
<220>
   <221> MISC_FEATURE
   <222> (610)..(610)
   <223> X= A, S, N, D or E
<220>
   <221> MISC_FEATURE
   <222> (612)..(612)
   <223> X= M or I
<220>
   <221> MISC_FEATURE
   <222> (613)..(613)
   <223> X= E, Q, R, K or D
<220>
   <221> MISC_FEATURE
   <222> (614)..(614)
   <223> X= A, T, Q, R or N
<220>
   <221> MISC_FEATURE
   <222> (615)..(615)
   <223> X= K, R or Q
<220>
   <221> MISC_FEATURE
   <222> (616)..(616)
   <223> X= I or V
<220>
   <221> MISC_FEATURE
   <222> (617)..(617)
   <223> X= K, R, E, T or S
<220>
   <221> MISC_FEATURE
   <222> (618)..(618)
   <223> X= A, T, D, R, N, Q or K
<220>
   <221> MISC_FEATURE
   <222> (619)..(619)
   <223> X= W or Y
<220>
   <221> MISC_FEATURE
   <222> (620)..(620)
   <223> X= L, Q or M
<400> 22
   Met Xaa Xaa Xaa Xaa Xaa Lys Tyr Pro Thr Leu Ala Xaa Xaa Xaa Xaa
   1               5                   10                  15
   Xaa Xaa Xaa Leu Arg Xaa Leu Pro Xaa Xaa Xaa Leu Pro Xaa Leu Xaa
               20                  25                  30
   Xaa Glu Leu Arg Xaa Xaa Leu Leu Xaa Xaa Val Ser Xaa Ser Ser Gly
           35                  40                  45
   His Phe Ala Ser Gly Leu Gly Xaa Val Glu Leu Thr Val Ala Xaa His
       50                  55                  60
   Tyr Val Tyr Xaa Thr Pro Phe Asp Xaa Xaa Xaa Trp Asp Val Gly His
   65                  70                  75                  80
   Gln Ala Tyr Pro His Lys Ile Leu Thr Gly Arg Arg Asp Xaa Ile Xaa
                   85                  90                  95
   Xaa Ile Arg Gln Lys Xaa Gly Xaa His Pro Phe Pro Trp Arg Xaa Glu
               100                 105                 110
   Ser Xaa Xaa Asp Xaa Leu Xaa Val Gly His Ser Ser Thr Ser Ile Ser
           115                 120                 125
   Ala Gly Xaa Gly Xaa Ala Xaa Ala Ala Xaa Xaa Glu Xaa Xaa Xaa Arg
       130                 135                 140
   Xaa Xaa Xaa Cys Val Ile Gly Asp Gly Ala Ile Thr Ala Gly Met Ala
   145                 150                 155                 160
   Phe Glu Ala Met Xaa His Ala Gly Asp Ile Xaa Xaa Asp Xaa Leu Val
                   165                 170                 175
   Xaa Leu Asn Asp Asn Xaa Met Ser Ile Ser Glu Asn Val Gly Xaa Leu
               180                 185                 190
   Asn Asn Xaa Leu Ala Gln Xaa Leu Ser Gly Lys Xaa Tyr Xaa Xaa Leu
           195                 200                 205
   Arg Glu Xaa Gly Lys Xaa Xaa Xaa Xaa Xaa Xaa Pro Pro Ile Lys Xaa
       210                 215                 220
   Leu Xaa Xaa Xaa Thr Glu Glu His Xaa Lys Gly Met Xaa Val Pro Xaa
   225                 230                 235                 240
   Thr Leu Phe Glu Glu Leu Gly Phe Asn Tyr Ile Gly Pro Val Asp Gly
                   245                 250                 255
   His Asp Val Xaa Xaa Leu Xaa Xaa Thr Leu Xaa Asn Met Arg Xaa Leu
   260 265 270
   Lys Xaa Pro Gln Xaa Leu His Xaa Met Thr Lys Lys Gly Xaa Gly Tyr
   275 280 285
   Xaa Pro Ala Glu Xaa Asp Pro Ile Xaa Xaa His Ala Val Pro Lys Phe
   290 295 300
   Asp Xaa Xaa Xaa Gly Xaa Leu Pro Lys Xaa Xaa Xaa Xaa Xaa Xaa Pro
   305 310 315 320
   Xaa Xaa Ser Xaa Xaa Phe Gly Xaa Trp Leu Xaa Glu Xaa Ala Ala Xaa
   325 330 335
   Asp Xaa Xaa Leu Met Ala Xaa Thr Pro Ala Met Arg Glu Gly Ser Gly
   340 345 350
   Met Xaa Xaa Phe Ser Xaa Xaa Xaa Pro Xaa Xaa Tyr Phe Asp Val Ala
   355 360 365
   Ile Ala Glu Gln His Xaa Xaa Thr Phe Ala Ala Gly Leu Ala Xaa Gly
   370 375 380
   Xaa Tyr Xaa Pro Xaa Val Ala Ile Tyr Ser Thr Phe Leu Gln Arg Ala
   385 390 395 400
   Tyr Asp Gln Xaa Xaa His Asp Xaa Ala Ile Gln Xaa Leu Pro Val Xaa
   405 410 415
   Phe Xaa Xaa Asp Arg Xaa Gly Xaa Val Gly Ala Asp Gly Gln Thr His
   420 425 430
   Gln Gly Xaa Phe Asp Leu Xaa Xaa Xaa Arg Cys Xaa Pro Xaa Met Xaa
   435 440 445
   Xaa Met Xaa Pro Ser Asp Glu Asn Glu Cys Arg Xaa Met Leu Xaa Thr
   450 455 460
   Gly Xaa His Xaa Xaa Xaa Gly Pro Xaa Xaa Val Arg Tyr Pro Arg Gly
   465 470 475 480
   Xaa Xaa Xaa Xaa Xaa Xaa Xaa Xaa Pro Leu Xaa Xaa Xaa Pro Xaa Gly
   485 490 495
   Lys Gly Xaa Xaa Xaa Arg Xaa Gly Xaa Xaa Xaa Ala Xaa Leu Xaa Phe
   500 505 510
   Gly Thr Leu Xaa Xaa Xaa Ala Xaa Xaa Xaa Ala Xaa Xaa Xaa Asn Ala
   515 520 525
   Thr Xaa Val Asp Met Arg Phe Val Lys Pro Leu Asp Xaa Xaa Leu Xaa
   530 535 540
   Xaa Xaa Xaa Ala Xaa Xaa His Xaa Xaa Xaa Xaa Thr Xaa Glu Glu Xaa
   545 550 555 560
   Ala Xaa Xaa Gly Gly Ala Gly Ser Gly Val Asn Glu Xaa Xaa Met Xaa
   565 570 575
   Xaa Xaa Xaa Xaa Xaa Xaa Val Leu Asn Xaa Gly Leu Xaa Asp Xaa Phe
   580 585 590
   Xaa Xaa Xaa Gly Xaa Gln Xaa Glu Xaa Xaa Xaa Xaa Xaa Xaa Leu Xaa
   595 600 605
   Xaa Xaa Gly Xaa Xaa Xaa Xaa Xaa Xaa Xaa Xaa Xaa Ala
   610 615 620
<210> 23
   <211> 657
   <212> PRT
   <213> Artificial sequence
<220>
   <223> Plant DXS consensus sequence
<220>
   <221> MISC_FEATURE
   <222> (7)..(7)
   <223> X= I or V
<220>
   <221> MISC_FEATURE
   <222> (11)..(11)
   <223> X= I or V
<220>
   <221> MISC_FEATURE
   <222> (13)..(13)
   <223> X= M, L or I
<220>
   <221> MISC_FEATURE
   <222> (16)..(16)
   <223> X= L or F
<220>
   <221> MISC_FEATURE
   <222> (17)..(17)
   <223> X= S or N
<220>
   <221> MISC_FEATURE
   <222> (18)..(18)
   <223> X= V, I, N or M
<220>
   <221> MISC_FEATURE
   <222> (19)..(19)
   <223> X= K, R, E or P
<220>
   <221> MISC_FEATURE
   <222> (20)..(20)
   <223> X= E or Q
<220>
   <221> MISC_FEATURE
   <222> (22)..(22)
   <223> X= K or R
<220>
   <221> MISC_FEATURE
   <222> (25)..(25)
   <223> X= A, S or C
<220>
   <221> MISC_FEATURE
   <222> (26)..(26)
   <223> X= D, N or K
<220>
   <221> MISC_FEATURE
   <222> (30)..(30)
   <223> X= S or A
<220>
   <221> MISC_FEATURE
   <222> (31)..(31)
   <223> X= D or E
<220>
   <221> MISC_FEATURE
   <222> (32)..(32)
   <223> X= V or I
<220>
   <221> MISC_FEATURE
   <222> (33)..(33)
   <223> X= V or I
<220>
   <221> MISC_FEATURE
   <222> (34)..(34)
   <223> X= F or H
<220>
   <221> MISC_FEATURE
   <222> (35)..(35)
   <223> X= N, E, T or G
<220>
   <221> MISC_FEATURE
   <222> (38)..(38)
   <223> X= K or R
<220>
   <221> MISC_FEATURE
   <222> (44)..(44)
   <223> X= G or S
<220>
   <221> MISC_FEATURE
   <222> (45)..(45)
   <223> X= S or A
<220>
   <221> MISC_FEATURE
   <222> (56)..(56)
   <223> X= L or M
<220>
   <221> MISC_FEATURE
   <222> (59)..(59)
   <223> X= V or I
<220>
   <221> MISC_FEATURE
   <222> (61)..(61)
   <223> X= N, D or S
<220>
   <221> MISC_FEATURE
   <222> (62)..(62)
   <223> X= A or T
<220>
   <221> MISC_FEATURE
   <222> (64)..(64)
   <223> X= Q, E or D
<220>
   <221> MISC_FEATURE
   <222> (66)..(66)
   <223> X= R or K
<220>
   <221> MISC_FEATURE
   <222> (67)..(67)
   <223> X= I or F
<220>
   <221> MISC_FEATURE
   <222> (68)..(68)
   <223> X= L or I
<220>
   <221> MISC_FEATURE
   <222> (75)..(75)
   <223> X= S or A
<220>
   <221> MISC_FEATURE
   <222> (77)..(77)
   <223> X= P, G or V
<220>
   <221> MISC_FEATURE
   <222> (81)..(81)
   <223> X= L or M
<220>
   <221> MISC_FEATURE
   <222> (86)..(86)
   <223> X= D, G or K
<220>
   <221> MISC_FEATURE
   <222> (87)..(87)
   <223> X= Q, K or G
<220>
   <221> MISC_FEATURE
   <222> (89)..(89)
   <223> X= H, P, A or Q
<220>
   <221> MISC_FEATURE
   <222> (91)..(91)
   <223> X= M, L or I
<220>
   <221> MISC_FEATURE
   <222> (93)..(93)
   <223> X= Q or K
<220>
   <221> MISC_FEATURE
   <222> (95)..(95)
   <223> X= D, N or G
<220>
   <221> MISC_FEATURE
   <222> (98)..(98)
   <223> X= A or S
<220>
   <221> MISC_FEATURE
   <222> (104)..(104)
   <223> X= S, G, D or A
<220>
   <221> MISC_FEATURE
   <222> (108)..(108)
   <223> X= Y or H
<220>
   <221> MISC_FEATURE
   <222> (110)..(110)
   <223> X= C or P
<220>
   <221> MISC_FEATURE
   <222> (113)..(113)
   <223> X= T or A
<220>
   <221> MISC_FEATURE
   <222> (119)..(119)
   <223> X= T or S
<220>
   <221> MISC_FEATURE
   <222> (123)..(123)
   <223> X= G or A
<220>
   <221> MISC_FEATURE
   <222> (128)..(128)
   <223> X= V or A
<220>
   <221> MISC_FEATURE
   <222> (132)..(132)
   <223> X= L, V or T
<220>
   <221> MISC_FEATURE
   <222> (134)..(134)
   <223> X= G or N
<220>
   <221> MISC_FEATURE
   <222> (135)..(135)
   <223> X= K or R
<220>
   <221> MISC_FEATURE
   <222> (136)..(136)
   <223> X= N or K
<220>
   <221> MISC FEATURE
   <222> (138)..(138)
   <223> X= N, H, S or Q
<220>
   <221> MISC_FEATURE
   <222> (140)..(140)
   <223> X= I or V
<220>
   <221> MISC_FEATURE
   <222> (141)..(141)
   <223> X= A or S
<220>
   <221> MISC_FEATURE
   <222> (148)..(148)
   <223> X= M or I
<220>
   <221> MISC_FEATURE
   <222> (150)..(150)
   <223> X= A or G
<220>
   <221> MISC_FEATURE
   <222> (152)..(152)
   <223> X= Q or M
<220>
   <221> MISC_FEATURE
   <222> (154)..(154)
   <223> X= Y or F
<220>
   <221> MISC_FEATURE
   <222> (159)..(159)
   <223> X= N or H
<220>
   <221> MISC_FEATURE
   <222> (161)..(161)
   <223> X= G or R
<220>
   <221> MISC_FEATURE
   <222> (162)..(162)
   <223> X= Y or F
<220>
   <221> MISC_FEATURE
   <222> (165)..(165)
   <223> X= S or K
<220>
   <221> MISC_FEATURE
   <222> (166)..(166)
   <223> X= D or N
<220>
   <221> MISC_FEATURE
   <222> (175)..(175)
   <223> X= K or Q
<220>
   <221> MISC_FEATURE
   <222> (182)..(182)
   <223> X= A or Q
<220>
   <221> MISC_FEATURE
   <222> (183)..(183)
   <223> X= T, N or Y
<220>
   <221> MISC_FEATURE
   <222> (184)..(184)
   <223> X= L or N
<220>
   <221> MISC_FEATURE
   <222> (185)..(185)
   <223> X= D, N or S
<220>
   <221> MISC_FEATURE
   <222> (186)..(186)
   <223> X= G or K
<220>
   <221> MISC_FEATURE
   <222> (187)..(187)
   <223> X= P or N
<220>
   <221> MISC_FEATURE
   <222> (188)..(188)
   <223> X= I, S or Q
<220>
   <221> MISC_FEATURE
   <222> (189)..(189)
   <223> X= P, D or A
<220>
   <221> MISC_FEATURE
   <222> (194)..(194)
   <223> X= L or M
<220>
   <221> MISC_FEATURE
   <222> (195)..(195)
   <223> X= S or A
<220>
   <221> MISC_FEATURE
   <222> (196)..(196)
   <223> X= S, G or no amino acid
<220>
   <221> MISC_FEATURE
   <222> (197)..(197)
   <223> X= A, T or no amino acid
<220>
   <221> MISC_FEATURE
   <222> (198)..(198)
   <223> X= L or no amino acid
<220>
   <221> MISC_FEATURE
   <222> (199)..(199)
   <223> X= S, A or no amino acid
<220>
   <221> MISC FEATURE
   <222> (200)..(200)
   <223> X= R or K
<220>
   <221> MISC_FEATURE
   <222> (201)..(201)
   <223> X= L or I
<220>
   <221> MISC FEATURE
   <222> (203)..(203)
   <223> X= S or A
<220>
   <221> MISC_FEATURE
   <222> (204)..(204)
   <223> X= N or S
<220>
   <221> MISC_FEATURE
   <222> (205)..(205)
   <223> X= R, P or K
<220>
   <221> MISC FEATURE
   <222> (206)..(206)
   <223> X= P or A
<220>
   <221> MISC_FEATURE
   <222> (213)..(213)
   <223> X= V or I
<220>
   <221> MISC_FEATURE
   <222> (217)..(217)
   <223> X= V or M
<220>
   <221> MISC FEATURE
   <222> (221)..(221)
   <223> X= I or L
<220>
   <221> MISC_FEATURE
   <222> (222)..(222)
   <223> X= G or P
<220>
   <221> MISC_FEATURE
   <222> (223)..(223)
   <223> X= G, A, D or T
<220>
   <221> MISC_FEATURE
   <222> (224)..(224)
   <223> X= P, V or A
<220>
   <221> MISC_FEATURE
   <222> (225)..(225)
   <223> X= M or V
<220>
   <221> MISC_FEATURE
   <222> (226)..(226)
   <223> X= H or Q
<220>
   <221> MISC_FEATURE
   <222> (227)..(227)
   <223> X= E, Q, K or N
<220>
   <221> MISC_FEATURE
   <222> (228)..(228)
   <223> X= L or A
<220>
   <221> MISC_FEATURE
   <222> (229)..(229)
   <223> X= A or T
<220>
   <221> MISC_FEATURE
   <222> (232)..(232)
   <223> X= V or I
<220>
   <221> MISC_FEATURE
   <222> (234)..(234)
   <223> X= E or V
<220>
   <221> MISC_FEATURE
   <222> (243)..(243)
   <223> X= S or T
<220>
   <221> MISC_FEATURE
   <222> (244)..(244)
   <223> X= G or R
<220>
   <221> MISC_FEATURE
   <222> (246)..(246)
   <223> X= T or S
<220>
   <221> MISC_FEATURE
   <222> (259)..(259)
   <223> X= V or L
<220>
   <221> MISC_FEATURE
   <222> (264)..(264)
   <223> X= I or L
<220>
   <221> MISC_FEATURE
   <222> (268)..(268)
   <223> X= V, L or I
<220>
   <221> MISC_FEATURE
   <222> (269)..(269)
   <223> X= A, T or S
<220>
   <221> MISC_FEATURE
   <222> (270)..(270)
   <223> X= I or V
<220>
   <221> MISC_FEATURE
   <222> (272)..(272)
   <223> X= K, R or S
<220>
   <221> MISC_FEATURE
   <222> (273)..(273)
   <223> X= E or D
<220>
   <221> MISC_FEATURE
   <222> (275)..(275)
   <223> X= K or R
<220>
   <221> MISC_FEATURE
   <222> (276)..(276)
   <223> X= S or A
<220>
   <221> MISC_FEATURE
   <222> (277)..(277)
   <223> X= T or A
<220>
   <221> MISC_FEATURE
   <222> (278)..(278)
   <223> X= K, R, H or E
<220>
   <221> MISC_FEATURE
   <222> (280)..(280)
   <223> X= T, V or I
<220>
   <221> MISC_FEATURE
   <222> (285)..(285)
   <223> X= I or V
<220>
   <221> MISC_FEATURE
   <222> (288)..(288)
   <223> X= V or I
<220>
   <221> MISC_FEATURE
   <222> (293)..(293)
   <223> X= R or H
<220>
   <221> MISC_FEATURE
   <222> (296)..(296)
   <223> X= P, L or E
<220>
   <221> MISC_FEATURE
   <222> (297)..(297)
   <223> X= Y or P
<220>
   <221> MISC FEATURE
   <222> (300)..(300)
   <223> X= K, R, T or A
<220>
   <221> MISC_FEATURE
   <222> (301)..(301)
   <223> X= A or S
<220>
   <221> MISC_FEATURE
   <222> (302)..(302)
   <223> X= A, D or Q
<220>
   <221> MISC_FEATURE
   <222> (305)..(305)
   <223> X= Y or M
<220>
   <221> MISC_FEATURE
   <222> (309)..(309)
   <223> X= T or V
<220>
   <221> MISC_FEATURE
   <222> (314)..(314)
   <223> X= A or R
<220>
   <221> MISC_FEATURE
   <222> (317)..(317)
   <223> X= K or R
<220>
   <221> MISC_FEATURE
   <222> (319)..(319)
   <223> X= F or V
<220>
   <221> MISC_FEATURE
   <222> (320)..(320)
   <223> X= K, Q or A
<220>
   <221> MISC_FEATURE
   <222> (321)..(321)
   <223> X= S, T, G or A
<220>
   <221> MISC_FEATURE
   <222> (322)..(322)
   <223> X= S, T or K
<220>
   <221> MISC_FEATURE
   <222> (323)..(323)
   <223> X= A, N, T or P
<220>
   <221> MISC_FEATURE
   <222> (324)..(324)
   <223> X= P, E or K
<220>
   <221> MISC_FEATURE
   <222> (325)..(325)
   <223> X= T or A
<220>
   <221> MISC_FEATURE
   <222> (326)..(326)
   <223> X= Q or M
<220>
   <221> MISC_FEATURE
   <222> (327)..(327)
   <223> X= S or T
<220>
   <221> MISC_FEATURE
   <222> (330)..(330)
   <223> X= T or N
<220>
   <221> MISC_FEATURE
   <222> (334)..(334)
   <223> X= E or D
<220>
   <221> MISC_FEATURE
   <222> (335)..(335)
   <223> X= A or S
<220>
   <221> MISC_FEATURE
   <222> (337)..(337)
   <223> X= I, V or T
<220>
   <221> MISC_FEATURE
   <222> (338)..(338)
   <223> X= A or S
<220>
   <221> MISC_FEATURE
   <222> (341)..(341)
   <223> X= E or K
<220>
   <221> MISC_FEATURE
   <222> (342)..(342)
   <223> X= V, A or R
<220>
   <221> MISC_FEATURE
   <222> (344)..(344)
   <223> X= K or S
<220>
   <221> MISC_FEATURE
   <222> (345)..(345)
   <223> X= D, N or R
<220>
   <221> MISC_FEATURE
   <222> (346)..(346)
   <223> X= I or V
<220>
   <221> MISC_FEATURE
   <222> (347)..(347)
   <223> X= V or M
<220>
   <221> MISC_FEATURE
   <222> (349)..(349)
   <223> X= I or V
<220>
   <221> MISC_FEATURE
   <222> (354)..(354)
   <223> X= G or A
<220>
   <221> MISC_FEATURE
   <222> (360)..(360)
   <223> X= N, Y or T
<220>
   <221> MISC_FEATURE
   <222> (361)..(361)
   <223> X= L, M or R
<220>
   <221> MISC_FEATURE
   <222> (363)..(363)
   <223> X= H, Q, S or E
<220>
   <221> MISC_FEATURE
   <222> (364)..(364)
   <223> X= R, K or N
<220>
   <221> MISC_FEATURE
   <222> (365)..(365)
   <223> X= R, K or A
<220>
   <221> MISC_FEATURE
   <222> (366)..(366)
   <223> X= F or L
<220>
   <221> MISC_FEATURE
   <222> (368)..(368)
   <223> X= T or D
<220>
   <221> MISC_FEATURE
   <222> (370)..(370)
   <223> X= C, T or V
<220>
   <221> MISC_FEATURE
   <222> (384)..(384)
   <223> X= A or S
<220>
   <221> MISC_FEATURE
   <222> (392)..(392)
   <223> X= I or L
<220>
   <221> MISC_FEATURE
   <222> (393)..(393)
   <223> X= K or V
<220>
   <221> MISC_FEATURE
   <222> (397)..(397)
   <223> X= A or T
<220>
   <221> MISC_FEATURE
   <222> (401)..(401)
   <223> X= S or T
<220>
   <221> MISC_FEATURE
   <222> (403)..(403)
   <223> X= M or L
<220>
   <221> MISC_FEATURE
   <222> (406)..(406)
   <223> X= A or G
<220>
   <221> MISC FEATURE
   <222> (410)..(410)
   <223> X= V or I
<220>
   <221> MISC_FEATURE
   <222> (411)..(411)
   <223> X= V or I
<220>
   <221> MISC_FEATURE
   <222> (415)..(415)
   <223> X= D, S or A
<220>
   <221> MISC_FEATURE
   <222> (418)..(418)
   <223> X= K or N
<220>
   <221> MISC_FEATURE
   <222> (422)..(422)
   <223> X= K or R
<220>
   <221> MISC_FEATURE
   <222> (430)..(430)
   <223> X= L or M
<220>
   <221> MISC_FEATURE
   <222> (436)..(436)
   <223> X= P, S or A
<220>
   <221> MISC_FEATURE
   <222> (444)..(444)
   <223> X= V or I
<220>
   <221> MISC_FEATURE
   <222> (445)..(445)
   <223> X= A or T
<220>
   <221> MISC_FEATURE
   <222> (446)..(446)
   <223> X= F or Y
<220>
   <221> MISC_FEATURE
   <222> (447)..(447)
   <223> X= M or L
<220>
   <221> MISC_FEATURE
   <222> (449)..(449)
   <223> X= C or S
<220>
   <221> MISC_FEATURE
   <222> (452)..(452)
   <223> X= N or H
<220>
   <221> MISC_FEATURE
   <222> (454)..(454)
   <223> X= V or I
<220>
   <221> MISC_FEATURE
   <222> (455)..(455)
   <223> X= V, T or C
<220>
   <221> MISC_FEATURE
   <222> (459)..(459)
   <223> X= A or S
<220>
   <221> MISC_FEATURE
   <222> (460)..(460)
   <223> X= D or N
<220>
   <221> MISC_FEATURE
   <222> (463)..(463)
   <223> X= E or D
<220>
   <221> MISC_FEATURE
   <222> (465)..(465)
   <223> X= F or I
<220>
   <221> MISC_FEATURE
   <222> (466)..(466)
   <223> X= H or N
<220>
   <221> MISC_FEATURE
   <222> (471)..(471)
   <223> X= A or C
<220>
   <221> MISC_FEATURE
   <222> (472)..(472)
   <223> X= A or V
<220>
   <221> MISC_FEATURE
   <222> (477)..(477)
   <223> X= R or A
<220>
   <221> MISC_FEATURE
   <222> (482)..(482)
   <223> X= R or G
<220>
   <221> MISC_FEATURE
   <222> (483)..(483)
   <223> X= Y or F
<220>
   <221> MISC_FEATURE
   <222> (489)..(489)
   <223> X= V, I or L
<220>
   <221> MISC_FEATURE
   <222> (491)..(491)
   <223> X= V, A or L
<220>
   <221> MISC_FEATURE
   <222> (492)..(492)
   <223> X= E, A, Q or D
<220>
   <221> MISC_FEATURE
   <222> (494)..(494)
   <223> X= P, A or E
<220>
   <221> MISC_FEATURE
   <222> (495)..(495)
   <223> X= P or A
<220>
   <221> MISC_FEATURE
   <222> (496)..(496)
   <223> X= G, Y, A or no amino acid
<220>
   <221> MISC_FEATURE
   <222> (497)..(497)
   <223> X= N or G
<220>
   <221> MISC_FEATURE
   <222> (498)..(498)
   <223> X= I or no amino acid
<220>
   <221> MISC_FEATURE
   <222> (499)..(499)
   <223> X= N, S, K or no amino acid
<220>
   <221> MISC_FEATURE
   <222> (500)..(500)
   <223> X= K or no amino acid
<220>
   <221> MISC_FEATURE
   <222> (501)..(501)
   <223> X= D or no amino acid
<220>
   <221> MISC_FEATURE
   <222> (502)..(502)
   <223> X= L, M or no amino acid
<220>
   <221> MISC_FEATURE
   <222> (505)..(505)
   <223> X= I, V or T
<220>
   <221> MISC_FEATURE
   <222> (507)..(507)
   <223> X= I or L
<220>
   <221> MISC_FEATURE
   <222> (509)..(509)
   <223> X= V or I
<220>
   <221> MISC_FEATURE
   <222> (511)..(511)
   <223> X= R or K
<220>
   <221> MISC_FEATURE
   <222> (513)..(513)
   <223> X= R or V
<220>
   <221> MISC_FEATURE
   <222> (514)..(514)
   <223> X= I or V
<220>
   <221> MISC_FEATURE
   <222> (515)..(515)
   <223> X= L or R
<220>
   <221> MISC_FEATURE
   <222> (516)..(516)
   <223> X= I, K, V or R
<220>
   <221> MISC_FEATURE
   <222> (517)..(517)
   <223> X= E or Q
<220>
   <221> MISC_FEATURE
   <222> (519)..(519)
   <223> X= E, D, K or T
<220>
   <221> MISC_FEATURE
   <222> (520)..(520)
   <223> X= R or D
<220>
   <221> MISC_FEATURE
   <222> (522)..(522)
   <223> X= A or C
<220>
   <221> MISC_FEATURE
   <222> (524)..(524)
   <223> X= L or V
<220>
   <221> MISC_FEATURE
   <222> (525)..(525)
   <223> X= G or A
<220>
   <221> MISC_FEATURE
   <222> (528)..(528)
   <223> X= T or S
<220>
   <221> MISC_FEATURE
   <222> (529)..(529)
   <223> X= A, V or S
<220>
   <221> MISC_FEATURE
   <222> (531)..(531)
   <223> X= Q or N
<220>
   <221> MISC_FEATURE
   <222> (532)..(532)
   <223> X= S, N or E
<220>
   <221> MISC_FEATURE
   <222> (533)..(533)
   <223> X= C or A
<220>
   <221> MISC_FEATURE
   <222> (534)..(534)
   <223> X= L or M
<220>
   <221> MISC_FEATURE
   <222> (535)..(535)
   <223> X= V, G or A
<220>
   <221> MISC_FEATURE
   <222> (537)..(537)
   <223> X= S or A
<220>
   <221> MISC_FEATURE
   <222> (538)..(538)
   <223> X= S, V, A, D or E
<220>
   <221> MISC_FEATURE
   <222> (539)..(539)
   <223> X= I, M, L or A
<220>
   <221> MISC_FEATURE
   <222> (541)..(541)
   <223> X= E or Q
<220>
   <221> MISC_FEATURE
   <222> (542)..(542)
   <223> X= Q, E, R or L
<220>
   <221> MISC_FEATURE
   <222> (543)..(543)
   <223> X= H, R, Q or D
<220>
   <221> MISC_FEATURE
   <222> (544)..(544)
   <223> X= G or D
<220>
   <221> MISC FEATURE
   <222> (545)..(545)
   <223> X= L or V
<220>
   <221> MISC_FEATURE
   <222> (546)..(546)
   <223> X= R, N or S
<220>
   <221> MISC_FEATURE
   <222> (547)..(547)
   <223> X= I, V, T or A
<220>
   <221> MISC_FEATURE
   <222> (550)..(550)
   <223> X= A or I
<220>
   <221> MISC_FEATURE
   <222> (560)..(560)
   <223> X= H, R or T
<220>
   <221> MISC_FEATURE
   <222> (561)..(561)
   <223> X= A, D or K
<220>
   <221> MISC_FEATURE
   <222> (563)..(563)
   <223> X= I or V
<220>
   <221> MISC_FEATURE
   <222> (565)..(565)
   <223> X= S or K
<220>
   <221> MISC_FEATURE
   <222> (566)..(566)
   <223> X= L or A
<220>
   <221> MISC_FEATURE
   <222> (568)..(568)
   <223> X= K, R or Q
<220>
   <221> MISC_FEATURE
   <222> (569)..(569)
   <223> X= S or E
<220>
   <221> MISC_FEATURE
   <222> (571)..(571)
   <223> X= E or P
<220>
   <221> MISC_FEATURE
   <222> (573)..(573)
   <223> X= L or M
<220>
   <221> MISC FEATURE
   <222> (574)..(574)
   <223> X= I or V
<220>
   <221> MISC FEATURE
   <222> (575)..(575)
   <223> X= T or S
<220>
   <221> MISC FEATURE
   <222> (576)..(576)
   <223> X= V or I
<220>
   <221> MISC_FEATURE
   <222> (580)..(580)
   <223> X= S, T or A
<220>
   <221> MISC FEATURE
   <222> (581)..(581)
   <223> X= I or V
<220>
   <221> MISC_FEATURE
   <222> (585)..(585)
   <223> X= G or A
<220>
   <221> MISC FEATURE
   <222> (586)..(586)
   <223> X= S or A
<220>
   <221> MISC_FEATURE
   <222> (589)..(589)
   <223> X= A, V or M
<220>
   <221> MISC_FEATURE
   <222> (590)..(590)
   <223> X= Q or H
<220>
   <221> MISC_FEATURE
   <222> (593)..(593)
   <223> X= A or T
<220>
   <221> MISC_FEATURE
   <222> (595)..(595)
   <223> X= N, D or E
<220>
   <221> MISC_FEATURE
   <222> (597)..(597)
   <223> X= L or F
<220>
   <221> MISC_FEATURE
   <222> (601)..(601)
   <223> X= T, K or G
<220>
   <221> MISC_FEATURE
   <222> (602)..(602)
   <223> X= T or L
<220>
   <221> MISC_FEATURE
   <222> (604)..(604)
   <223> X= W or F
<220>
   <221> MISC_FEATURE
   <222> (605)..(605)
   <223> X= S or R
<220>
   <221> MISC_FEATURE
   <222> (608)..(608)
   <223> X= V, T or C
<220>
   <221> MISC_FEATURE
   <222> (609)..(609)
   <223> X= L or M
<220>
   <221> MISC_FEATURE
   <222> (612)..(612)
   <223> X= R or H
<220>
   <221> MISC_FEATURE
   <222> (613)..(613)
   <223> X= Y or F
<220>
   <221> MISC_FEATURE
   <222> (615)..(615)
   <223> X= D or E
<220>
   <221> MISC_FEATURE
   <222> (618)..(618)
   <223> X= A or D
<220>
   <221> MISC_FEATURE
   <222> (619)..(619)
   <223> X= P or Y
<220>
   <221> MISC_FEATURE
   <222> (620)..(620)
   <223> X= A, S or R
<220>
   <221> MISC_FEATURE
   <222> (623)..(623)
   <223> X= L or M
<220>
   <221> MISC_FEATURE
   <222> (624)..(624)
   <223> X= A, I or N
<220>
   <221> MISC_FEATURE
   <222> (625)..(625)
   <223> X= M, E or L
<220>
   <221> MISC_FEATURE
   <222> (629)..(629)
   <223> X= T or M
<220>
   <221> MISC_FEATURE
   <222> (630)..(630)
   <223> X= P, A or S
<220>
   <221> MISC_FEATURE
   <222> (631)..(631)
   <223> X= S, Q or G
<220>
   <221> MISC_FEATURE
   <222> (635)..(635)
   <223> X= A, S or G
<220>
   <221> MISC_FEATURE
   <222> (636)..(636)
   <223> X= T or S
<220>
   <221> MISC_FEATURE
   <222> (637)..(637)
   <223> X= V or A
<220>
   <221> MISC_FEATURE
   <222> (638)..(638)
   <223> X= F or L
<220>
   <221> MISC_FEATURE
   <222> (639)..(639)
   <223> X= N, T or Q
<220>
   <221> MISC_FEATURE
   <222> (640)..(640)
   <223> X= I, L, M or T
<220>
   <221> MISC_FEATURE
   <222> (641)..(641)
   <223> X= I or L
<220>
   <221> MISC_FEATURE
   <222> (643)..(643)
   <223> X= Q, A or R
<220>
   <221> MISC_FEATURE
   <222> (644)..(644)
   <223> X= T, P, A or K
<220>
   <221> MISC_FEATURE
   <222> (645)..(645)
   <223> X= R, K or A
<220>
   <221> MISC_FEATURE
   <222> (646)..(646)
   <223> X= E, D or G
<220>
   <221> MISC_FEATURE
   <222> (648)..(648)
   <223> X= L or A
<220>
   <221> MISC_FEATURE
   <222> (649)..(649)
   <223> X= E, F, Q or K
<220>
   <221> MISC_FEATURE
   <222> (650)..(650)
   <223> X= I, V, F, Y or no amino acid
<220>
   <221> MISC_FEATURE
   <222> (651)..(651)
   <223> X= M, S, A or no amino acid
<220>
   <221> MISC_FEATURE
   <222> (652)..(652)
   <223> X= L, S, I or no amino acid
<220>
   <221> MISC_FEATURE
   <222> (653)..(653)
   <223> X= Sor no amino acid
<220>
   <221> MISC_FEATURE
   <222> (654)..(654)
   <223> X= A, N or no amino acid
<220>
   <221> MISC_FEATURE
   <222> (655)..(655)
   <223> X= L, T or no amino acid
<220>
   <221> MISC_FEATURE
   <222> (656)..(656)
   <223> X= Q, G or no amino acid
<220>
   <221> MISC_FEATURE
   <222> (657)..(657)
   <223> X= A, L or no amino acid
<400> 23
   Thr Pro Leu Leu Asp Thr Xaa Asn Tyr Pro Xaa His Xaa Lys Asn Xaa
   1               5                   10                  15
   Xaa Xaa Xaa Glx Leu Xaa Gln Leu Xaa Xaa Glu Leu Arg Xaa Xaa Xaa
               20                  25                  30
   Xaa Xaa Xaa Val Ser Xaa Thr Gly Gly His Leu Xaa Xaa Ser Leu Gly
           35                  40                  45
   Val Val Glu Leu Thr Val Ala Xaa His Tyr Xaa Phe Xaa Xaa Pro Xaa
       50                  55                  60
   Asp Xaa Xaa Xaa Trp Asp Val Gly His Gln Xaa Tyr Xaa His Lys Ile
   65                  70                  75                  80
   Xaa Thr Gly Arg Arg Xaa Xaa Met Xaa Thr Xaa Arg Xaa Thr Xaa Gly
   85 90 95
   Leu Xaa Gly Phe Thr Lys Arg Xaa Glu Ser Glu Xaa Asp Xaa Phe Gly
   100 105 110
   Xaa Gly His Ser Ser Thr Xaa Ile Ser Ala Xaa Leu Gly Met Ala Xaa
   115 120 125
   Gly Arg Asp Xaa Lys Xaa Xaa Xaa Asn Xaa Val Xaa Xaa Val Ile Gly
   130 135 140
   Asp Gly Ala Xaa Thr Xaa Gly Xaa Ala Xaa Glu Ala Met Asn Xaa Ala
   145 150 155 160
   Xaa Xaa Leu Asp Xaa Xaa Met Ile Val Ile Leu Asn Asp Asn Xaa Gln
   165 170 175
   Val Ser Leu Pro Thr Xaa Xaa Xaa Xaa Xaa Xaa Xaa Xaa Pro Val Gly
   180 185 190
   Ala Xaa Xaa Xaa Xaa Xaa Xaa Xaa Xaa Gln Xaa Xaa Xaa Xaa Leu Arg
   195 200 205
   Glu Leu Arg Glu Xaa Ala Lys Gly Xaa Thr Lys Gln Xaa Xaa Xaa Xaa
   210 215 220
   Xaa Xaa Xaa Xaa Xaa Ala Lys Xaa Asp Xaa Tyr Ala Arg Gly Met Ile
   225 230 235 240
   Ser Gly Xaa Xaa Ser Xaa Leu Phe Glu Glu Leu Gly Leu Tyr Tyr Ile
   245 250 255
   Gly Pro Xaa Asp Gly His Asn Xaa Asp Asp Leu Xaa Xaa Xaa Leu Xaa
   260 265 270
   Xaa Val Xaa Xaa Xaa Xaa Thr Xaa Gly Pro Val Leu Xaa His Val Xaa
   275 280 285
   Thr Glu Lys Gly Xaa Gly Tyr Xaa Xaa Ala Glu Xaa Xaa Xaa Asp Lys
   290 295 300
   Xaa His Gly Val Xaa Lys Phe Asp Pro Xaa Thr Gly Xaa Gln Xaa Xaa
   305 310 315 320
   Xaa Xaa Xaa Xaa Xaa Xaa Xaa Tyr Thr Xaa Tyr Phe Ala Xaa Xaa Leu
   325 330 335
   Xaa Xaa Glu Ala Xaa Xaa Asp Xaa Xaa Xaa Xaa Ala Xaa His Ala Ala
   340 345 350
   Met Xaa Gly Gly Thr Gly Leu Xaa Xaa Phe Xaa Xaa Xaa Xaa Pro Xaa
   355 360 365
   Arg Xaa Phe Asp Val Gly Ile Ala Glu Gln His Ala Val Thr Phe Xaa
   370 375 380
   Ala Gly Leu Ala Cys Glu Gly Xaa Xaa Pro Phe Cys Xaa Ile Tyr Ser
   385 390 395 400
   Xaa Phe Xaa Gln Arg Xaa Tyr Asp Gln Xaa Xaa His Asp Val Xaa Leu
   405 410 415
   Gln Xaa Leu Pro Val Xaa Phe Ala Met Asp Arg Ala Gly Xaa Val Gly
   420 425 430
   Ala Asp Gly Xaa Thr His Cys Gly Ala Phe Asp Xaa Xaa Xaa Xaa Ala
   435 440 445
   Xaa Leu Pro Xaa Met Xaa Xaa Met Ala Pro Xaa Asx Glu Ala Xaa Leu
   450 455 460
   Xaa Xaa Met Val Ala Thr Xaa Xaa Ala Ile Asp Asp Xaa Pro Ser Cys
   465 470 475 480
   Phe Xaa Xaa Pro Arg Gly Asn Gly Xaa Gly Xaa Xaa Leu Xaa Xaa Xaa
   485 490 495
   Xaa Xaa Xaa Xaa Xaa Xaa Lys Gly Xaa Pro Xaa Glu Xaa Gly Xaa Gly
   500 505 510
   Xaa Xaa Xaa Xaa Xaa Gly Xaa Xaa Val Xaa Leu Xaa Xaa Tyr Gly Xaa
   515 520 525
   Xaa Val Xaa Xaa Xaa Xaa Xaa Ala Xaa Xaa Xaa Leu Glx Xaa Xaa Xaa
   530 535 540
   Xaa Xaa Xaa Thr Val Xaa Asp Ala Arg Phe Cys Lys Pro Leu Asp Xaa
   545 550 555 560
   Xaa Leu Xaa Arg Xaa Xaa Ala Xaa Xaa His Xaa Val Xaa Xaa Xaa Xaa
   565 570 575
   Glu Glu Gly Xaa Xaa Gly Gly Phe Xaa Xaa His Val Xaa Xaa Phe Leu
   580 585 590
   Xaa Leu Xaa Gly Xaa Leu Asp Gly Xaa Xaa Lys Xaa Xaa Pro Met Xaa
   595 600 605
   Xaa Pro Asp Xaa Xaa Ile Xaa His Gly Xaa Xaa Xaa Asp Gln Xaa Xaa
   610 615 620
   Xaa Ala Gly Leu Xaa Xaa Xaa His Ile Ala Xaa Xaa Xaa Xaa Xaa Xaa
   625 630 635 640
   Xaa Gly Xaa Xaa Xaa Xaa Ala Xaa Xaa Xaa Xaa Xaa Xaa Xaa Xaa Xaa
   645 650 655
   Xaa
<210> 24
   <211> 72
   <212> DNA
   <213> Artificial sequence
<220>
   <223> PCR Primer
<400> 24
   ggggacaagt ttgtacaaaa aagcaggctt ggttccgcgt ggatcaatga gttttgatat 60
tgccaaatac cc 72
<210> 25
   <211> 57
   <212> DNA
   <213> Artificial sequence
<220>
   <223> PCR Primer
<400> 25
   ggggaccact ttgtacaaga aagctgggtt cattatgcca gccaggcctt gattttg 57
<210> 26
   <211> 36
   <212> DNA
   <213> Artificial sequence
<220>
   <223> Mutagenic Primer
<400> 26
   gcgcgaaggc gggaacaaag ttttctctgg cgtgcc 36
<210> 27
   <211> 36
   <212> DNA
   <213> Artificial sequence
<220>
   <223> Mutagenic Primer
<400> 27
   ggcacgccag agaaaacttt gttcccgcct tcgcgc 36
<210> 28
   <211> 38
   <212> DNA
   <213> Artificial sequence
<220>
   <223> Mutagenic Primer
<400> 28
   cgcaccgaag aacatatttg cggcatggta gtgcctgg 38
<210> 29
   <211> 38
   <212> DNA
   <213> Artificial sequence
<220>
   <223> Mutagenic Primer
<400> 29
   ccaggcacta ccatgccgca aatatgttct tcggtgcg 38

## Description

### BACKGROUND OF THE INVENTION

### Field of the invention

This invention relates to the use of a mutated isolated polypeptide having a 1-deoxy-D-xylulose 5-phosphate synthase (DXS) activity to increase terpenes production and/or accumulation in a host cell transformed by said mutated isolated polypeptide having a DXS activity.

Transgenic plants, bacteria or yeast that express this polypeptide, and are capable of a greatly enhanced accumulation of terpenes, in comparison to plants, bacteria or yeast expressing a typical DXS gene are also disclosed.

A method of production of terpenes by culturing transgenic plants, bacteria or yeast that express this polypeptide is also disclosed.

Finally, transgenic plants that express this polypeptide, and are resistant to clomazone are disclosed.

### Description of the related art

Isoprenoids (also known as terpenoids or terpenes) are considered to be the largest family of natural products occurring in nature, with over 29 000 individual compounds identified to date. Chemically, they are extremely diverse in their structure and complexity. Isoprenoids are involved in numerous fundamental biological functions and therefore, they are essential for the normal growth and developmental processes in all living organisms. For instance, isoprenoids include eukaryotic membrane stabilizers (sterols), animal and plant hormones (steroids, retinoids, gibberellins and abscisic acid), pigments for photosynthesis (carotenoids and phytol side chain of chlorophyll), and carriers for electron transport (menaquinone, plastoquinone and ubiquinone).

Consequently, isoprenoids are a large, diverse group of complex natural products with considerable commercial interest. Isoprenoids are today mostly extracted from plants or chemically synthesized to be used as pharmaceuticals (e.g. taxol, bisabolol and artemisinin), animal feed supplements and food colorants (various carotenoids such as lycopene and β-carotene) or flavors and fragrances (e.g. menthol, patchoulol, and nootkatone).

Isoprenoids are classified into groups according to the number of carbons they contain; the major groups of interest are monoterpenes (C₁₀), sesquiterpenes (C₁₅), diterpenes (C₂₀), and triterpenes (C₃₀).

All isoprenoids are synthesized via a common metabolic precursor, isopentenyl diphosphate (IPP; C₅). It was previously assumed that IPP was synthesized exclusively from mevalonate by the so-called « mevalonate pathway ». However, more recent investigations have shown that in eubacteria, green algae, and plants, IPP is also synthesized by a different pathway, designated the 2-C-methyl-D-erythritol-4-phosphate (MEP) pathway. Therefore, plants possess both the mevalonate and the MEP pathways, responsible respectively for the biosynthesis of IPP in the cytosol and in plastids.

The first intermediate of the MEP pathway is 1-deoxyxylulose-5-phosphate (DXP), whose biosynthesis from glyceraldehyde-3-phosphate (G3P) and pyruvate is catalyzed by the thiamine-dependent enzyme 1-deoxyxylulose-5-phosphate synthase (DXS). DXS has been shown to catalyze a rate-limiting step in the formation of isoprenoids in bacteria and in plants. Indeed, in *Arabidopsis thaliana* and tomato (*Lycopersicon esculentum*), the over-expression of DXS resulted in elevated levels of plastid-derived isoprenoids like carotenoids. This is also the case in aromatic plants such as spike lavender (*Lavandula latifolia*), where the over-expression of DXS led to a substantial increase in essential oil production.

Terpenoids are involved in aromas and fragrances of many plant-derived products, and, among them, monoterpenols contribute strongly to the aroma profiles of table grapes and wines. Floral flavors described as rose or lily of the valley, are related to the presence of molecules such as linalool, geraniol, nerol, alpha-terpineol or citronellol. The highest concentrations of these molecules are found in varieties of the "Muscat" group or in Gewurztraminer, leading to very characteristic aromas in these varieties. Floral flavors have appeared spontaneously in genotypes not related to the Muscat group.

### Deficiencies in the prior art

Certain diterpenoids have been commercially exploited, particularly in the pharmaceutical sector. This is true in particular for diterpenoids from the taxane class, paclitaxel and docetaxel, used in the treatment of breast and ovarian cancer. Paclitaxel is a natural molecule extracted from the yew (*Taxus* sp.), while docetaxel is a semi-synthetic molecule, derived from a paclitaxel precursor, 10-deacetyl baccatin III (or 10-DAB III), also extracted from yew. Most of the paclitaxel biosynthetic genes of yew have been described. These molecules are costly to produce due to the relatively low abundance of 10-DAB III and especially of paclitaxel in yew extracts, and due to the absence of a synthetic method that can be scaled up industrially, on account of the structural complexity of the molecules.

Thus there is a high demand for methods for producing terpenes of interest at a lower cost, but also for producing terpene derivatives that are not yet easily accessible to synthesis.

The chemical industry, which produces organic molecules by traditional chemical processes, is increasingly turning to production processes utilizing microbial cell factories. The key drivers for this development towards green chemistry are that such so-called "biotechnological processes" are more environmentally friendly, that many compounds produced by microorganisms are too complex to be obtained by organic synthesis and that the microbial cell factory represents an unlimited supply of the particular compound. Currently, isoprenoids are produced at large scale by extraction from plants or by chemical synthesis. The major drawbacks of both of these methods are low yields and high costs. A third option is to produce the desired isoprenoids by *in vitro* enzymatic conversion, but this approach is limited by the availability of precursors, and therefore in most cases not economically viable.

Metabolic engineering of microorganisms for isoprenoid production may lead to production of large amounts of isoprenoids from cheap carbon sources in fermentation processes, and thereby solve many of the current problems in industrial isoprenoid production, whilst allowing for biotechnological exploitation of the large diversity found in the isoprenoid group of natural compounds.

A number of isoprenoid products have been produced by genetically engineered microorganisms, including limonene, carotenoids, epi-cedrol, taxadiene, and others. In order to enhance isoprenoid production in *Escherichia coli,* the genes *dxs* (encoding DXP synthase), *dxr* (DXP reductoisomerase) and *idi* (IPP isomerase) have been overexpressed with good results for several of the above-mentioned isoprenoid products (reviewed in MAURY *et al*., 2005). A further increase in amorphadiene production by *E coli* has been obtained by expressing the mevalonate pathway from *Saccharomyces cerevisiae* in an amorphadiene producing strain of *E. coli.* Some isoprenoid compounds have also been produced in yeasts, including epi-cedrol in *S. cerevisiae,* lycopene and beta-carotene in *S. cerevisiae* and *Candida utilis.* In several cases, isoprenoid production in yeast has been shown to be enhanced by overexpression of HMG1 (encoding HMG-COA reductase) (reviewed in MAURY *et al*., 2005).

In spite of the background depicted above, there is still a need for further processes for producing terpenes and terpenoids and, in particular, for ways of accumulating terpenes in microorganisms with higher yields and in a less costly and time intensive manner than in the prior known methods. A method for producing terpenes or terpenoids that fulfils this need is described herein.

A microorganism that accumulates and/or secretes high amounts of terpenes to the surrounding medium is disclosed. The production of terpenes by such a microorganism is preferably stable over time.

Furthermore, engineered plants with enhanced isoprenoid production with an aim to purify the isoprenoid component, as a means of increasing the nutritional value of food crops, or to enhance the fitness of the plant itself by increasing resistance to herbivores, pests or pathogens are also disclosed.

The present invention seeks to overcome these and other drawbacks inherent in the prior art by using one of these two genes to provide transgenic plants, bacteria or yeast that express one of these two genes, and are capable of a greatly enhanced accumulation of terpenes, in comparison to plants, bacteria or yeast expressing a typical *dxs* gene.

### BRIEF DESCRIPTION OF THE DRAWINGS

The drawings form part of the present specification and are included to further demonstrate certain aspects of the present invention. The invention may be better understood by reference to one or more of these drawings in combination with the detailed description of specific embodiments presented herein.

The Figure 1 shows the nucleotide sequence of cDNAs corresponding to different DXS alleles in *V. vinifera* cv Muscat Ottonel (MO) (MODXS1 , SEQ ID N°8, and MODXS2, SEQ ID N°9) and Gewurztraminer (GW) (GWDXS2, SEQ ID N°10). Positions corresponding to SNPs indicated in bold letters.

The Figure 2 shows the comparison of the proteins corresponding to the different DXS alleles in MO and GW with the associated consensus sequence corresponding to SEQ ID N°2.

The Figure 3 shows the consensus sequence (by which the sequence SEQ ID N°1 was obtained) resulting from the comparison of DXS proteins from different plant species:
First line: Consensus
2^{nd} line: Mo_DXS1
3^{rd} line: Mo_DXS2
4^{th} line: GW_DXS2
5^{th} line: Arabidopsis thaliana
6^{th} line: Capsicum annuum
7^{th} line: Catharanthus roseus
8^{th} line: Glycine max
9^{th} line: Narcissus pseudonarcissus
10^{th} line: Nicotiana tabacum
11^{th} line: Oryza sativa

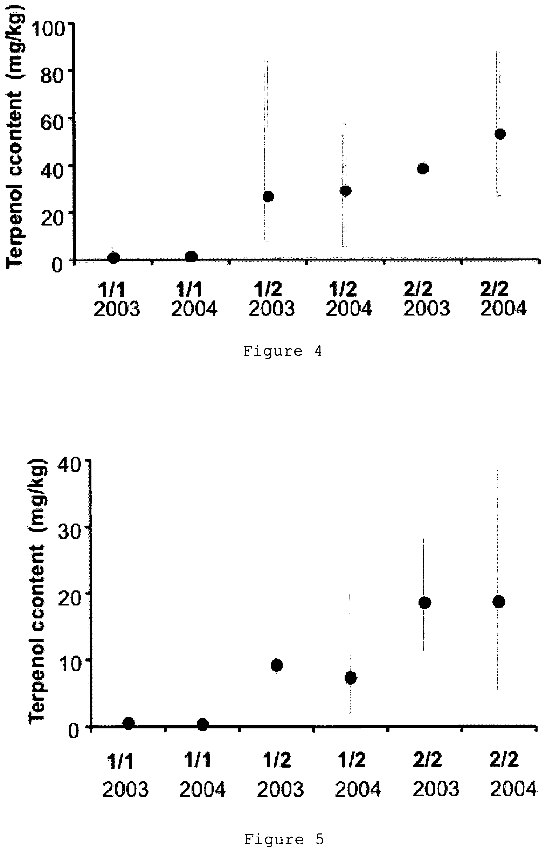

The Figure 4 shows the Terpenol content (pool of 5 major terpenols: geraniol, linalol, citronellol, nerol, alpha-terpineol) in grape berry skins and DXS genotype in a Muscat Ottonel progeny (years 2003 and 2004)(1: MoDXS1; 2 : MoDXS2).

The Figure 5 shows the Terpenol content (pool of 5 major terpenols: geraniol, linalol, citronellol, nerol, alpha-terpineol) in grape berry skins and DXS genotype in a Gewurztraminer progeny (years 2004 and 2005) (1: GwDXS1 = MoDXS1; and 2 : GwDXS2).

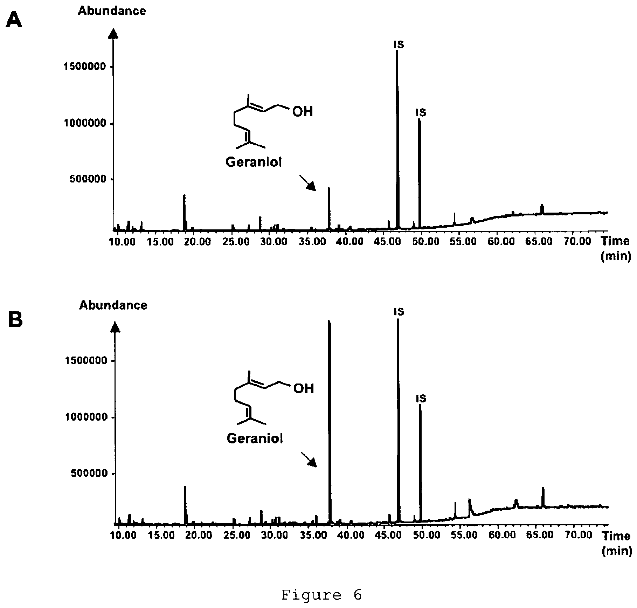

The Figure 6 shows GC-MS analysis of terpenes produced in tobacco leaves co-expressing DXS alleles from grapevine and geraniol synthase (GES) from basil. A: Analysis of tobacco leaves co-expressing MoDXS1 and GES. B: Analysis of tobacco leaves co-expressing MoDXS2 and GES

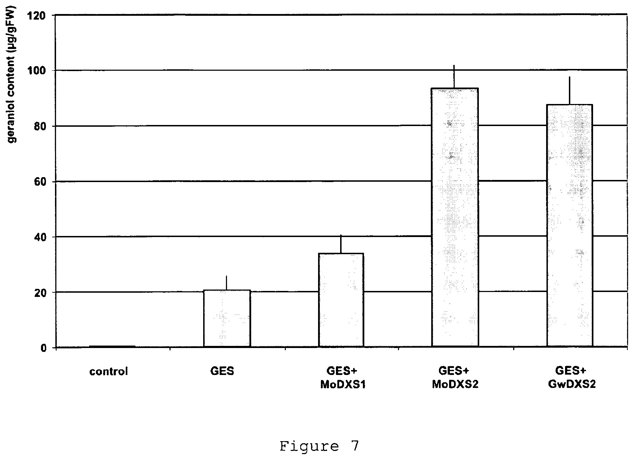

The Figure 7 shows the *In planta* geraniol biosynthesis, following transient co-expression of GES and DXS alleles in tobacco leaves. Untransformed tobacco leaves (control), leaves expressing GES alone and leaves co-expressing GES and DXS alleles were analysed using GC-MS, 4 days post *Agrobacterium-*mediated transformation. For each condition, geraniol amounts are means (± standard errors) of 9 independent experiments.

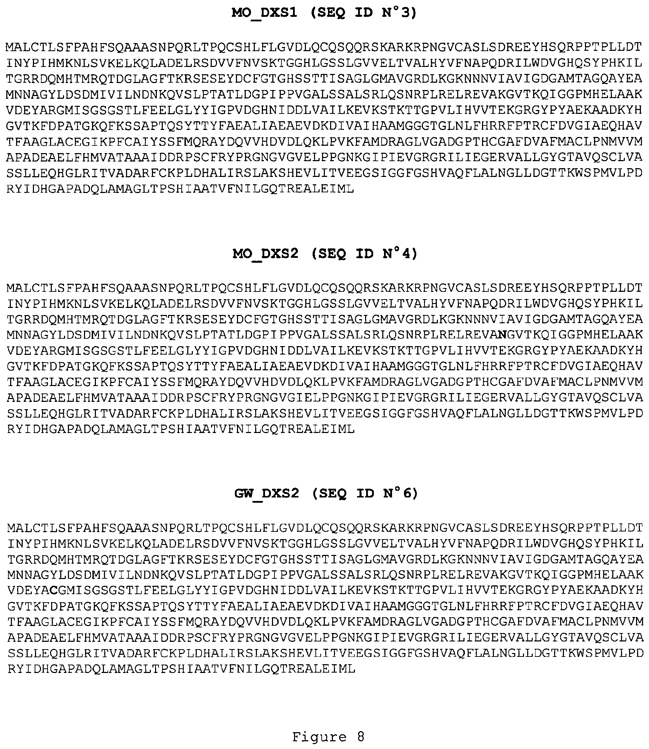

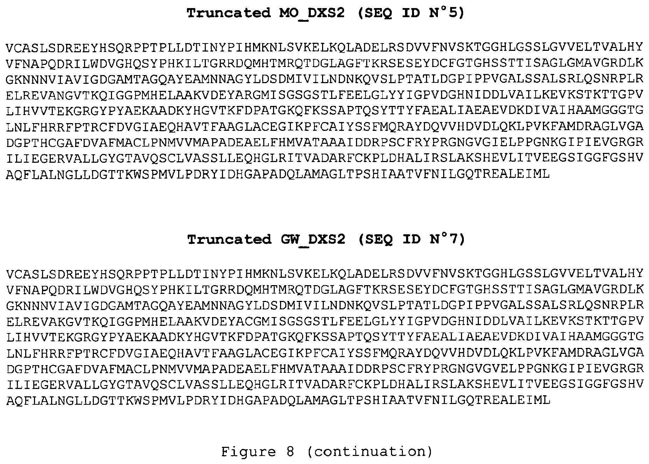

The Figure 8 shows the amino acid sequence of MODXS1 (SEQ ID N°3), MODXS2 (SEQ ID N°4), truncated MODXS2 with DXS activity (SEQ ID N°5) and GWDXS2 (SEQ ID N°6), truncated GWDXS2 with DXS activity (SEQ ID N°7).

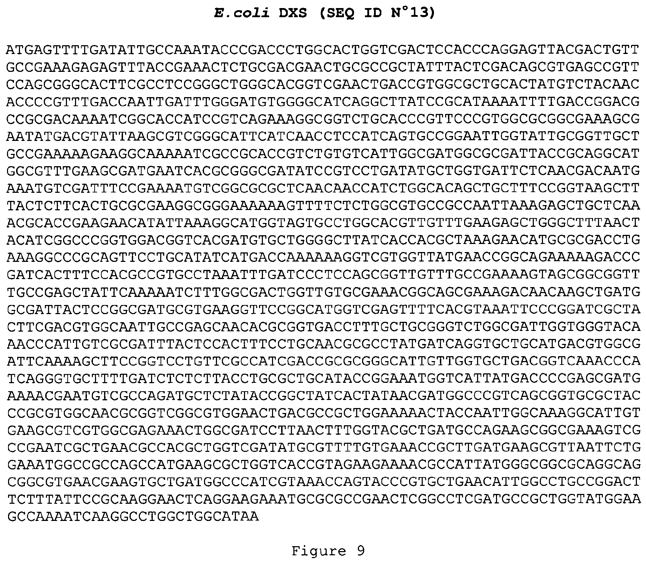

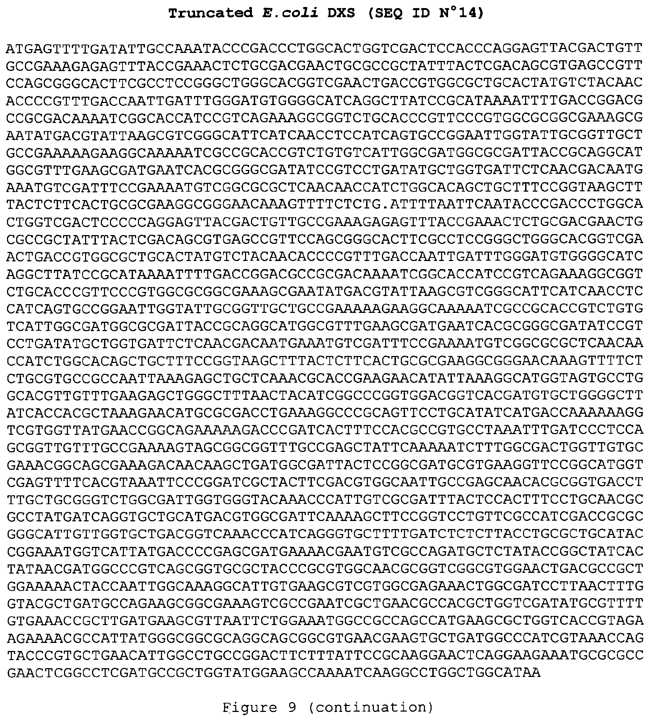

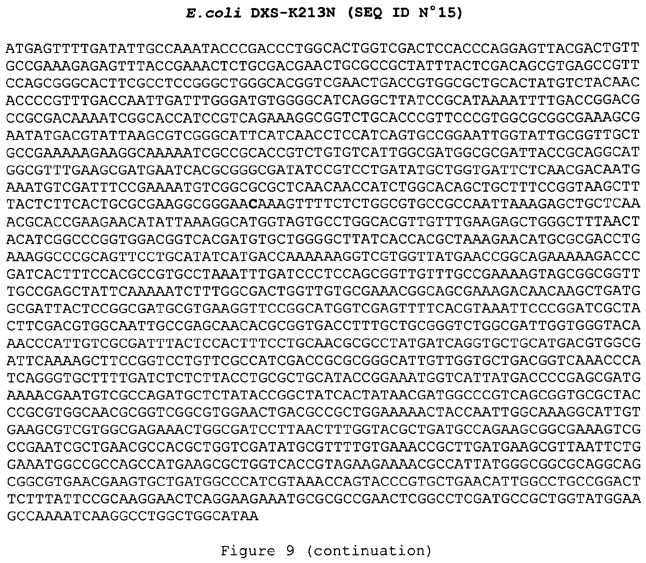

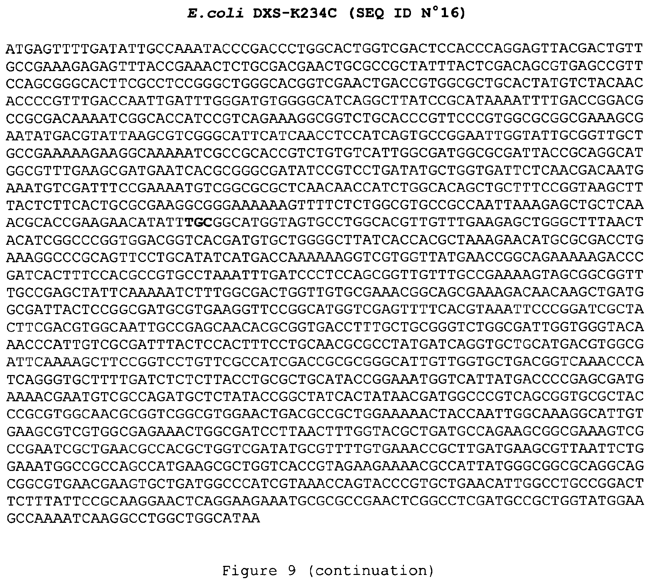

The Figure 9 shows the nucleotide sequence of the wild type DXS gene from E. coli (Ecoli DXS), the sequence of the gene encoding the truncated form of the DXS protein (truncated Ecoli DXS) and the sequence of the genes encoding the DXS protein harbouring the K213→N and the R306→C mutations (Ecoli DXS-K213N and Ecoli DXS-K234C, respectively).

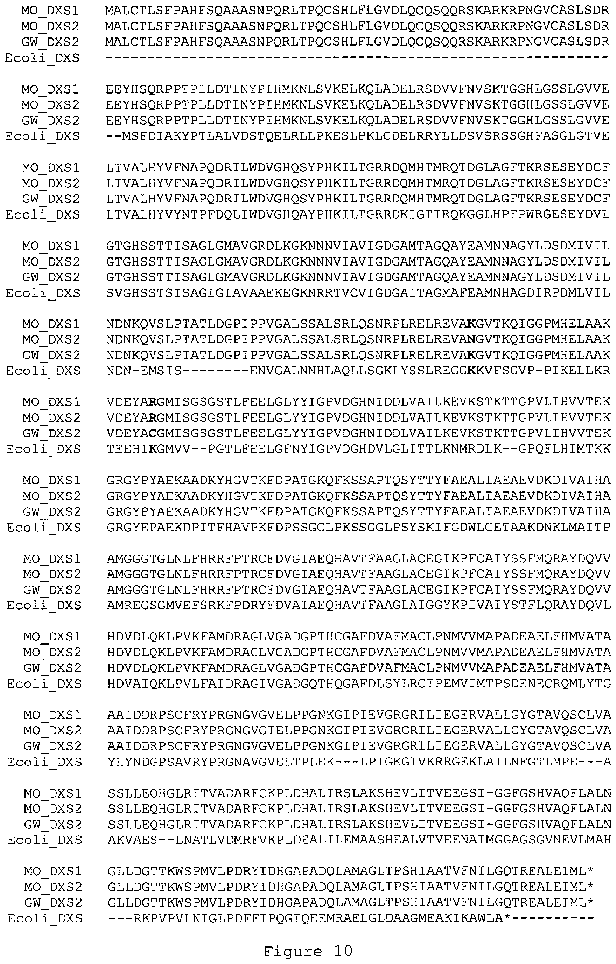

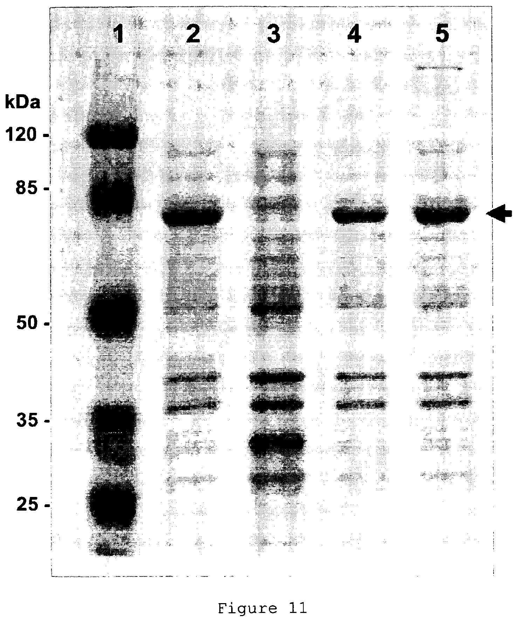

The Figure 10 shows the comparison of the proteins corresponding to the different DXS alleles in *V. vinifera* cv Muscat Ottonel (MO) (MODXS1 , SEQ ID N°8, and MODXS2, SEQ ID N°9) and Gewurztraminer (GW) (GWDXS2, SEQ ID N°10) with the DXS protein from Escherichia coli (Lois et al., 1998). The amino acids corresponding to the SNPs in MODXS2 and GWDXS2 and the corresponding amino acids in the DXS from *E coli* are indicated in bold letters. The Figure 11 shows a sodium dodecyl sulfate polyacrylamide gel electrophoresis (SDS-PAGE) analysis of recombinant DXS expressed in the *E. coli* strain BL21-Gold(DE3)pLysS.

### Lane 1: molecular weight marker.

Lane 2: BL21-Gold(DE3)pLysS transformed with the plasmid pHGWA-EcoliDXS.

Lane 3: BL21-Gold(DE3)pLysS transformed with the plasmid pHGWA-TruncatedEcoliDXS.

Lane 4: BL21-Gold(DE3)pLysS transformed with the plasmids pHGWA-EcoliDXS-K213N. Lane 5: BL21-Gold(DE3)pLysS transformed with the plasmids pHGWA-EcoliDXS-K234C. DXS expression was induced with 1 mM IPTG for 24 h at 28 °C.

The arrow indicates the position of the DXS protein and the molecular weight of the markers are indicated.

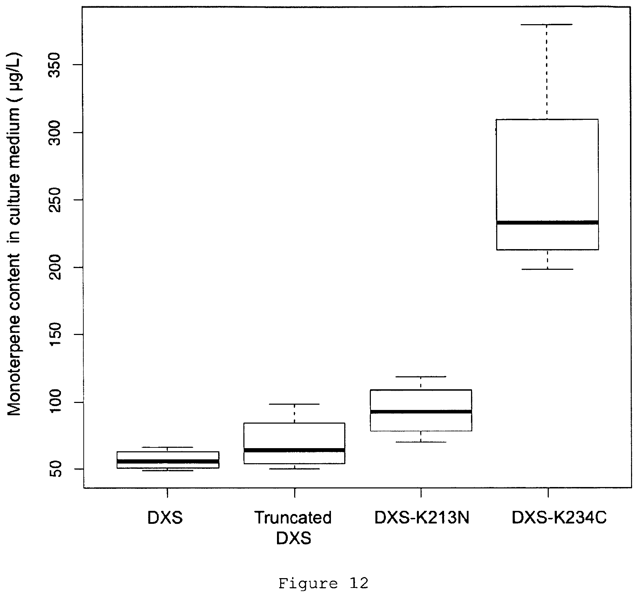

The Figure 12 shows a box plot presenting monoterpene contents in culture medium of *E*. *coli* transformed with different DXS constructs: wild type EcoliDXS, TruncatedEcoliDXS, EcoliDXS-K213N, EcoliDXS-K234C. Monoterpene contents represent the sum of the two major monoterpenes accumulated in *E. coli* cultures : geraniol and geranyl acetate. The data correspond to the results of 4 independent experiments. For each construct, the bold bar represents the median, the limits of the box are the 1^{st} and the 4^{th} quartiles and the bars out of the box indicate the extreme values.

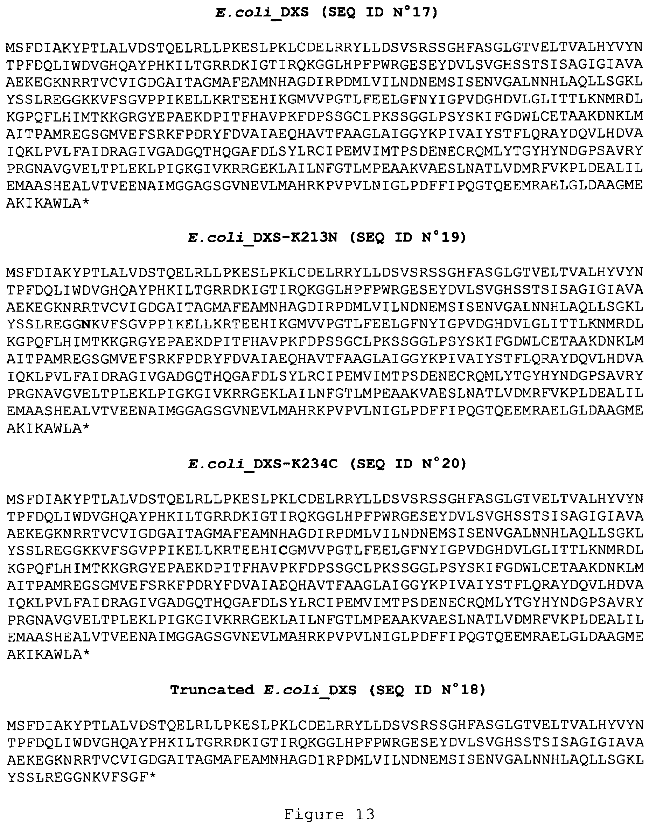

The figure 13 shows the amino acids sequence of E.coli DXS, *E.coli* DXS-K213N, *E.coli* DXS-K234C and Truncated *E.coli* DXS.

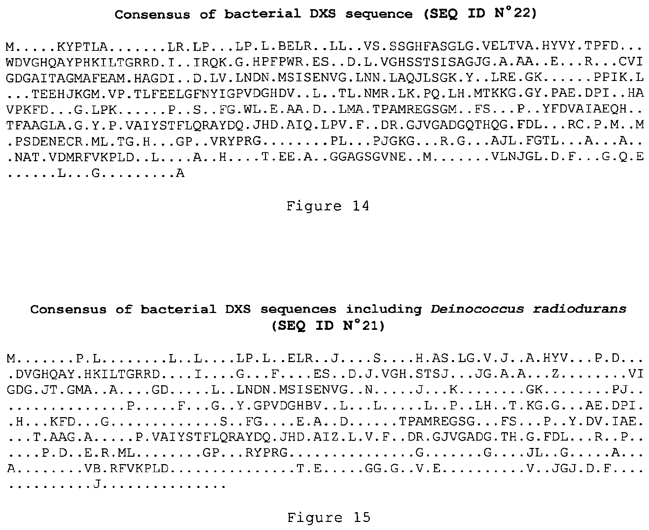

The figure 14 shows the consensus of bacterial DXS sequence (conserved amino acids are indicated, dots represent non-conserved amino acids). Amino acids corresponding to K213 and K234 in the DXS protein from E. coli are shaded.

The figure 15 shows the consensus of bacterial DXS sequences including Deinococcus radiodurans (conserved amino acids are indicated, dots represent non-conserved amino acids).

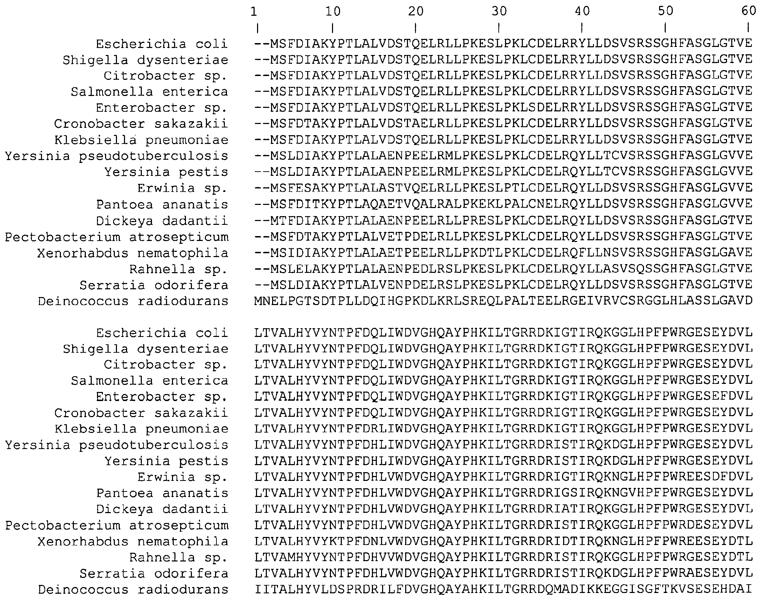

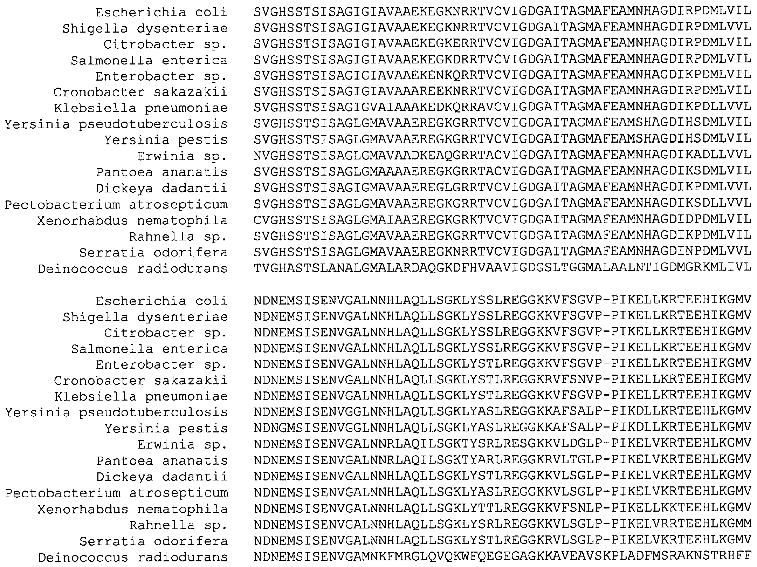

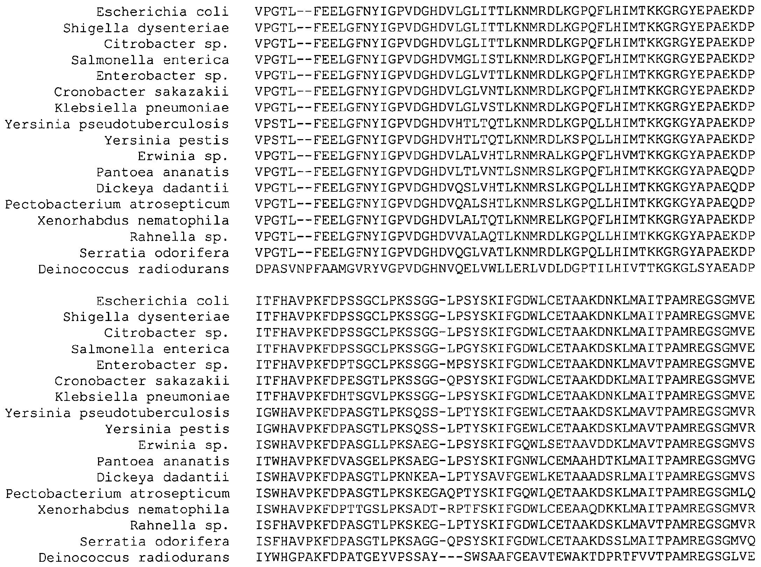

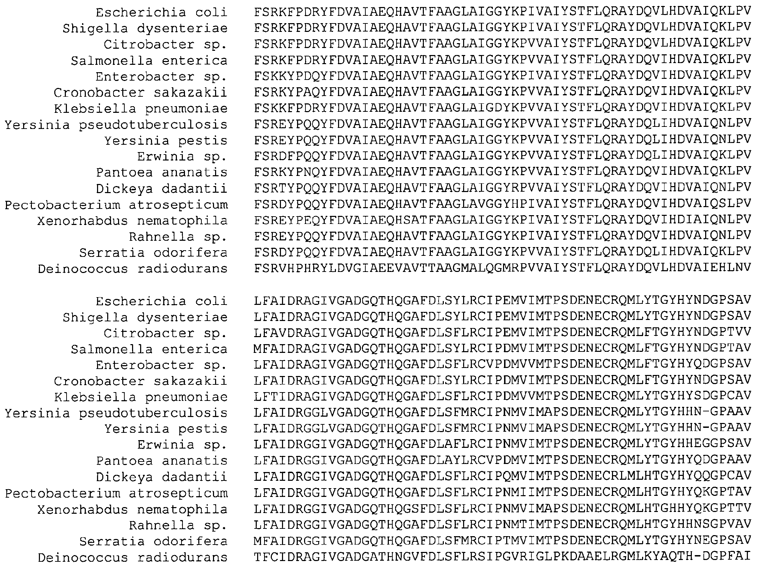

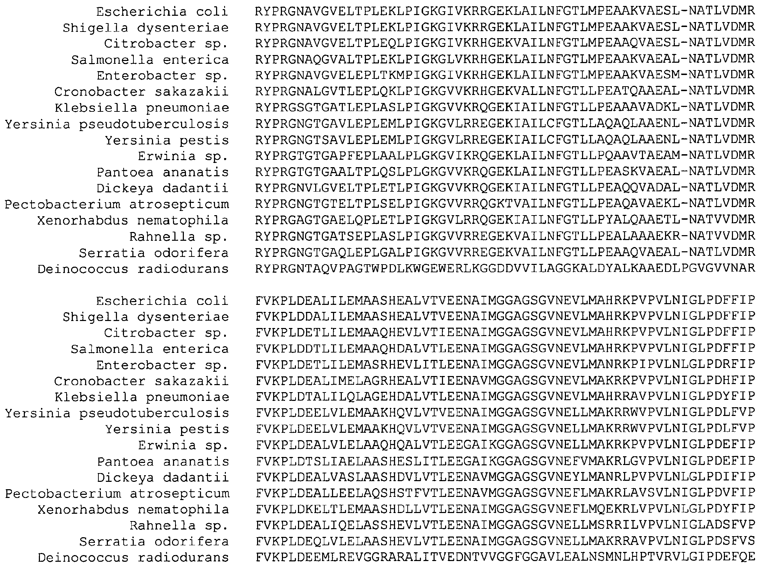

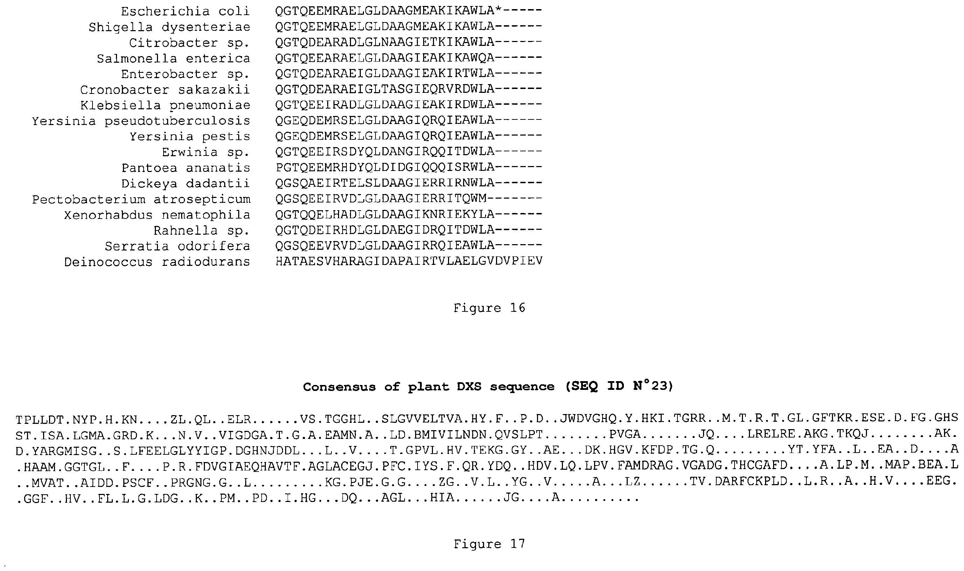

The figure 16 shows the amino acids sequence of DXS proteins from *Escherichia coli, Shigella dysenteriae, Citrobocter sp., Salmonella enterica, Enterobacter sp., Cronobacter sakazakii, Klebsiella pneumonia, Yersinia pseudotuberculosis, Yersinia pestis, Erwinia sp., Pantoea ananatis, Dickeya dadantii, Pectobacterium atrosepticum, Xenorhabdus nematophila, Rahnella sp., Serratia odorifera* and *Deinococcus radiodurans.*

The figure 17 shows the consensus of plant DXS sequence (conserved amino acids are indicated, dots represent non-conserved amino acids). Amino acids corresponding to K284 and R306 in the DXS protein from V. vinifern are shaded.

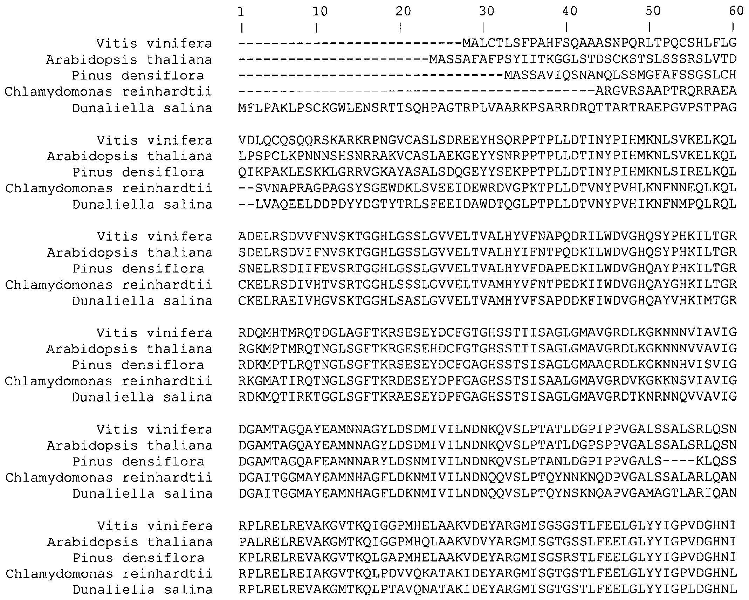

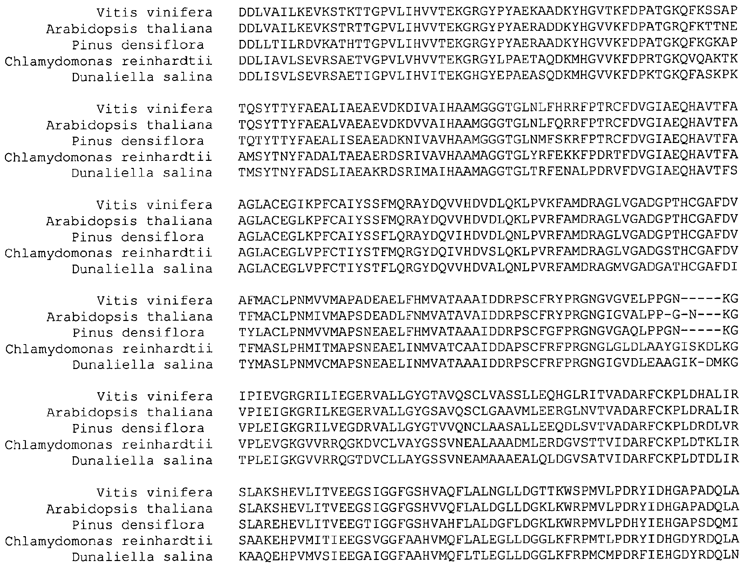

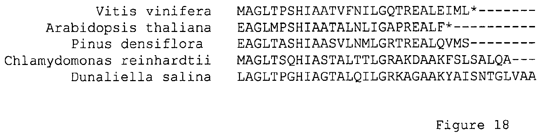

The figure 18 shows the amino acids sequence of DXS proteins from *Vitis vinifera, Arabidopsis thaliana, Pinus densiflora, Chlamydomonas reinhardtii* and *Dunaliella salina.*

### DETAILED DESCRIPTION OF THE INVENTION

A method of enhancement of the 1-deoxy-D-xylulose 5-phosphate synthase (DXS) activity of plants or bacteria to increase terpenes production is disclosed herein, said method comprising the steps of:
a) Determining at least one mutation site, preferably two, on the polypeptide having a DXS activity of a plant or a bacteria by doing a sequence comparison between a reference sequence selected in the group comprising SEQ ID N°2, SEQ ID N°4, SEQ ID N°6, SEQ ID N°19, SEQ ID N°20, SEQ ID N°21, SEQ ID N°22 and SEQ ID N°23 and the sequence of said plant or bacteria;
b) Proceeding to the mutation on the at least one mutation sites, preferably on two mutation sites;
c) Obtaining a polypeptide having an enhanced DXS activity compared to the typical DXS.

The step (a) of determination of mutation site(s) can be done by proceeding to a sequence comparison. The sequence of plant or bacteria to mute can be optimally aligned with a reference sequence selected from the group comprising SEQ ID N°2, SEQ ID N°4, SEQ ID N°6, SEQ ID N°19, SEQ ID N°20, SEQ ID N°21, SEQ ID N°22 and SEQ ID N°23.

Preferably, the reference sequence is selected from the group comprising SEQ ID N°4, SEQ ID N°6, SEQ ID N°19 and SEQ ID N°20.

More preferably, SEQ ID N°4 or SEQ ID N°6 are used as reference sequences for determining a mutation site on a plant polypeptide and SEQ ID N°19 or SEQ ID N°20 are used as reference sequences for determining a mutation site on a bacteria polypeptide.
As an example, the amino acids corresponding to K284 and R306 in the DXS from *Vitus vinifera* can be identified in a DXS protein from another organism using a multiple sequence alignment computer program, such as ClustalW (Thompson et al., 1994) or MUSCLE (Edgar, 2004).

As used herein, the terms "enhancement of DXS activity" and "enhanced DXS" refer to a modified DXS that permit to increase terpenes production and/or accumulation in a host cell, for example plant, bacteria or yeast, transformed by this modified DXS in comparison to a host cell expressing a typical DXS gene.

The expression "typical DXS gene" as used herein is the gene appearing at high frequency, for example the wild type of DXS MoDXS1, also known as SEQ ID N°8.

As used herein, the term "mutation" corresponds to any modification in the sequence of the original nucleic acid sequence. These mutations comprise small-scale mutations, or large scale mutations. Small scale mutations are those affecting a gene in one or a few nucleotides, including point mutations, insertions or deletions of one or more extra nucleotides in the DNA. Point mutations can be silent, missense and nonsense mutation. Large scale mutation in the genomic structure, such as gene duplications, deletions, or mutations whose effect is to juxtapose previously separate pieces of DNA, potentially bringing together separate genes to form functionally distinct fusion genes. These last mutations include chromosomal translocations, interstitial deletions, chromosomal inversions and loss of heterozygosity.

Preferably, the present mutation is a point mutation. Point mutations are individual substitutions, deletions or additions which alter, add or delete a single amino acid or a small percentage of amino acids (typically less than 5%, more typically less than 4%, 2% or 1%, or less).

As used herein, the term "mutation site" refers to the locus of the polynucleotide represented by one or several nucleotide that is subject to a mutation as described previously.

The methods for proceeding to said mutations are well known to the man of the art. For example, site-directed mutagenesis can be performed using the QuikChange kit (Stratagene, La Jolla, CA). Site-directed mutagenesis methods have also been described by Zoller & Smith (1982) "Oligonucleotide-directed mutagenesis using M13-derived vectors: an efficient and general procedure for the production of point mutations in any DNA fragment" Nucleic Acids Res. 10:6487-6500.

An object of the invention relates to the use of a mutated isolated polypeptide having a 1-deoxy-D-xylulose 5-phosphate synthase (DXS) activity consisting of converting glyceraldehyde-3-phosphate and pyruvate into 1-deoxyxylulose-5-phosphate, derived from the 1-deoxy-D-xylulose 5-phosphate synthase (DXS) corresponding to the consensus sequence SEQ ID N°21, said mutated isolated polypeptide comprising one or two of the following two mutations:
- substitution of the amino acid in position 213 of SEQ ID N°21 by an asparagine,
- substitution of the amino acid in position 234 of SEQ ID N°21 by a cysteine,

and the sequence of the said mutated isolated polypeptide having at least 90% identity with the sequence SEQ ID N°21,
to increase terpenes production and/or accumulation in a host cell transformed by the said mutated isolated polypeptide having a DXS activity.

As used herein, the expression "increase terpenes production" means that the amount of terpene produced and/or accumulated in a host cell, for example plant, bacteria or yeast, transformed by this modified DXS is higher than the amount of terpene produced and/or accumulated in a host cell expressing the typical DXS gene.

Preferably, terpene production resulting from the use of a mutated isolated polypeptide having a DXS activity according to the present invention is increased by a factor ten.

As an example, the mutation K234→C introduced in the polynucleotide encoding the DXS enzyme of *E.coli* permits to increase the terpene production by at least a factor ten.
Also provided herein is a polypeptide of plant or bacteria, having an enhanced DXS activity compared to the typical DXS of said plant or bacteria.
Also provided herein is a mutated isolated polypeptide having a DXS activity, having a sequence having at least 90 % identity with the sequence SEQ ID N°21, wherein the amino acid at position 213 of SEQ ID N°21 is substituted by an asparagine and/or the amino acid at position 234 of SEQ ID N°21 is substituted by a cysteine.

SEQ ID N°21 is a consensus of bacterial DXS sequences including *Deinococcus radiodurans,* which has often been characterized in the literature.

Also provided herein is the isolated polypeptide having the sequence SEQ ID N°22 or fragments thereof, wherein the amino acid at position 213 of SEQ ID N°22 is an asparagine and/or the amino acid at position 234 of SEQ ID N°22 is a cysteine.

SEQ ID N°22 is a consensus of bacterial DXS sequences characterized in that it is similar to SEQ ID N°21 except that it doesn't encompass the DXS sequence of *D. radiodurans*

In a more preferred embodiment, the mutated isolated polypeptide used to increase terpenes production and/or accumulation in a host cell according to the invention has the sequence SEQ ID N° 19.

In a more preferred embodiment, the mutated isolated polypeptide used to increase terpenes production and/or accumulation in a host cell according to the invention has the sequence SEQ ID N° 20.

The present disclosure relates to an isolated polypeptide having a deoxy-D-xylulose synthase (DXS) activity, which comprises a sequence having at least 90 % identity with the sequence SEQ ID N° 1 or fragments thereof, wherein the amino acid at position 310 of SEQ ID N°1 is a cysteine and/or the amino acid at position 288 of SEQ ID N°1 is an asparagine.

As used herein, the terms "amino acid" and "amino acids" refer to all naturally occurring L-a-amino acids or their residues. The amino acids are identified by either the single-letter or three-letter designations (Asp D aspartic acid; Ile I isoleucine; Thr T threonine; Leu L leucine ; Ser S serine ; Tyr Y tyrosine; Glu E glutamic acid; Phe F phenylalanine; Pro P proline; His H histidine; Gly G glycine; Lys K lysine; Ala A alanine; Arg R arginine; Cys C cysteine; Trp W tryptophan; Val V valine; Gln Q glutamine; Met M methionine; Asn N asparagine).

It will also be understood that natural amino acids may be also replaced by chemically modified amino acids. Typically, such chemically modified amino acids enable to increase the polypeptide half life.

The term "1-deoxy-D-xylulose 5-phosphate synthase" (abbreviated as "DXS") is used herein to mean an enzyme capable of catalyzing a transketolase-type condensation involving pyruvate and glyceraldehyde-3-phosphate (GAP) to form 1-deoxy-D-xylulose-5-phosphate. Said enzymatic activity can be simply determined using a radiolabelled substrate as described by Lois et al. (1998). The enzyme reaction mixture consists of 50mM Tris-HCl pH=7.5, 2.5 mM MgCl2, 1 mM thiamin diphosphate, 5 mM 2-mercaptoethanol, 0.2 mM [2-14C]pyruvate (15.9 mCi / mmol, NEN), 50 mM pyruvate, 50 mM DL-glyceraldehyde 3-phosphate in a final volume of 50 *µ*L. After incubation for 2 h at 37°C, the reactions are stopped by heating at 80°C for 3 min. After centrifugation at 13,000 g for 5 min, 10 *µ*L of the supernatant (2-5 ml) are loaded onto a TLC plate (silica gel 60, Merck). Labeled D-1-deoxyxylulose 5-phosphate is separated from [2-14C]pyruvate by using n-propyl alcohol /ethyl acetate / H2O (6:1:3) as solvent and quantified by autoradiography or scintillation counting. Alternatively, a fluorimetric assay of 1-deoxy-D-xylulose 5-phosphate synthase can be used, as described by Querol et al. (2001).

Preferably, the isolated polypeptide disclosed herein comprises a sequence having at least 90% amino acids sequence identity with the polypeptide sequence SEQ ID N°2 or fragments thereof, wherein the amino acid at position 306 of SEQ ID N°2 is a cysteine and/or the amino acid at position 284 of SEQ ID N°2 is an asparagine.

Still preferably, the polypeptide disclosed herein has an enzymatic activity, which is at least 50% superior as compared to the polypeptide SEQ ID N°3, preferably at least 70%, and most preferably at least 100% superior as compared to SEQ ID N°3.

In a more preferred embodiment, the polypeptide of the disclosure comprises the sequence SEQ ID N° 4.

In a more preferred embodiment, the polypeptide of the disclosure comprises the sequence SEQ ID N° 6.

As used herein, "percentage of identity" between two amino acids sequences, means the percentage of identical amino-acids, between the two sequences to be compared, obtained with the best alignment of said sequences, this percentage being purely statistical and the differences between these two sequences being randomly spread over the amino acids sequences. As used herein, "best alignment" or "optimal alignment", means the alignment for which the determined percentage of identity (see below) is the highest. Sequences comparison between two amino acids sequences are usually realized by comparing these sequences that have been previously aligned according to the best alignment; this comparison is realized on segments of comparison in order to identify and compare the local regions of similarity. The best sequences alignment to perform comparison can be realized, beside by a manual way, by using the global homology algorithm developed by SMITH and WATERMAN (Ad. App. Math., vol.2, p:482, 1981), by using the local homology algorithm developed by NEDDLEMAN and WUNSCH (J. Mol. Biol., vol.48, p:443, 1970), by using the method of similarities developed by PEARSON and LIPMAN (Proc. Natl. Acd. Sci. USA, vol.85, p:2444, 1988), by using computer softwares using such algorithms (GAP, BESTFIT, BLAST P, BLAST N, FASTA, TFASTA in the Wisconsin Genetics software Package, Genetics Computer Group, 575 Science Dr., Madison, WI USA), by using the MUSCLE multiple alignment algorithms (Edgar, Robert C., Nucleic Acids Research, vol. 32, p:1792, 2004). To get the best local alignment, one can preferably use BLAST software, with the BLOSUM 62 matrix, or the PAM 30 matrix. The identity percentage between two sequences of amino acids is determined by comparing these two sequences optimally aligned, the amino acids sequences being able to comprise additions or deletions in respect to the reference sequence in order to get the optimal alignment between these two sequences. The percentage of identity is calculated by determining the number of identical position between these two sequences, by dividing this number by the total number of compared positions, and by multiplying the result obtained by 100 to get the percentage of identity between these two sequences.

According to a preferred embodiment, the polypeptide of the disclosure has an identity of at least 95%, preferably of at least 99%, and most preferably of 100% with the amino acids sequences SEQ ID N°1 or SEQ ID N°2.

As used herein, a fragment of SEQ ID N°1 or SEQ ID N°2 refers to a polypeptide having a length of at least 300 amino acids, preferably having a length of at least 400 amino acids, more preferably having a length of at least 500 amino acids.

As an example, a fragment of SEQ ID N°1 or SEQ ID N°2 with a DXS activity, one can cite SEQ ID N°5 corresponding to truncated MODXS2 polypeptide, or SEQ ID N°7 corresponding to truncated GWDXS2 polypeptide. As an example, a consensus of plant DXS sequence is SEQ ID N°23. It comprises the sequence of DXS of *V. vinifera* (Mo_DXS1), and the sequence of DXS of *A. thaliana, P. densiflora, C. reinhardtii* and *D. salina.*

The disclosure relates to an isolated polynucleotide encoding for a polypeptide disclosed previously.

The polynucleotide may be in the form of RNA or in the form of DNA, preferably in the form of DNA.

The DNA may be double-stranded or single-stranded.

According to a preferred embodiment, the polynucleotide comprises a sequence selected in the group comprising SEQ ID N° 9 (MODXS2), SEQ ID N° 10 (GWDXS2), SEQ ID N° 15 (E.coli DXS-K213N) and SEQ ID N°16 (E.coli DXS-K234C).

The disclosure relates to a vector comprising the polynucleotide disclosed previously.

Said vector can be a cloning or an expression vector, preferably an expression vector, and may be for example in the form of a plasmid, a cosmid, a phagemid, a viral particle, a phage, etc.

Such vectors may include bacterial plasmids, phage DNA, baculovirus, yeast plasmids, vectors derived from combinations of plasmids and phage DNA, viral DNA such as vaccinia, adenovirus, fowl pox virus, and pseudorabies. Large numbers of suitable vectors are known to those of skill in the art and are commercially available. The following vectors are provided by way of example. Bacterial: pQE70, pQE60, pQE-9 (QIAGEN), pbs, pD10, phagescript, psiX174, pbluescript SK, pbsks, pNH8A, pNH16a, pNH18A, pNH46A (STRATAGENE), ptrc99a, pKK223-3, pKK233-3, pDR540, pRIT5 (PHARMACIA). Eukaryotic: pWLNEO, pSV2CAT, pOG44, pXT1, pSG (STRATAGENE), pSVK3, pBPV, pMSG, pSVL (PHARMACIA). However, any other vector may be used as long as it is replicable and viable in the host.

The polynucleotide sequence, preferably the DNA sequence in the expression vector is operatively linked to an appropriate expression control sequence(s) (promoter) to direct mRNA synthesis. As representative examples of such promoters, one can mention prokaryotic or eukaryotic promoters such as CMV immediate early, HSV thymidine kinase, early and late SV40, LTRs from retrovirus, and mouse metallothionein-I. The expression vector also contains a ribosome binding site for translation initiation and a transcription vector. The vector may also include appropriate sequences for amplifying expression.

In addition, the expression vectors preferably contain one or more selectable marker genes to provide a phenotypic trait for selection of transformed host cells such as dihydrofolate reductase or neomycin resistance for eukaryotic cell culture, or such as tetracycline or ampicillin resistance in *E*. *coli.*

The vector of the disclosure containing the appropriate polynucleotide sequence as herein above described, as well as an appropriate promoter or control sequence, may be employed to transform an appropriate host to permit the host to express the polypeptide.

As an example, transcription of a DNA encoding for the polypeptide described previously by higher eukaryotes can be increased by inserting an enhancer sequence into the vector. Enhancers are cis-acting elements of DNA, usually about from 10 to 300 pb that act on a promoter to increase its transcription. Examples of enhancer include the SV40 enhancer, the CMV early promoter enhancer, and adenovirus enhancers.

The disclosure relates to a transformed host cell comprising the polynucleotide or the vector described previously.

The terms "transformed host cell," "transformed" and "transformation" refer to the introduction of DNA into a cell. The cell is termed a "host cell", and it may be a prokaryotic or a eukaryotic cell. Typical prokaryotic host cells include various strains of *E. coli.* Typical eukaryotic host cells are plant cells, such as maize cells, yeast cells, insect cells or animal cells. The selection of an appropriate host is deemed to be within the scope of those skilled in the art from the teachings herein.

The transformed host cell used according to the present invention is a prokaryotic or an eukaryotic cell. The introduction of the polynucleotide or vector described previously into the host cell can be effected by method well known from one of skill in the art such as calcium phosphate transfection, DEAE-Dextran mediated transfection, or electroporation.

According to a preferred embodiment, the transformed host cell used according to the present invention is a prokaryotic cell selected in the group comprising eubacterial, archaebacterial and cyano-bacterial cells.

More preferably, the transformed host cell used according to the present invention is a prokaryotic cell and even more preferably is an *Escherichia coli* cell.

Prokaryotes may also be used as host cells for the initial cloning steps of this invention. They are particularly useful for rapid production of large amounts of DNA, for production of single-stranded DNA templates used for site-directed mutagenesis, for screening many mutants simultaneously, and for DNA sequencing of the mutants generated. Suitable prokaryotic host cells include *E. coli* K12 strain 94 (ATCC No. 31,446), *E*. *coli* strain W3110 (ATCC No. 27,325) *E*. *coli* X1776 (ATCC No. 31,537), and *E. coli* B; however many other strains of *E. coli,* such as HB101, JM101, NM522, NM538, NM539, and many other species and genera of prokaryotes including bacilli such as *Bacillus subtilis,* other enterobacteriaceae such as *Salmonella typhimurium* or *Serratia marcesans,* and various *Pseudomonas* species may all be used as hosts.

According to another preferred embodiment, the transformed host cell used according to the present invention is a eukaryotic cell selected in the group comprising animal, fungal, yeast, and plant cells.

Preferably, the eukaryotic cell is a fungal microorganism, more particularly a yeast.

A non-exhaustive list of suitable microorganisms will include the following: a species belonging to the genera *Saccharomyces* -e.g. *S. cerevisiae, S. bayanus, S. pastorianus, S. paradoxus, S. exiguous, S. servazzi, S. uvarum, S. kluyveri;* and *S. castellii-, a* species belonging to the genera *Kluyveromyces -e.g. K. lactis*, *K marxianus var. marxianus, K. thermotolorens, K. waltii, K. delphensis, K. nonfermentas, and K. wickerhamii*- a species belonging to the genera *Candida* -e.g. *C. utills, C. tropicalis, C. castellii, and C. humilis*-*,* a species belonging to the genera *Zygosaccharomyces* -e.g. Z. *rouxii, Z. bailii, Z. fermentati, Z. bisporus,* and *Z. florentinus*-*,* a species belonging to the genera *Pichia*-e.g. *P. stipidis, P. pastoris, P. sorbithophila,* and *P. anomala*-*,* or other species -e.g. *Hansenula polymorpha, yarrowia lipolytica, Debaromyces hansenii, Schizosaccharomyces pombe, Torulaspora delbueckii, Ashbya gossipie, Aspergillus niger, Aspergillus awamori, Aspergillus oryzae, Aspergillus nidulans, Penecillium chrysogenum, Rhizopus oryzae,* and *Mucor circenelloides*-*.*

More preferably, the eukaryotic cell is yeast and even more preferably, the microorganism is *Saccharomyces cerevisiae.* For example, it is the *S. cerevisiae* strain deposited at DSMZ-Deutsche Sammlung von Mikroorganismen und Zellkulturen GmbH. Mascheroder Weg 1b, D-38124 Braunschweig, Germany, accession number: DSMZ 17900, on Jan. 27, 2006.

A heterologous pathway, for the purpose of the present invention, is a pathway which, in its activity to form a compound by defined intermediate steps and from defined starting materials, is not present in the wild-type of the microorganism. More preferably, the heterologous pathway is a pathway derived from DNA of a different species.

Alternatively, the eukaryotic cell used according to the invention is a plant cell, more preferably a plant cell selected in the group comprising *Vitis vinifera, Nicotiana tabacum,* and *Arabidopsis thaliana* cells, even more preferably said plant cell is a *Vitis vinifera* cell, preferably a *Vitis vinifera* Cv Muscat Ottonel or a *Vitis vinifera* Cv Gewurztraminer cell, more preferably a *Vitis vinifera* Cv Gewurztraminer cell.

The disclosure relates to a transgenic bacterium comprising the polynucleotide or the vector described previously.

The disclosure relates to a transgenic plant comprising the polynucleotide, the vector or the host cell described previously.

Transformation of cultured plant host cells is normally accomplished through *Agrobacterium tumifaciens.* Thus, transgenic plants can be obtained, for example, by transferring vector of the present invention (i.e. encoding 1-deoxyxylulose-5-phosphate synthase) and a selectable marker gene (e.g., the kan gene encoding resistance to kanamycin) into *Agrobacterium tumifaciens* containing a helper Ti plasmid as described in HOECKEMA et al. (Nature, 303:179-181, 1983) and culturing the Agrobacterium cells with leaf slices, or other tissues or cells, of the plant to be transformed as described by AN et al. (Plant Physiology, 81:301-305, 1986). However, other methods for introducing DNA into cells can be used such as Polybrene (Kawai and Nishizawa, Mol. Cell. Biol., 4:1172 [1984]), protoplast fusion (Schaffner, Proc. Natal. Acad. Sci. USA, 77:2163 [1980]), electroporation (Neumannet al., EMBOJ., 1:841 [1982]), and direct microinjection into nuclei (Capecchi, Cell, 22:479 [1980]).

Transformed plant calli may be selected through the selectable marker by growing the cells on a medium containing, e.g., kanamycin, and appropriate amounts of phytohormone such as naphthalene acetic acid and benzyladenine for callus and shoot induction. The plant cells may then be regenerated and the resulting plants transferred to soil using techniques well known to those skilled in the art.

The transgenic plant of the disclosure has incorporated into its genome a polynucleotide or a vector as described previously.

Preferably said plant is selected in the group comprising trees, vegetables, succulents and ornamental plants.

Still preferably, said transgenic plant is resistant to clomazone also called dimethazone (FMC 57020; 2-(2-chlorophenyl)methyl-4,4-dimethyl-3-isoxalidinone).

As used herein a transgenic plant which is resistant to clomazone corresponds to a plant which grows in the presence of 10 µM clomazone (Carretero-Paulet L, Cairó A, Botella-Pavía P, Besumbes O, Campos N, Boronat A, Rodríguez-Concepción M. (2006) Enhanced flux through the methylerythritol 4-phosphate pathway in Arabidopsis plants overexpressing deoxyxylulose 5-phosphate reductoisomerase. Plant Mol Biol 62: 683-95).

Still preferably, said transgenic plant has enhanced aromas.

As an example, said transgenic plant is selected in the group comprising Lamiaceae (e.g Ocimum (basil), Lavandula (Lavender), Origanum (oregano), Mentha (mint), Salvia (sage), Rosmecinus (rosemary), Thymus (thyme), Satureja and Monarda); Umbelliferae (e.g. Carum (caraway), Anethum (dill), feniculum (fennel) and Daucus (carrot)); Asteraceae (Compositae) (e.g. Artemisia (tarragon, sage brush), and Tanacetum (tansy)); Rutaceae (e.g., citrus plants); Rosaceae (e.g., roses); Myrtaceae (e.g., eucalyptus, Melaleuca); the Gramineae (e.g., Cymbopogon (citronella)); Geranaceae (Geranium) and certain conifers including Abies (e.g., Canadian balsam), Cedrus (cedar) and Thuja and Juniperus.

The disclosure relates to a progeny of any generation of the transgenic plant, said progeny comprising the polypeptide -, the polynucleotide -, the vector -, or the host cell described previously.

Said progeny can have the form of a plant or of a seed.

The disclosure relates to a method of preparing a transgenic plant as described previously, comprising the steps of:
a. transforming a plant cell with a vector as described previously;
b. selecting a transformed plant cell which express a polypeptide as described previously; and
c. generating a transgenic plant from said transformed plant cell.

The step (a) of transformation can be done by well known methods as described previously such as electroporation, transfection, naked DNA uptake, protoplast generation, direct transfer of DNA into pollen, embryo or pluripotent plant cell, Agro- bacterium-mediated transformation, particle bombardment, or microprojectile bombardment.

The step (b) of selection can be done by well known methods.

The step (c) of generating a clomazone-resistant transgenic plant from said transformed plant cell can be done by well known methods like the generation of pluripotent plant cells from said transformed plant cell.

According to a preferred embodiment, said method is for preparing a plant seed, said method further comprises the step of:
d. obtaining a seed from said transgenic plant.

According to a particular embodiment, the method is for obtaining a transgenic plant which is resistant to clomazone.

Preferably, said transgenic plant is obtained by well known methods such as those described in Klee H, Horsch R, Rogers S (1987) Agrobacterium-Mediated Plant Transformation and its Further Applications to Plant Biology. Annual Review of Plant Physiology 38, 467-486 and Potrykus I (1991) Gene Transfer to Plants: Assessment of Published Approaches and Results. Annual Review of Plant Physiology and Plant Molecular Biology 42, 205-225.

According to another particular embodiment, the method is for obtaining a transgenic plant which has enhanced terpene biosynthetic capabilities. A plant with enhanced terpene biosynthetic capabilities will synthesis a least 50% more of terpenes as compared to the wild type of said transgenic plant, preferably at least 70%, more preferably at least 100%.

Preferably, terpene relates to geraniol, linalol, citronellol, nerol and alpha-terpineol.

Preferably, the polypeptide disclosed previously has an enzymatic activity, which is at least 50% superior as compared to the polypeptide SEQ ID N°3, preferably at least 70%, and most preferably at least 100% superior as compared to SEQ ID N°3.

Said plant having terpene biosynthetic capabilities may be aromatic plants as for example *Mentha piperita, Lavandula latifolia,* or *Rosmarinus officinalis* and medicinal plants, as for example *Artemisia annua, Taxus baccata*, *Cathoranthus roseus,* or *Lithospermum erythrorhizon* as well as any plant species producing valuable terpene compounds, as for example plants producing carotenoids, as *Lycopersicon esculentum, Manihot esculenta,* or *Oryza sativa.*

Preferably, said transgenic plant is selected by well known methods such as those described in Klee et al. (1987) and Potrykus (1991).

The disclosure relates to a method of production of enhanced DXS enzyme which increases terpenes production in plants, bacteria or yeast comprising the following steps:
a. Culturing a transformed host cell as defined previously;
b. Obtaining an enhanced DXS enzyme which permit to increase terpenes production in plants or bacteria.

Preferably, the polypeptide having an enhanced DXS activity is likely to be obtained by the method described previously.

The disclosure relates to a method of production of terpenes in a host cell comprising the steps of:
a. Culturing a transformed host cell as defined previously under conditions effective to produce the terpene. (e.g., plant, bacteria or yeast cell) as described previously under conditions effective to produce more terpene substrate (monoterpene, diterpene, triterpene, and/or polyterpene) by the DXS2 enzyme, said production of more terpene substrate resulting in production of more terpene by enzymatic modification of the terpene substrate.
b. Obtaining said terpene from said host cell

A terpene is a saturated or unsaturated, optionally substituted hydrocarbon based on, or composed essentially of, isoprene units (C₅). Terpenes may be acyclic or cyclic. Terpenes, as used herein include terpenes, terpene derivatives, and compounds referred to as terpenoids, which may or may not fall in one of the two foregoing classes of compounds. Terpene derivatives include compounds that have undergone one or more steps of functionalization such as hydroxylations, isomerizations, oxido-reductions or acylations, for example. Preferably, for the purpose of the present invention, a terpene is a compound which fulfils the above condition and/or whose carbon skeleton originates, at least in part but preferably totally, from the MEP and/or MEV pathway. Accordingly, terpenes include terpene alcohols, for example C₁₅H₂₆O and C₁₀H₁₈O compounds such as patchoulol, epi-cedrol, cubebol, linalool, nerolidol, for example, and dialcohols, for example sclareol. Terpenes also include diphosphate compounds such as bornyl-diphosphate (monoterpene) and copalyl-diphosphate (diterpene), just to mention a few specimens of the vast category of terpenes.

As used herein, a "derivative" is any compound obtained from a known or putative compound and containing essential elements of the parent substance.

For example, terpenes are compounds having carbon skeletons of C₁₀, C₁₅, C₂₀, C₃₀, C₄₀, C₄₅ and so forth.

Accordingly, the term "terpene" encompasses mono, sesqui, di, tri, tetra and/or polyterpenes.

Preferably, said terpenes comprise geraniol, linalol, citronellol, nerol and alpha-terpineol.

According to a preferred embodiment, the transformed microorganism used according to the invention provides a yield of at least 30 µg, more preferably at least 100 µg of terpene per g fresh-weight of the microorganism. The terpene yield of a microorganism is preferably established by the protocol in Example 2.

The terpenes may accumulate in the cells. For example, they may accumulate in the cytoplasm, in mitochondria, cellular membranes or other cell organelles. Many terpenes are lipophilic compounds, which accumulate in the plasma membrane of the cell.

For the purpose of the present invention, the term "accumulating in the medium" also encompasses accumulation in a solvent during a two-phase fermentation process.

The method comprises the step of cultivating the transformed host cell under conditions conducive to the production of said terpene. These conditions are known to the skilled person. Generally, they may be adjusted by selection of an adequate medium, fermentation vessel, temperature, and pH.

The method for producing a terpene may comprise the step of isolating the terpene from the medium, from the cells and/or from an organic solvent, in case a two-phase fermentation is performed. The terpene may be isolated by any method used in the art including but not limited to chromatography, extraction and distillation, for example.

It is well known to the man skilled in the art that, for example, monoterpenes are normally produced in the plastid, and that sesquiterpenes in the cytosol.

In contrast to plants, microbial systems are more amenable to large-sale engineering projects where many different modifications may be compared and combined. However, microbes do suffer some technical drawbacks compared with plants, particularly when it comes to expressing heterologous genes.

The disclosure relates to a method of selecting an aromatic plant comprising the steps of:
a. Identifying at least one mutation of DXS that lead to an allelic variation of DXS with enhanced synthesis of terpene and/or analysis of the accumulation of terpene in the biological sample of said plant
b. Selecting the plant comprising said mutation.

The disclosure relates to a method of producing a DXS enzyme which are more resistant to clomazone comprising the steps of:
a. Culturing a transformed host cell as described previously;
b. Obtaining a DXS enzyme which is more resistant to clomazone from cell extract, cell suspension, protein fraction, crystal fraction, cell culture, cell homogenate, cell lysate, cell supernatant, cell filtrate, or cell pellet of said transformed host cell.

The following examples are included to demonstrate preferred embodiments of the invention. It should be appreciated by those of skill in the art that the techniques disclosed in the following examples represent well-functioning techniques to the practice of the invention as discovered by the inventor, and thus can be considered to constitute preferred modes for its practice.

### EXAMPLES

### 1) Cloning of DXS cDNAs from Vitis vinifera cv Muscat Ottonel and Gewurztraminer

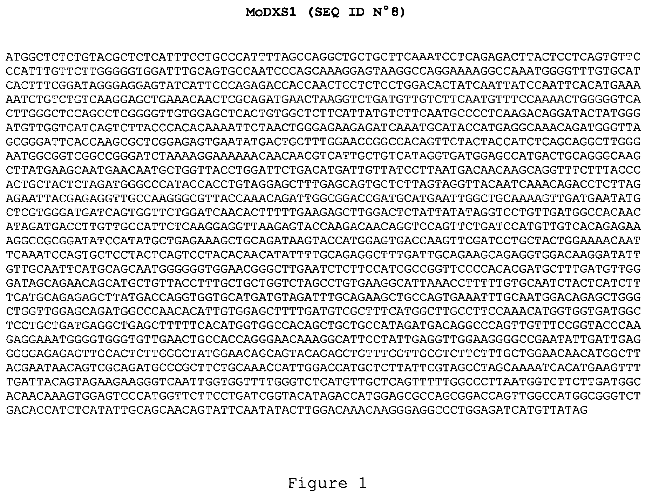

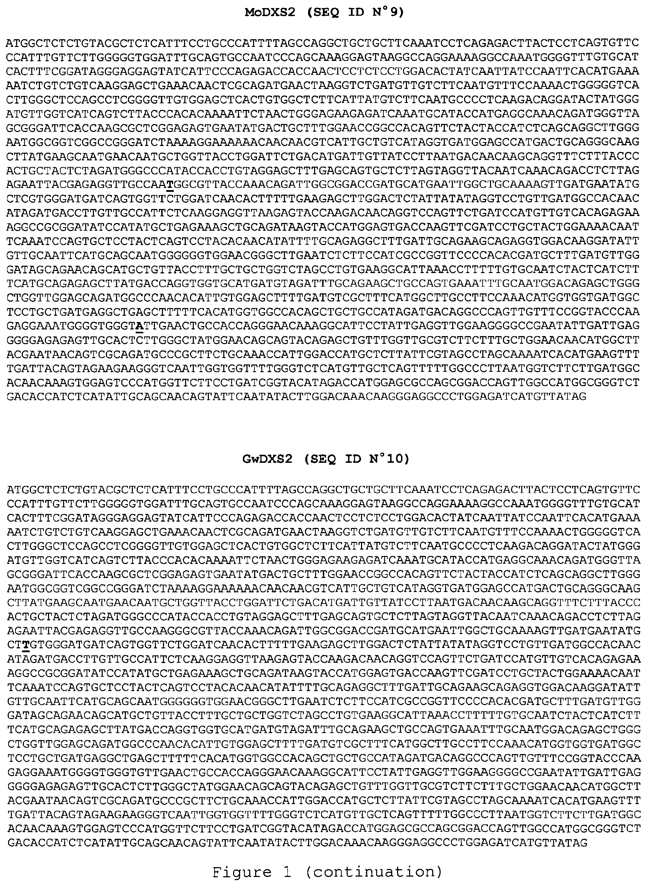

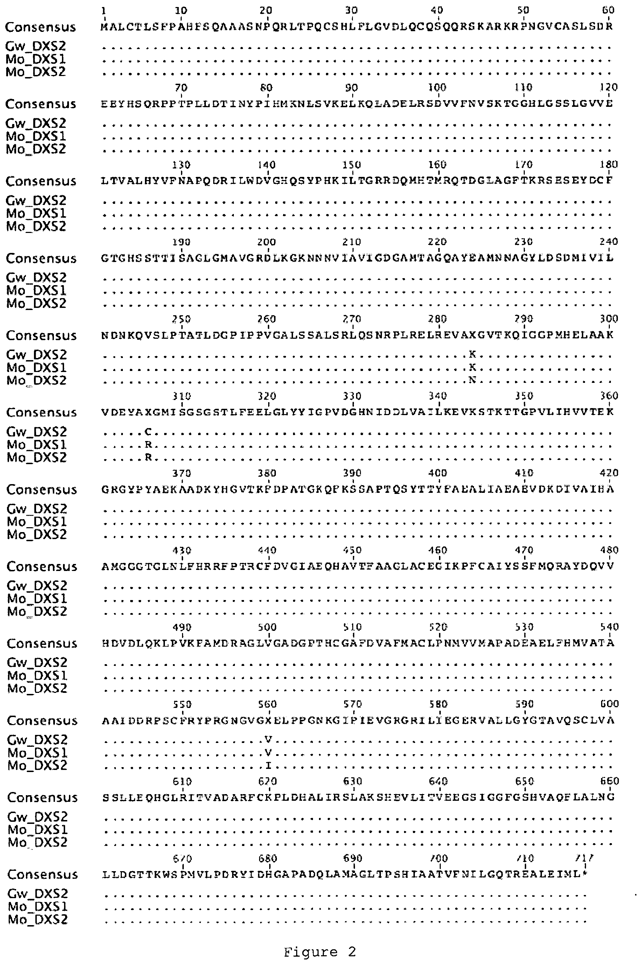

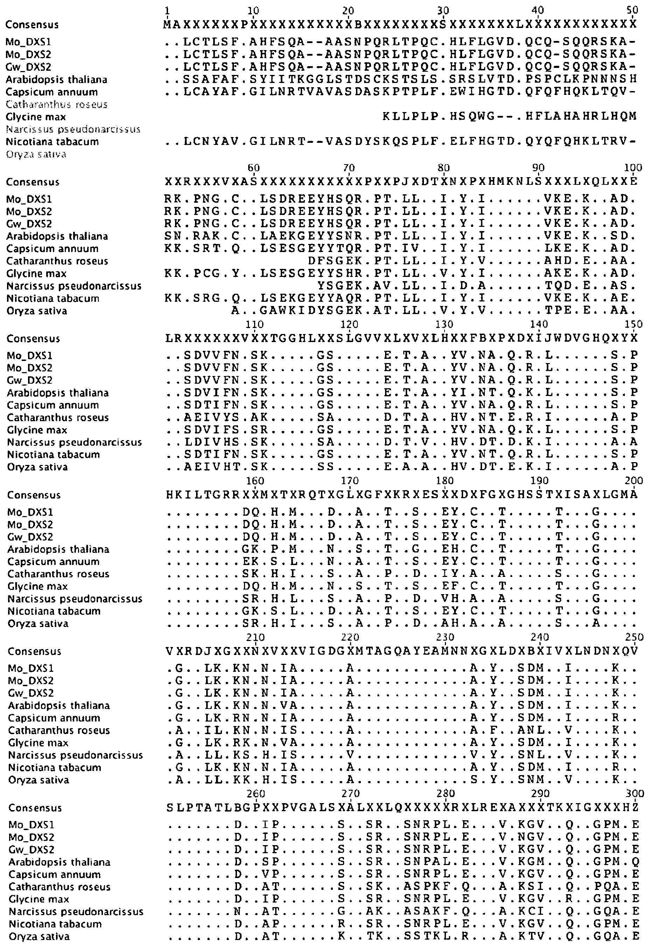

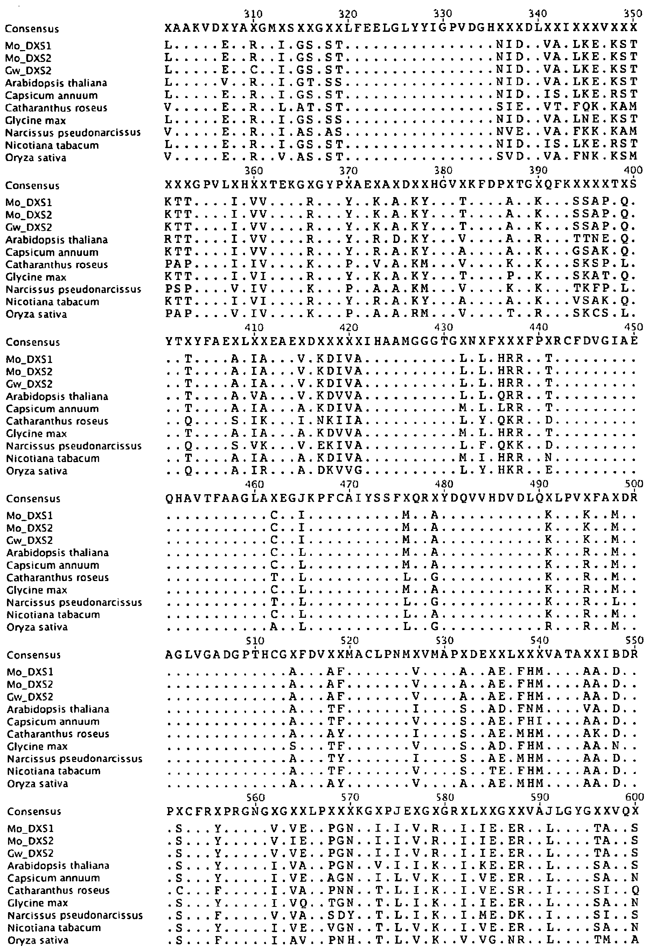

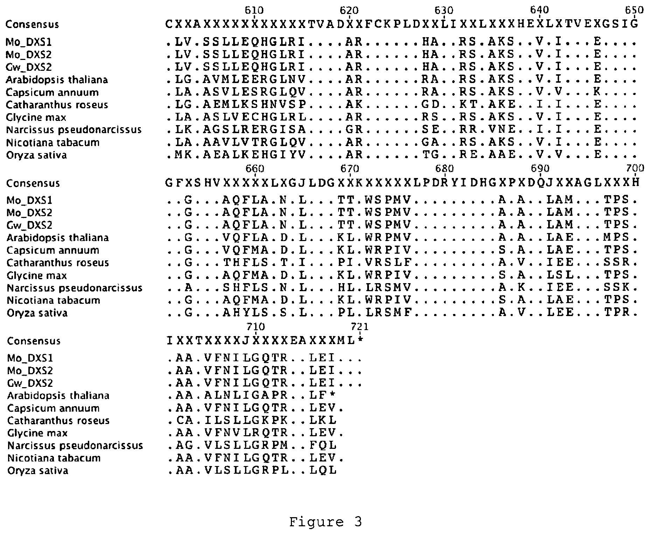

DXS cDNAs from *Vitis vinifera* cv Muscat Ottonel (MO) and Gewurztraminer (Gw) were amplified using RT-PCR and cloned. Sequencing of these clones revealed that both MO and Gw are heterozygous at the DXS locus. MO and Gw share the same allele designated MoDXS1 (Fig. 1). In addition, Mo and Gw possess the MoDXS2 and GwDXS2 alleles, respectively. MoDXS2 and GwDXS2 alleles differ from MoDXS1 in 2 and 1 SNP, respectively, which modify the amino-acid sequence of the corresponding proteins (Fig. 2). The K284→ N and R306→ C mutations (in reference to SEQ ID N°2), characteristic of MoDXS2 and GwDXS2, respectively, affect amino-acids that are conserved in all plant DXS sequences available in databases (Fig 3). Furthermore, the amino-acids K284 and R306 belong to the active site of the enzyme (Xiang et al., 2007), indicating that they may modify the activity of the MoDXS2 and GwDXS2 alleles, in comparison to MoDXS1.

### 2) Impact of the MoDXS2 and GwDXS2 alleles on terpenol content in grape berries

### Material and methods: Isolation of free and bound monoterpenols by solid phase extraction (SPE)

For each sampling point, three replicates of terpenol analysis were performed. For each replicate, at least 25 berries were used. Seeds were removed and skins separated from mesocarps. After weighing, skins were ground under liquid nitrogen and then suspended in 40 mL of water after addition of 40 mg of sodium sulphite. The supernatant obtained after a 15-min centrifugation (11400 g at 4°C) was passed through a glass fibre pre-filter and a glass filter. Twenty microlitres of a 1 g/L 4-nonanol solution was added as external standard to allow the quantification of the main terpenols. Extraction of terpenols was adapted from Mateo et al. (1997), ethanol being used instead of methanol. The filtrate was passed through a 1 g phase C18 silica-bonded non-polar SPE column (BOND ELUT JR.), previously washed with 5 mL of methanol and 15 mL of ultra-pure water, at a rate of approximately one drop/s. The sample was then divided in two equal subsamples, one for the analysis of free and glycosidically bound monoterpenols and one for the analysis of free terpenols. Total fractions of free and bound monoterpenols were eluted with 4 mL of absolute ethanol. This total terpenol extract was diluted in 40 mL of citrate/phosphate buffer (pH 4.5) and the resulting solution was incubated with 50 mg of AR2000 glycolytic enzyme (Gist-Brocades, Seclin, France) overnight at 37.5°C to release the glycosidically bound terpenols (Tamborra et al. 2004). The released terpenols were separated by SPE on a C18 column and eluted with 4 mL of dichloromethane after rinsing with 3x 5 mL water. For free terpenol analysis, the hydrolysis step was not performed and terpenols were directly eluted with dichloromethane after rinsing with water. Extracts were dried in Pasteur pipettes filled with 0.5 g of anhydrous sodium sulphite and concentrated to 500 *µ*L under a gentle nitrogen flux. Twenty microlitres of a 1 g/L solution of m-cresol was added to each sample as internal control. Samples were stored at -20°C prior to gas chromatography (GC) analysis.

### Results:

Example 2 shows that DXS alleles have a major impact on terpenol content in grape berries, as shown by the investigation of terpenol contents as a function of DXS alleles in a MO (Fig. 4) and in a GW (Fig. 5) progeny. MoDXS1 (= GwDXS1) homozygous genotypes exhibit extremely low levels of terpenols, whereas heterozygous or DXS2 homozygous genotypes accumulate large amounts of terpenols.

### 3) Functional characterization of DXS alleles in plants

In order to characterize DXS alleles activity, the inventors used Agrobacterium-mediated transient transformation of tobacco (*Nicotiana benthamiana*), which allows a fast and efficient gene expression in tobacco leaves (Batoko et al., 2000). This approach was already used successfully to characterize genes involved in grapevine secondary metabolism (Schmidlin et al., 2008; Hugueney et al., 2009). MoDXS1, MoDXS2 and GwDXS2 alleles were expressed in tobacco leaves, together with a cDNA encoding the enzyme geraniol synthase (GES) from basilicum (*Ocimum basilicum*) (Iijima et al., 2004). In this system, GES was used as a reporter gene responsible for the biosynthesis of the monoterpenol geraniol, which normally does not accumulate in tobacco. As DXS activity has been shown to be limiting for terpenes biosynthesis in plants (Estevez et al., 2001; Enfissi et al., 2005), the amount of geraniol formed by GES activity depended directly on DXP substrate availability, and therefore, on DXS activity in tobacco leaves. Thus, GC-MS analyses of tobacco leaves co-expressing GES and DXS alleles allowed an accurate comparison of DXS alleles activity in planta.

### Material and methods:

Grapevine DXS cDNAs were amplified by PCR using the upstream primer 5'-GGGGACAAGTTTGTACAAAAAAGCAGGCTTGGTTCCGCGTGGATCAATGGCTCTCTG TACGCTCTCATTTCC-3' (SEQ ID N°11) and the downstream primer 5'-GGGGACCACTTTGTACAAGAAAGCTGGGTTCACTATAACATGATCTCCAGGGCCTCC -3' (SEQ ID N°12), and cloned into the pDONR207 Gateway compatible vector (Invitrogen, Carlsbad, CA) using Gateway BP clonase (Invitrogen) according to the manufacturer's instructions. DXS cDNAs were sequenced to verify that no mutation had been introduced. For Agrobacterium-mediated transient expression, DXS cDNAs were subsequently transferred into the GATEWAY binary vector pMDC32 (Curtis and Grossniklaus., 2003). Geraniol synthase cDNA from basilicum (*Ocimum basilicum*) (Iijima et al., 2004) was cloned into the GATEWAY binary vector pMDC83 (Curtis and Grossniklaus., 2003) and expressed as a GFP fusion protein. All constructs were introduced into *Agrobacterium tumefaciens* strain C58 (pMP90) by electroporation. Nicotiana benthamiana leaves were infiltrated with *A. tumefaciens* cultures (OD600 0.5) according to Voinnet et al. (2003). Leaf sectors were and analysed for terpene content 96 h after Agrobacterium infiltration.

Then, an extraction of terpenols from tobacco leaves was realized:
For each sampling point, three replicates of terpenol analysis were performed. After weighing, tobacco leaf sectors (about 1 g) were ground under liquid nitrogen and then suspended in 2 mL of ultra-pure water. After a 5-min centrifugation (6000 g at 4°C), the supernatant was colleted and 20 µL of a 1 g/L 4-nonanol solution was added as an external standard. The supernatant was passed through a 1 g phase C18 silica-bonded non-polar SPE column (Bond Elut Jr., Varian), previously washed with 5 mL of methanol and 15 mL of ultra-pure water. Total terpenols were eluted with 2 mL of absolute ethanol. This total terpenol extract was diluted in 20 mL of citrate/phosphate buffer (pH 4.5) and the resulting solution was incubated with 25 mg of AR2000 glycolytic enzyme (Gist-Brocades, Seclin, France) overnight at 37.5°C. The released terpenols were separated by SPE on a C18 column and eluted with 4 mL of dichloromethane after washing with 3x 5 mL water. Extracts were dried in Pasteur pipettes filled with 0.5 g of anhydrous sodium sulphite and concentrated to 500 *µ*L under a gentle nitrogen flux. Twenty microlitres of a 1 g/L solution of m-cresol was added to each sample as internal control. Samples were stored at -20°C prior to gas chromatography (GC) analysis.

Finally, the terpenes were analysed by gas chromatography and mass spectrometry (GC-MS) using an Agilent 6890N gas chromatograph equipped with a Gerstel MPS2 XL sampler and coupled to an Agilent 5975B inert mass spectrometer (Agilent Technologies). The gas chromatograph was fitted with a DB-Wax capillary column (60 m × 0.32 mm i.d. × 0.5 *µ*m film thickness, J&W Scientific) and helium was used as carrier gas (1 ml min-1 constant flow). The GC oven temperature was programmed without initial hold time from 45°C to 82°C at a rate of 20°C min-1 (hold 1 min) then from 82°C to 235°C (hold 15 min) at a rate of 2.7°C/min. The injector was set to 250°C and used in pulsed splitless mode (25 psi for 0.50 min). The temperatures of the interface, MS ion source and quadrupole were 270°C, 230°C and 150°C, respectively. The Mass spectrometer was operated in electron impact ionization mode (EI, 70 eV) and the masses were scanned over a m/z range of 29 - 300 amu. Agilent MSD ChemStation software (G1701DA, Rev D.03.00) was used for instrument control and data processing. The mass spectra were compared with the Wiley's library reference spectral bank.

### Results:

Agrobacterium-mediated transient expression of GES alone led to the accumulation of significant amounts of geraniol, whose biosynthesis depended on the endogenous DXS activity in tobacco (Fig. 6, Fig. 7). Co-expression of GES and MoDXS1 led to substantial increases in geraniol biosynthesis, due to the enhanced DXP substrate availability. However, co-expression of GES and MoDXS2 or GwDXS2 led, in average, to a three time increase in geraniol accumulation in tobacco leaves (Fig. 7), indicating that these alleles possessed an enhanced DXS activity, compared to MoDXS1.

Therefore, the inventors conclude that the massive accumulation of terpenols in MO and GW is due to the presence of the MoDXS2 or GwDXS2. The K284→ N and R306→ C mutations, which affect amino-acids in the active site of the enzyme, confer an enhanced DXS activity compared to MoDXS1, which in turn is responsible for the increased accumulation of terpenes in planta.

The aim of the following experiments is to investigate whether the role of these mutations can be extended to DXS enzymes from other sources. As a model, the inventors have selected the DXS from *Escherichia coli,* whose enzymatic properties are well characterized (Querol et al., 2001; Xiang et al., 2007).

Comparison of the DXS proteins sequences from *E. coli* and *V. vinifera* indicated that the amino acid K284 and R306 in grapevine DXS correspond to K213 and K234 in the DXS from *E.coli,* respectively (Figure 10). In order to assess the impact of MO- and GW-like mutations on the activity of a non-plant DXS, the mutations K213→N and the K234→C were introduced in the DXS protein of *E*. *coli.* The impacts of these mutations on the terpene biosynthetic capabilities of *E. coli* were subsequently investigated.

### 4) Cloning and site-directed mutagenesis of the DXS gene from Escherichia coli

### Material and methods:

The DXS gene from *Escherichia coli* (Lois et al., 1998) was amplified by PCR using the upstream primer GGGGACAAGTTTGTACAAAAAAGCAGGCTTGGTTCCGCGTGGATCAATGAGTTTTG ATATTGCCAAATACCC (SEQ ID N°24) and the downstream primer GGGGACCACTTTGTACAAGAAAGCTGGGTTCATTATGCCAGCCAGGCCTTGATTTTG (SEQ ID N°25) and cloned into the pDONR207 Gateway compatible vector (A technology that enables rapid cloning of one or more genes. The entry vector confers resistance to gentamycin)(Invitrogen, Carlsbad, CA) using BP clonase, a mixture of proteins that catalyse the in vitro recombination of PCR products or DNA segments from clones, (Invitrogen) according to the manufacturer's instructions. The DXS gene was subsequently transferred into the GATEWAY-compatible expression vector pHGWA (Busso et al., 2003) using LR clonase, a mixture of enzymes that catalyse the in vitro recombination between an entry clone and a destination vector to generate the expression clone, (Invitrogen),

yielding the plasmid pHGWA-EcoliDXS. This plasmid was used for site-directed mutagenesis, in order to introduce the K213→N and the K234→C mutations into the DXS protein form *E coli.* Site-directed mutagenesis was performed using the QuikChange kit (Stratagene, La Jolla, CA) according to the manufacturer's instructions. QuikChange kit is based on mutagenic primers and DNA polymerase. It is designed to help introduce single or multiple point mutations into a gene of choice to produce enzymes mutants with amino acids changes, insertions or deletions.

The forward primer GCGCGAAGGCGGGAACAAAGTTTTCTCTGGCGTGCC (SEQ ID N°26) and the reverse primer GGCACGCCAGAGAAAACTTTGTTCCCGCCTTCGCGC (SEQ ID N°27) were used to introduce the K213→N mutation, yielding the plasmid pHGWA-EcoliDXS-K213N. The forward primer CGCACCGAAGAACATATTTGCGGCATGGTAGTGCCTGG (SEQ ID N°28) and the reverse primer CCAGGCACTACCATGCCGCAAATATGTTCTTCGGTGCG (SEQ ID N°29) were used to introduce the K234→C mutation, yielding the plasmid pHGWA-EcoliDXS-K234C.

### Results:

During the site-directed mutagenesis, a mutant DXS gene harbouring a frameshift was isolated. This frameshift resulted in the synthesis of a truncated DXS protein, which was used as a control. The plasmid encoding this truncated DXS is termed pHGWA-truncated-EcoliDXS.

### 5) Expression of wild type and mutant E. coli DXS proteins in E.coli

***Material and methods:*** pHGWA-EcoliDXS, pHGWA-truncnted-EcoliDXS, pHGWA-EcoliDXS-K213N and pHGWA-EcoliDXS-K234C plasmids were transformed into the BL21-Gold(DE3)pLysS *E*. *coli strain* (Stratagene, La Jolla, CA). BL21-Gold(DE3)pLysS *E*. *coli* strain are competent cells that provide increased transformation efficiency. They are resistant to tetracycline and chloramphenicol. Transformants were grown at 28°C in 50 mL LB medium supplemented with glucose (10 g/L), in the presence of ampicillin (100 *µ*g/mL), chloramphenicol (25 *µ*g/mL), and tetracyclin (2 *µ*g/mL). When the cultures reached an OD of 0.1, DXS expression was induced with 1 mM of isopropyl β-D-1-thiogalnctopyranoside (IPTG) and the culture was grown for 24h.

### 6) Impact of DXS mutants on terpene biosynthesis in E. coli

The impact of MO- and GW-like mutations (K213→N and K234→C, respectively) on the activity the DXS enzyme from *E. coli* was assessed by quantifying the terpenes in culture media, after induction of DXS expression.

### Material and methods: Analysis of terpenes in E. coli culture media

Terpenes in culture media were analysed using stir bar sorptive extraction (SBSE), using a method adapted from Coelho et al. (2009). A stir bar with PDMS coating (0.5mm x 10mm; Twister; Gerstel, Mullheim, Germany) was introduced into each culture medium sample (10 mL). Extractions were performed at 20 °C for 150 min, at a rotation speed of 800 rpm. Terpenes were then analysed by gas chromatography and mass spectrometry (GC-MS) using an Agilent 6890N gas chromatograph equipped with a Gerstel MPS2 XL sampler and coupled to an Agilent 5975B mass spectrometer (Agilent Technologies), as described above.

### Results:

Example shows that the mutations introduced in the DXS from *E. coli* impact the monoterpene content in culture media (Fig. 5). In particular, the mutation K234→C in the DXS from *E coli* leads to a greatly enhanced accumulation of terpenes in culture media. Therefore, the inventors conclude that non-plant DXS enzymes can be modified at the positions corresponding to K284 and R306 in the DXS proteins of grapevine, in order to improve DXS activity. Such improved DXS mutants may be used for the metabolic engineering of microorganisms, in order to enhance the production of isoprenoids of interest.

## Claims

1. Use of a mutated isolated polypeptide having a 1-deoxy-D-xylulose 5-phosphate synthase (DXS) activity consisting of converting glyceraldehyde-3-phosphate and pyruvate into 1-deoxyxylulose-5-phosphate, derived from the 1-deoxy-D-xylulose 5-phosphate synthase (DXS) corresponding to the consensus sequence SEQ ID N°21, said mutated isolated polypeptide comprising one or two of the following two mutations:
- substitution of the amino acid in position 213 of SEQ ID N°21 by an asparagine,
- substitution of the amino acid in position 234 of SEQ ID N°21 by a cysteine, and the sequence of the said mutated isolated polypeptide having at least 90% identity with the sequence SEQ ID N°21,
to increase terpenes production and/or accumulation in a host cell transformed by the said mutated isolated polypeptide having a DXS activity.

2. The use of the mutated isolated polypeptide having a DXS activity according to claim 1, wherein the mutated isolated polypeptide has the sequence SEQ ID N°19.

3. The use of the mutated isolated polypeptide having a DXS activity according to claim 1, wherein the mutated isolated polypeptide has the sequence SEQ ID N°20.

4. The use of the mutated isolated polypeptide having a DXS activity according to claims 1 to 3, wherein the transformed host cell is a prokaryotic cell selected in the group comprising eubacterial, archaebacterial and cyano-bacterial cells, preferably said prokaryotic cell is an *Escherichia coli* cell.

5. The use of the mutated isolated polypeptide having a DXS activity according to claims 1 to 3, wherein the transformed host cell is a eukaryotic cell selected in the group comprising animal, fungal, yeast, and plant cells preferably a plant cell selected in the group comprising *Vitis vinifera, Nicotiana tabacum,* and *Arabidopsis thaliana* cells.

## Patentansprüche

1. Gebrauch eines mutierten, isolierten Polypeptids mit 1-Desoxy-D-xylulose-5-phosphat-Synthase-Aktivität, darin bestehend, Glycerinaldehyd-3-phosphat und Pyruvat in 1-Desoxyxylulose-5-phosphat umzusetzen, und von der 1-Desoxy-D-xylulose-5-phosphat-Synthase (DXS) abstammend, die der Konsensussequenz SEQ ID Nr. 21 entspricht, wobei das genannte mutierte, isolierte Polypeptid eine oder beide der folgenden beiden Mutationen aufweist:
- Substitution der Aminosäure an der Stelle 213 von SEQ ID Nr. 21 durch Asparagin,
- Substitution der Aminosäure an der Stelle 234 von SEQ ID Nr. 21 durch Cystein,
wobei die Sequenz des genannten mutierten, isolierten Polypeptids zu mindestens 90% mit der Sequenz SEQ ID Nr. 21 identisch ist,
zur Erhöhung der Erzeugung und/oder Sammlung von Terpenen in einer Wirtszelle, die durch das genannte mutierte, isolierte Polypeptid mit DXS-Aktivität transformiert wurde.

2. Gebrauch des mutierten, isolierten Polypeptids mit DXS-Aktivität nach Patentanspruch 1, in dem das mutierte, isolierte Polypeptid die Sequenz SEQ ID Nr. 19 hat.

3. Gebrauch des mutierten, isolierten Polypeptids mit DXS-Aktivität nach Patentanspruch 1, in dem das mutierte, isolierte Polypeptid die Sequenz SEQ ID Nr. 20 hat.

4. Gebrauch des mutierten, isolierten Polypeptids mit DXS-Aktivität nach den Patentansprüchen 1 bis 3, wobei die transformierte Wirtszelle eine prokaryotische Zelle aus der Gruppe ist, die Eubakterien-, Archaebakterien- und Cyanobakterienzellen umfasst, wobei die genannte prokaryotische Zelle vorzugsweise eine *Escherichia coli-Zelle* ist.

5. Gebrauch des mutierten, isolierten Polypeptids mit DXS-Aktivität nach den Patentansprüchen 1 bis 3, wobei die transformierte Wirtszelle eine eukaryotische Zelle aus der Gruppe ist, die Tier-, Pilz-, Hefe- und Pflanzenzellen umfasst, vorzugsweise eine Pflanzenzelle aus der Gruppe, die Zellen von *Vitis vinifera, Nicotiana tabacum,* und *Arabidopsis thaliana* umfasst.

## Revendications

1. Utilisation d'un polypeptide isolé muté ayant une activité de 1-désoxy-D-xylulose 5-phosphate synthase (DXS) consistant à convertir le glycéraldéhyde-3-phosphate et le pyruvate en 1-désoxyxylulose-5-phosphate, dérivé de la 1-désoxy-D-xylulose 5-phosphate synthase (DXS) correspondant à la séquence consensus SEQ ID N°21, ledit polypeptide isolé muté comprenant une ou deux des mutations suivantes :
- substitution de l'acide aminé en position 213 de la SEQ ID N°21 par une asparagine,
- substitution de l'acide aminé en position 234 de la SEQ ID N°21 par une cystéine,
et la séquence dudit polypeptide isolé muté ayant au moins 90% d'identité avec la séquence SEQ ID N°21,
pour augmenter la production et/ou l'accumulation de terpènes dans une cellule hôte transformée par ledit polypeptide isolé muté ayant une activité DXS.

2. Utilisation du polypeptide isolé muté ayant une activité DXS selon la revendication 1, dans laquelle le polypeptide isolé muté a la séquence SEQ ID N°19.

3. Utilisation du polypeptide isolé muté ayant une activité DXS selon la revendication 1, dans laquelle le polypeptide isolé muté a la séquence SEQ ID N°20.

4. Utilisation du polypeptide isolé muté ayant une activité DXS selon les revendications 1 à 3, dans laquelle la cellule hôte transformée est une cellule procaryote choisie dans le groupe comprenant les eubactéries, les archaebactéries et les cyanobactéries, de préférence ladite cellule procaryote est une *Escherichia coli.*

5. Utilisation du polypeptide isolé muté ayant une activité DXS selon les revendications 1 à 3, dans laquelle la cellule hôte transformée est une cellule eucaryote choisie dans le groupe comprenant des cellules animales, fongiques, de levure et végétales, de préférence une cellule végétale choisie dans le groupe comprenant des cellules de *Vitis vinifera, Nicotiana tabacum* et *Arabidopsis thaliana.*
